# EUROPEAN PATENT APPLICATION

(11) **EP 4 763 231 A1**
(43) Date of publication of application: **24.06.2026**
(21) Application number: 24222634.8
(22) Date of filing: 20.12.2024
(51) Int. Cl.: A61K 51/04, A61K 103/40, A61P 35/00

(54) **PARA-AMINOHIPPURIC ACID AS A STABILIZER IN RADIOPHARMACEUTICAL COMPOSITIONS**

(71) Applicant: ITM Isotope Technologies Munich SE, 85748 Garching bei München (DE)
(72) Inventor: Fuchs, Elisabeth, 85749 Garching (DE); Meckel, Marian, 80807 München (DE); Bolik, Kim, 85748 Garching (DE); Schmidt, Theresa, 85749 Garching (DE)
(74) Representative: Maiwald GmbH

(57) **Abstract**

The present invention provides a pharmaceutical composition comprising a radiolabeled complex comprising a radionuclide and a targeting moiety linked to a chelating moiety, and a stabilizer against radiolytic degradation of a radiolabeled complex comprising para-aminohippuric acid or a pharmaceutically acceptable salt or carboxylic derivative thereof. The pharmaceutical composition of the present invention is characterized by high stability of its radiolabeled complex against radiolytic degradation. The present invention also provides a method for preparing such a pharmaceutical composition, and use of para-aminohippuric acid or a pharmaceutically acceptable salt or carboxylic derivative thereof for the stabilization of the radiolabeled complex of such a pharmaceutical composition.

## Description

The present invention relates to the use of para-aminohippuric acid or a pharmaceutically acceptable salt or carboxylic derivative thereof for the stabilization of radiopharmaceutical compositions. It also relates to radiopharmaceutical compositions comprising a radiolabeled pharmaceutical compound and comprising para-aminohippuric acid or a pharmaceutically acceptable salt or carboxylic acid derivate thereof as a stabilizer. The present application also relates to a method for preparing such radiopharmaceutical compositions.

Radiopharmaceuticals are drugs which contain radioactive isotopes (radionuclides). Radiopharmaceuticals can be used to treat various conditions, including cancers, blood disorders and hyperthyroidism. In radionuclide therapy of cancer, a molecule labeled with a radionuclide is used to deliver a toxic level of radiation to disease sites. Accordingly, the molecule is used to "target" the disease site, e.g. specific cancer cells. Accordingly, the radionuclide complex combines the specificity of cancer cell targeting with the known antitumor effects of ionizing radiation. Thereby, not only the primary tumor site, but also its metastases can be targeted. The choice of the molecule that carries the radiation to the tumor is usually determined by its selectivity and affinity to the tumor's target structures, such as antigens, receptors or proteins. Even if a target structure is not selective for a certain kind of cancer, overexpressed target structures are of interest, because they allow the delivery of the radionuclide complex after its systemic administration in high concentration to those (overexpressing) target cells while leaving other cells (with no or minor expression only) essentially unaffected.

Radionuclides are usually linked to the targeting moiety via a chelating moiety. Thereby, strong complexes with the metal ion of the radionuclide can be formed. The radioactive decay of the radionuclides can cause significant damage to cancer cells by releasing high energy electrons, positrons or alpha particles as well as gamma rays at the target site.

However, radioactive decay of the radionuclide occurs constantly, including during manufacturing and storage of the radionuclide complex. The high energy emitted in radioactive decay can induce the cleavage of the chemical bonds of the radionuclide complex, thereby leading to partial destruction of the drug due to its radioactivity. The radiolytic degradation of e.g. the targeting moiety of the radionuclide complex may result in a reduced specificity of the radionuclide complex, thereby leading to a decrease in its efficacy and/or to an increase in undesired side effects.

After radiolabeling the precursor compounds, the stability of radiopharmaceuticals is usually restricted to a few hours or days only. This results in various challenges regarding the manufacture, storage and transport of radiopharmaceuticals. Therefore, for the application of radiopharmaceuticals only a small window is available after manufacturing.

In order to reduce this problem, usually antioxidants, such as gentisic acid, ethanol, ascorbic acid and methionine, are added to the formulation of the radionuclide complex. However, in particular for peptides labeled with Lu-177, such as DOTA-TOC (edotreotide) and DOTA-TATE (oxodotreotide), often complex mixtures of antioxidants or specific strategies, e.g. time points of their addition are required to obtain the desired effect over an acceptable period of time. For example, Maus et al. reports the addition of ascorbic acid after radiolabeling and purification of [¹⁷⁷Lu]Lu-DOTA-TATE (Maus S. et al. Aspects on radiolabeling of 177Lu-DOTA-TATE: After C18 purification re-addition of ascorbic acid is required to maintain radiochemical purity, International Journal of Diagnostic Imaging, 2014, Vol. 1, No. 1).

US patent 10,596,278 B2 requires a complex mixture of gentisic acid, ascorbic acid, and EDTA after radiolabeling to obtain a desired stability against radiolysis of 95% at 72 hours after synthesis. De Blois and Breeman et al. even suggest the addition of a mixture of 50 mM ascorbic acid, 10% (v/v) ethanol and 50 mM L-methionine (De Blois E. et al. Effectiveness of Quenchers to Reduce Radiolysis of 111In- or 177Lu-labeled Methionine-Containing Regulatory Peptides. Maintaining Radiochemical Purity as Measured by HPLC. Curr Top Med Chem. 2012;12(23):2677-85; Wouter A. P. Breeman; Practical Aspects of labeling DTPA- and DOTA-Peptides with 90Y, 111In, 177Lu, and 68Ga for Peptide-Receptor Scintigraphy and Peptide-Receptor Radionuclide Therapy in Preclinical and Clinical Applications; The University of New Mexico Health Sciences Center, VOLUME 16, LESSON 5: 11/16/2012). According to these reports, for ¹⁷⁷Lu-labeled peptides, the stabilizer, or the second or third stabilizing component, respectively, is added after radiolabeling in acetate or HEPES buffer.

The strength of radiolysis also depends in particular on the amount and the nature of the chemical precursor (peptide) used, the radioactivity concentration and the type of antioxidant, as well as their concentrations. In addition to radiolysis stability, particular attention must also be paid to pH and osmolarity in the case of parenterally administered radiopharmaceuticals. The composition of a multicomponent formulation for the stabilization of therapeutic radiopharmaceuticals is a complex matter for this reason.

In the current literature, however, no formulation for radiolabeled complexes, such as Lu-177 labeled compounds, Ac-225 labeled compounds, or Tb-161 labeled compounds, suitable for injection purposes, has yet been described that ensures stability over at least 96 h with a purity of ≥ 95%.

In view of the above, it is the object of the present invention to overcome the drawbacks outlined above and to provide a pharmaceutical composition including a radionuclide complex which ensures increased stability of the radionuclide complex over an increased period of time. It is further object of the present invention to provide a straightforward method for preparing a pharmaceutical composition which provides for a radiolysis-stable formulation containing as few components as possible and, in particular, suitable for parenteral injection purposes.

This object is achieved by means of the subject-matter set out below and in the appended claims.

Although the present invention is described in detail below, it is to be understood that this invention is not limited to the particular methodologies, protocols and reagents described herein as these may vary. It is also to be understood that the terminology used herein is not intended to limit the scope of the present invention which will be limited only by the appended claims. Unless defined otherwise, all technical and scientific terms used herein have the same meanings as commonly understood by one of ordinary skill in the art.

In the following, the elements of the present invention will be described. These elements are listed with specific embodiments, however, it should be understood that they may be combined in any manner and in any number to create additional embodiments. The variously described examples and preferred embodiments should not be construed to limit the present invention to only the explicitly described embodiments. This description should be understood to support and encompass embodiments which combine the explicitly described embodiments with any number of the disclosed and/or preferred elements. Furthermore, any permutations and combinations of all described elements in this application should be considered disclosed by the description of the present application unless the context indicates otherwise.

Throughout this specification and the claims which follow, unless the context requires otherwise, the term "comprise", and variations such as "comprises" and "comprising", will be understood to imply the inclusion of a stated member, integer or step but not the exclusion of any other non-stated member, integer or step. The term "consist of" is a particular embodiment of the term "comprise", wherein any other non-stated member, integer or step is excluded. In the context of the present invention, the term "comprise" encompasses the term "consist of". The term "comprising" thus encompasses "including" as well as "consisting" e.g., a composition "comprising" X may consist exclusively of X or may include something additional e.g., X + Y.

The terms "a" and "an" and "the" and similar reference used in the context of describing the invention (especially in the context of the claims) are to be construed to cover both the singular and the plural, unless otherwise indicated herein or clearly contradicted by context. Recitation of ranges of values herein is merely intended to serve as a shorthand method of referring individually to each separate value falling within the range. Unless otherwise indicated herein, each individual value is incorporated into the specification as if it were individually recited herein. No language in the specification should be construed as indicating any non-claimed element essential to the practice of the invention.

The word "substantially" does not exclude "completely" e.g., a composition which is "substantially free" from Y may be completely free from Y. Where necessary, the word "substantially" may be omitted from the definition of the invention.

The term "about" in relation to a numerical value x means x ± 20%, preferably x ± 10%, more preferably x + 5%, even more preferably x ± 2% and still more preferably x + 1 %.

### Pharmaceutical composition

In a first aspect the present invention provides a pharmaceutical composition comprising:
(a) a radiolabeled complex comprising (i) a radionuclide, and (ii) a targeting moiety covalently linked to a chelating moiety; and
(b) a stabilizer against radiolytic degradation of the radiolabeled complex,
   wherein the stabilizer comprises a compound according to Formula (1) or a pharmaceutically acceptable salt thereof: 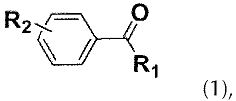
   wherein R₁ is selected from the group consisting of H, OH, NH₂, an alanine residue, and a glycine residue, and
   R₂ is selected from the group consisting of H, OH, NH₂, F, Cl, Br, I, SO₂, methyl, O-methyl, and O-ethyl,
   and wherein the compound according to Formula (1) or a pharmaceutically acceptable salt thereof is present in the pharmaceutical composition in a concentration of 1 mg/mL to 300 mg/mL.

The present inventors have surprisingly found that such a formulation of the radionuclide complex ensures increased radiochemical stability of the radionuclide complex of ≥ 95% over at least 96 hours, when stored at room temperature (RT), and also ensures high radiochemical stability at elevated temperatures of e.g. 40°C. Due to the stability over an extended period of time the radiolabeled complex of the pharmaceutical is provided with consistently high quality; moreover, on site production (at the site of use) as applied for radiolabeled complexes of lower stability allowing them to be administered without delay after their preparation is no longer required. Rather, centralized production with sufficient time for subsequent distribution of the drug to the treatment centers can be ensured.

interestingly, the increased stability/shelf life can be achieved with a formulation containing fewer components as compared to prior art formulations for radionuclide complexes. In particular, para-aminohippuric acid or a pharmaceutically acceptable salt or carboxylic derivative thereof used as a stabilizer of the formulation is pharmaceutically compatible and toxicologically safe and may also serve as a pH modulator or buffer in the pharmaceutical formulation providing a pH range of 4.5 - 8 suitable for parenteral injection purposes so that complicated mixtures can be avoided.

Moreover, the present formulation is suitable for various radionuclides with different radiation properties. These include alpha-emitters, beta-emitters and Auger electron emitters, such as Ac-225, Tb-161, Lu-177, etc. It has been found that the present formulation ensures radiolysis-stable radioactivity concentrations of 0.3 - 1.0 GBq/mL for beta emitters as well as 0.2 - 1.0 MBq/mL for alpha emitters. Further, the formulation solution ensures radiolysis-stable volumes of 1-20 mL and a shelf life of the medicinal product of ≥96 h after preparation.

The present pharmaceutical composition is suitable for a variety of targeting moieties, such as radiolabeled peptides, peptidomimetics and small molecules, as a constituent of the radiolabeled complex included in the present pharmaceutical composition.

### Radiolabeled complex

The radiolabeled complex of the pharmaceutical composition according to the present invention comprises (i) a radionuclide, and (ii) a targeting moiety, wherein the targeting moiety is covalently linked to a chelating moiety.

### Radionuclide

Various radionuclides (radioisotopes) are known to be useful in the field of radionuclide therapy. In particular, the term "radionuclide" (or "radioisotope") refers to isotopes of natural or artificial origin with an unstable neutron to proton ratio that disintegrates with the emission of corpuscular (i.e. positrons (beta plus-radiation); helium nuclei (alpha-radiation) or electrons (beta minus-radiation, Auger and conversion electron radiation) or electromagnetic radiation (gamma-radiation). In other words, radionuclides undergo radioactive decay. Said radionuclide may preferably be useful for cancer imaging or therapy.

Non-limiting examples of suitable radionuclides include Ac-225, At-211, Bi-212, Bi-213, Co-55, Co-57, Cu-61, Cu-64, Cu-67, Dy-166, Er-165, Er-169, F-18, Ga-67, Ga-68, Ho-166, I-123, I-124, 1-131, In-111, Lu-177, Pb-212, Pd-103, Pd-109, Re-186, Re-188, Rh-103m, Sc-43, Sc-44, Sc-47, Sm-153, Tb-149, Tb-152, Tb-155, Tb-161, Tc-99m, Th-227, Tc-99m, Y-86, and Y-90.

Accordingly, the radionuclide of the radiolabeled complex and the radiolabeled compound of the pharmaceutical composition according to the present invention may be any one of the before-mentioned examples. The choice of suitable radionuclides may depend *inter alia* on the chemical structure and chelating capability of the chelating agent, and the intended application of the resulting (complexed) conjugate (e.g. diagnostic vs. therapeutic). For instance, the beta-minus emitters such as Y-90, I-131, Tb-161 and Lu-177 may be used for systemic radionuclide therapy, while F-18, Ga-67, Tc-99m, In-111, and 1-123 may be used for nuclear medical imaging.

Preferably, the radionuclide is selected from the group consisting of Ac-225, At-211, Bi-213, Bi-212, Co-55, Co-57, Cu-61, Cu-64, Cu-67, Dy-166, Ga-67, Ga-68, Ho-166, In-111, Lu-177, Pb-212, Sc-43, Sc-44, Sc-47, Sm-153, Tb-161, Y-86, and Y-90. In particular embodiments, the radionuclide may be Ac-225, Lu-177, orTb-161, for example.

Thus, in a particular embodiment, beta-emitting radionuclides may be selected from Lu-177 and Tb-161, and alpha-emitting radionuclides may be selected from Ac-225, Pb-212, and Tb-149, for example.

### Chelating moiety

In the radiolabeled complex, the radionuclide metal ion usually forms a non-covalent bond with functional groups of the chelating moiety, e.g. amines or carboxylic acids. Typically, the chelating moiety has at least two such complexing functional groups to be able to form a chelate complex.

As used herein, the term "chelating moiety" (also referred to as "chelator") refers to polydentate (multiple bonded) ligands capable of forming two or more separate coordinate bonds with ("coordinating") a central (metal) ion, in particular the radionuclide metal ion. Specifically, such molecules or molecules sharing one electron pair may also be referred to as "Lewis bases". The central (metal) ion is usually coordinated by two or more electron pairs to the chelating agent. The terms, "bidentate chelating agent", "tridentate chelating agent", and "tetradentate chelating agent" are known in the art and refer to chelating agents having two, three, and four electron pairs, respectively, which are readily available for simultaneous donation to a metal ion coordinated by the chelating agent. Usually, the electron pairs of a chelating moiety forms coordinate bonds with a single central (metal) ion; however, in certain examples, a chelating moiety may form coordinate bonds with more than one metal ion, with a variety of binding modes being possible.

The terms "coordinating" and "coordination" refer to an interaction in which one multi-electron pair donor coordinatively bonds (is "coordinated") to, i.e. shares two or more unshared pairs of electrons with, one central (metal) ion.

The chelator or chelating moiety is preferably a macrocyclic bifunctional chelator having a metal chelating group at one end and a reactive functional group at the other end, which is capable to bind to other moieties, in particular to targeting moieties, e.g. peptides, or linkers serving as spatial separators between targeting moiety and chelator. Preferably, the chelator may be selected such that the chelator forms a square bi-pyramidal complex for complexing the radionuclide. In another embodiment, the chelator does not form a planar or a square planar complex.

The chelating moiety may be selected based on its ability to coordinate the desired central (metal) ion, usually the radionuclide as described herein.

Accordingly, the chelating moiety may be a macrocyclic or linear chelator characterized by one of the following Formulae (2a) to (2ff): 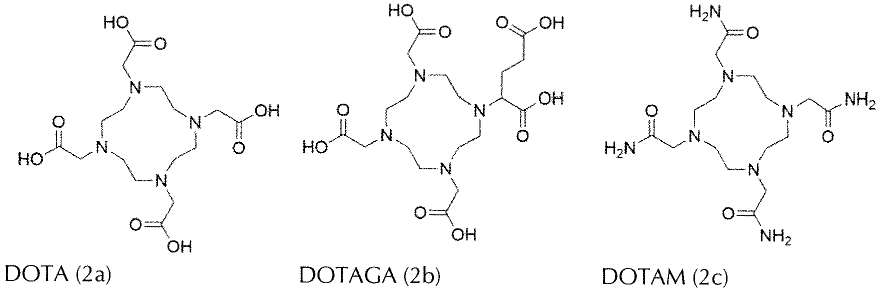 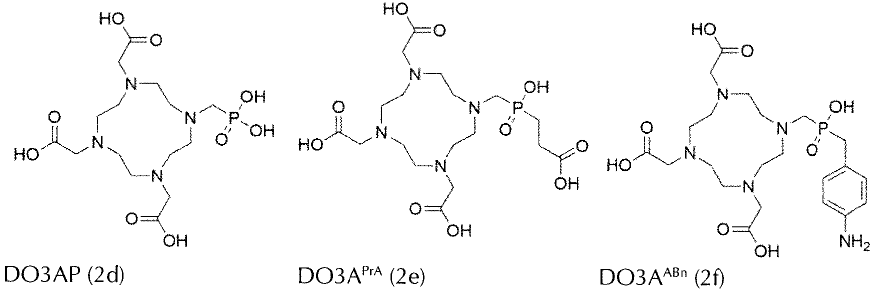 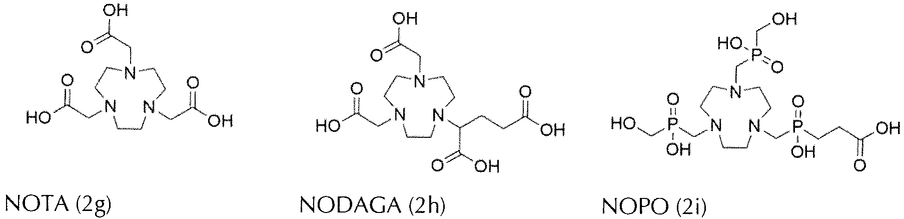 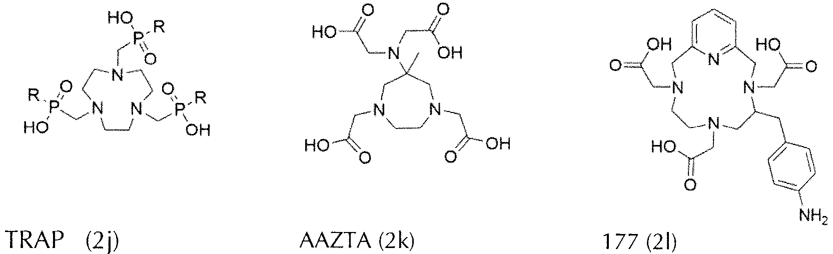
R =H, TRAPH
R = (CH₂)₂CO₂H, TRAP-Pr
R = CH₂OH, TRAP-OH
R = phenyl, TRAP-Ph

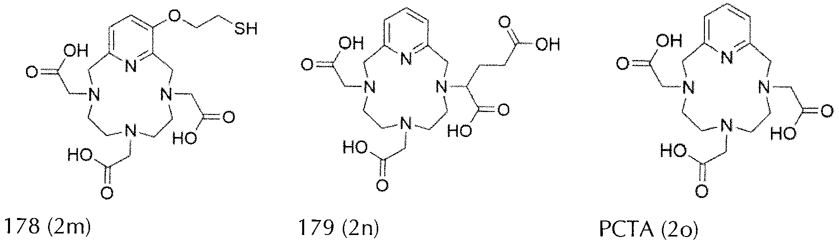

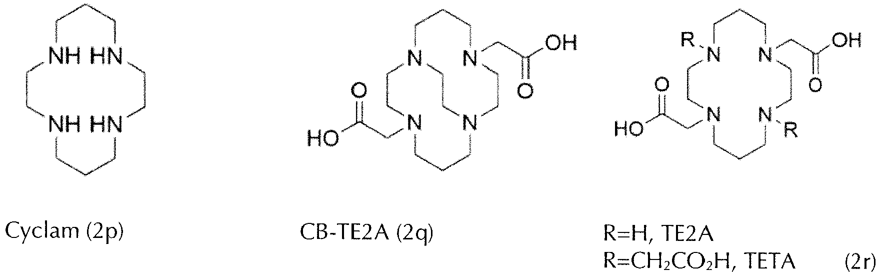

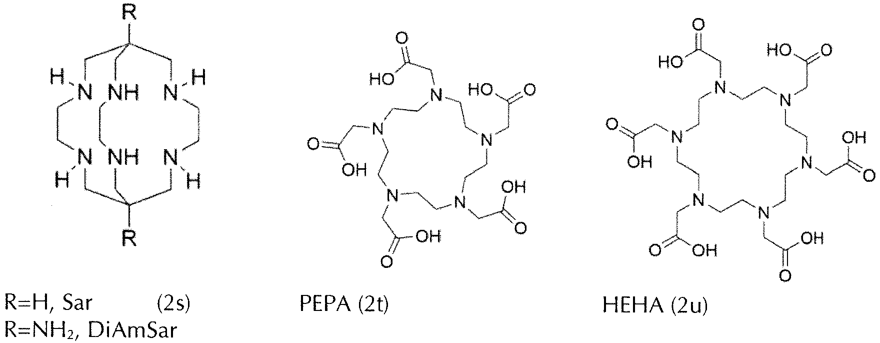

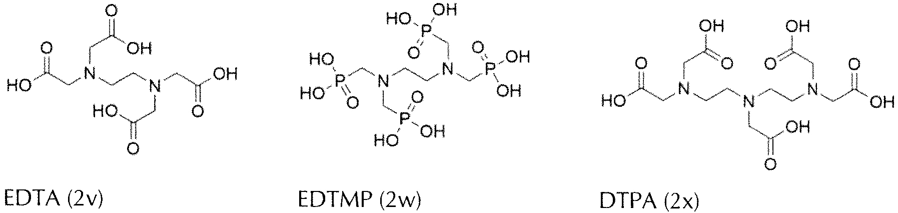

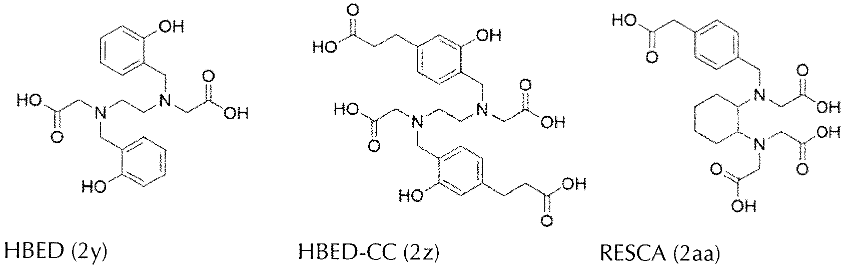

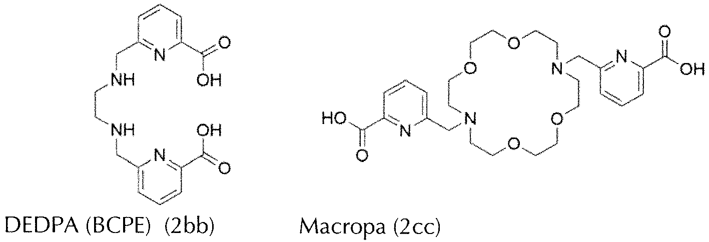

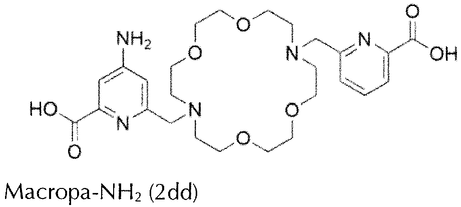

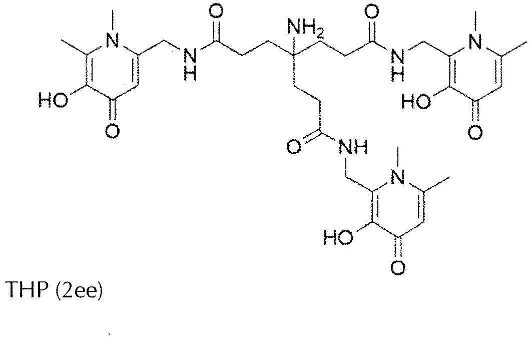

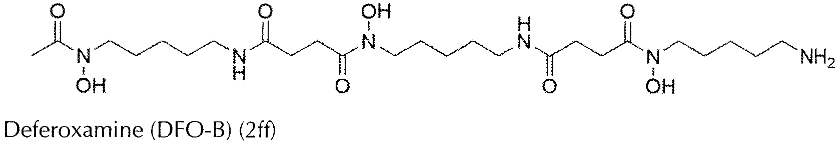

Preferably, the chelating moiety is selected from 1,4,7,10-tetraazacyclododecane-1,4,7,10-tetraacetic acid (DOTA), 1,4,7-triazacyclononane-1,4,7-triacetic acid (NOTA), 2-(4,7-bis(carboxymethyl)-1,4,7-triazonan-1-yl)pentanedioic acid (NODAGA), *N*,*N*'-bis[2-hydroxy-5-(carboxyethyl)-benzyl]ethylenediamine-*N*,*N*'-diacetic acid (HBED-CC), 2-(4,7,10-tris(carboxymethyl)-1,4,7,10-tetraazacyclododecan-1-yl)-pentanedioic acid (DOTAGA), 1,4,7-triazacyclononane phosphinic acid (TRAP), 1,4,7-triazacydononane-1-[methyl(2-carboxyethyl)-phosphinic acid]-4,7-bis[methyl(2-hydroxymethyl)phosphinic acid] (NOPO), 3,6,9, 15-tetraazabicyclo[9,3,1]pentadeca-1(15),11,13-triene-3,6,9-triacetic acid (PCTA), N'-{5-[Acetyl(hydroxy)amino]pentyl}-N-[5-({4-[(5-aminopentyl)(hydroxy)amino]-4-oxobutanoyl}amino)pentyl]-N-hydroxysuccinamide (DFO), Diethylenetriaminepentaacetic acid (DTPA), ethylenediaminetetraacetic acid (EDTA), 1,4,7,10-Tetraazacyclododecane-1,4,7-triacetic acid (DO3A) and derivatives thereof (cf. formulae 2d, 2e, 2f), hydrazinonicotinic acid (HYNIC), and 1,4,7,10-Tetrakis(carbamoylmethyl)-1,4,7,10-tetraazacyclododecane (DOTAM).

In a particular embodiment, the chelating moiety may be selected from DOTA (which may be characterized by Formula (2a)), DOTAM (which may be characterized by Formula (2c)), NOTA (which may be characterized by Formula (2g)), NODAGA (which may be characterized by Formula (2h)) and DOTAGA (which may be characterized by Formula (2b)).

For example, DOTA, as chelating agent, may advantageously enable the use of Lu-177, Tb-161, Sc-43, Sc-44, Sc-47, Ac-225, Bi-213, Bi-212, or Pb-212 as radionuclides, while NODAGA is more favorably used for chelation of Ga-68, Ga-67 and Cu-61, Cu-64, Cu-67.

Preferably, the chelating moiety is selected from DOTA (1,4,7,10-tetraazacyclododecane-1,4,7,10-tetraacetic acid, and DOTAGA (2-(4,7,10-tris(carboxymethyl)-1,4,7,10-tetraazacyclododecan-1-yl)-pentanedioic acid) or derivatives thereof.

Particularly preferably, the chelating agent is DOTA. Advantageously, DOTA effectively forms complexes with diagnostic (e.g. Ga-68) and therapeutic (e.g. Tb-161 or Lu-177) radionuclides and thus enables the use of the same conjugate (targeting molecule linked to the chelating agent) for both imaging and therapeutic purposes, i.e. as a theragnostic agent. DOTA derivatives capable of complexing Scandium radionuclides (Sc-43, Sc-44, Sc-47), including DO3AP, DO3AP^{PrA}, or DO3AP^{ABn} (which may be characterized by Formulae (2d), (2e) and (2f)) may also be preferred and are described in Kerdjoudj et al. Dalton Trans., 2016, 45, 1398-1409.

The chelating agent, for example DOTA, may be complexed with any appropriate radionuclide (in particular with the radionuclide as described above) as a central (metal) ion. It is within the skill and knowledge of the skilled person in the art to select suitable combinations of conjugates and radionuclides.

In some embodiments, the chelator may be DOTA and the radionuclide may be Ga-68. In other embodiments, the chelator may be NODAGA and the radionuclide may be Ga-68. In other embodiments, the chelator may be DOTA and the radionuclide may be Sc-44. In yet further embodiments, the chelator may be DOTA and the radionuclide may be Cu-64. In other embodiments, the chelator may be NODAGA and the radionuclide may be Cu-61, Cu-64 or Cu-67. In other embodiments, the chelator may be DOTAM and the radionuclide may be Pb-212. In a preferred embodiment, the chelator may be DOTA and the radionuclide may be Tb-161. In another preferred embodiment, the chelator may be DOTAGA and the radionuclide may be Lu-177. In another preferred embodiment, the chelator may be DOTA and the radionuclide may be Lu-177. In another preferred embodiment, the chelator may be DOTA and the radionuclide may be Ac-225.

### Targeting moiety

As used herein, the term "targeting moiety" refers to a molecule, which is able to address (specifically) to a "target", such as a target cell (e.g., a cancer cell). In particular, the "target" may be a molecule located at the cell surface of a target cell (e.g., a cancer cell). Such a cell surface molecule, to which the targeting moiety binds, may be, for example, a receptor located at the surface of the cell. In particular, the cell surface molecule is specific for or overexpressed by the target cell (e.g., a cell "marker"). The targeting moiety may bind, for example, to a disease (e.g., cancer) marker (which is expressed/located at the surface of the cell involved in the disease, e.g. a cancer cell). Thereby, the targeting moiety can guide the radionuclide specifically to the cell involved in the disease, e.g. a cancer cell. Accordingly, the targeting moiety is usually selected depending on the disease to be treated or diagnosed. In the context of a disease, e.g. cancer, the cells to be targeted with the radiolabeled complex, e.g. cancer cells, usually express specific molecules (or overexpress specific molecules), which may serve as "target" (surface molecule). The targeting moiety is typically selected such that it binds to said "targets" (surface molecules and, thus, target cells, e.g. cancer cells). The binding of the targeting moiety to the surface molecule may be reversible or irreversible. Preferably, the targeting moiety is selected from peptides, peptidomimetics, antibodies, antibody fragments, antibody mimetics, and small molecules. In some embodiments, the targeting moiety is a peptide or polypeptide or a modified peptide or polypeptide.

### PSMA-targeting compounds

In some embodiments, the radiolabeled complex of the pharmaceutical composition according to the present invention comprises a targeting moiety which targets PSMA and thus is able to bind to PSMA.

The human prostate-specific membrane antigen (PSMA) (also referred to as glutamate carboxypeptidase II (GCPII), folate hydrolase 1, folypoly-gamma-glutamate carboxypeptidase (FGCP), and N-acetylated-alpha-linked acidic dipeptidase I (NAALADase I)) is a type II transmembrane zinc metallopeptidase. The term "human prostate-specific membrane antigen" or "PSMA" as used herein preferably refers to the protein encoded by the human FOLH1 gene. More preferably, the term refers to the protein as characterized under UniProt Acc. No. Q04609 (entry version 186, last modified May 10, 2017), or functional variants, isoforms, fragments or (post-translationally or otherwise modified) derivatives thereof.

The prostate-specific membrane antigen (PSMA) is overexpressed in the majority of prostate cancer cases (Silver, D. A.; Pellicer, I.; Fair, W. R.; Heston, W. D.; Cordon-Cardo, C. Prostate-specific membrane antigen expression in normal and malignant human tissues. Clin Cancer Res 1997, 3, (1), 81-5; Cunha, A. C.; Weigle, B.; Kiessling, A.; Bachmann, M.; Rieber, E. P. Tissue-specificity of prostate specific antigens: comparative analysis of transcript levels in prostate and non-prostatic tissues. Cancer Lett 2006, 236, (2), 229-38). It emerged, therefore, as a promising target for nuclear imaging and radionuclide therapy of metastatic castration-resistant prostate cancer (mCRPC) (Bouchelouche, K.; Choyke, P. L. Prostate-specific membrane antigen positron emission tomography in prostate cancer: a step toward personalized medicine. Curr Opin Oncol 2016, 28, (3), 216-21; Haberkorn, U.; Eder, M.; Kopka, K.; Babich, J. W.; Eisenhut, M. New strategies in prostate cancer: prostate-specific membrane antigen (PSMA) ligands for diagnosis and therapy. Clin Cancer Res 2016, 22, (1), 9-15; Eiber, M.; Fendler, W. P.; Rowe, S. P.; Calais, J.; Hofman, M. S.; Maurer, T.; Schwarzenboeck, S. M.; Kratowchil, C.; Herrmann, K.; Giesel, F. L. Prostate-specific membrane antigen ligands for imaging and therapy. J Nucl Med 2017, 58, (Suppl 2), 67S-76S). PSMA is (i) mainly restricted to the prostate (although is also detected in lower amounts in the neovasculature of numerous other solid tumors, including bladder, pancreas, lung, and kidney cancers, but not in normal vasculature), (ii) abundantly expressed as protein at all stages of prostate cancer (in amounts of up to 10⁶ PSMA molecules per cancer cell) (iii) presented at the cell surface but not shed into the circulation, and (iv) associated with enzymatic or signaling activity. Moreover, PSMA expression is further up-regulated in poorly differentiated, androgen-insensitive or metastatic cancers and the expression usually correlates with disease progression.

The PSMA-binding targeting moiety of the radiolabeled complex included in the pharmaceutical composition according to the present invention may generally be a binding entity capable of selectively (and optionally irreversibly) binding to (human) prostate-specific membrane antigen (e.g., as described in Chang Rev Urol. 2004; 6 (Suppl 10): S13-S18). The PSMA targeting moiety is preferably chosen by its ability to confer selective affinity towards PSMA. Preferred PSMA binding moieties are described in WO 2013/022797 A1, WO 2015/055318 A1 and EP 2862857 A1, which are incorporated herein by reference in their entirety.

Accordingly, the PSMA targeting moiety may preferably be characterized by General Formula (3): 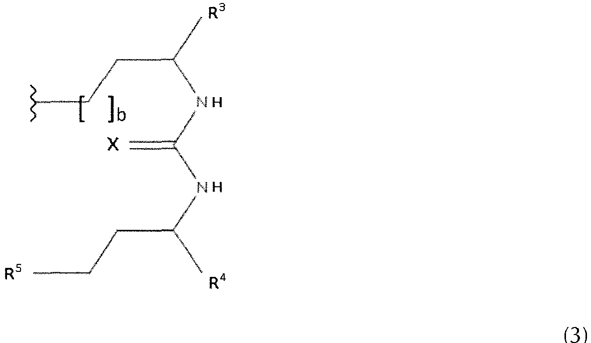 wherein
X is selected from O, N, S or P,
R³, R⁴ and R⁵ are each independently selected from -COH, -CO₂H, -SO₂H, -SO₃H, - SO₄H, -PO₂H, -PO₃H, -PO₄H₂, -C(O)-(C₁-C₁₀)alkyl, -C(O)-O(C₁-C₁₀)alkyl, -C(O)-NHR⁸, or - C(O)-NR⁸R^{9,} wherein R⁸ and R⁹ are each independently selected from H, bond, (C1-C10)alkylene, F, Cl, Br, I, C(O), C(S), -C(S)-NH-benzyl-, -C(O)-NH-benzyl, -C(O)-(C₁-C₁₀)alkylene, -(CH₂)ₚ-NH, -(CH₂)ₚ-(C₁-C₁₀)alkyene, -(CH₂)ₚ-NH-C(O)-(CH₂)_{q}, -(CHᵣCH₂)ₜ-NH-C(O)-(CH₂)ₚ, -(CH₂)ₚ-CO-COH, -(CH₂)ₚ-CO-CO₂H, -(CH2)ₚ-C(O)NH-C[(CH₂)_{q}-COH]₃, - C[(CH₂)ₚ-COH]₃, -(CH2)ₚ-C(O)NH-C[(CH₂)_{q}-CO₂H]₃, -C[(CH₂)ₚ-CO₂H]₃ or -(CH₂)ₚ-(C₅-C₁₄)heteroaryl, and
b, p, q, r, t is each independently an integer selected from 0, 1, 2, 3, 4, 5, 6, 7, 8, 9, or 10.

In preferred PSMA targeting moieties, b may be an integer selected from 1, 2, 3, 4 or 5, R³, R⁴ and R⁵ may each be CO₂H, X may be O.

Examples of small-molecule PSMA targeting agents capable of binding to the extracellular domain of PSMA include, but are not limited to: N-[N-[(S)-1,3-dicarboxypropyl]carbamoyl]-S-[12c]methyl-l-cysteine (DCFBC), several urea-based peptidomimetic PSMA-inhibitors as described in Bouchelouche et al. Discov Med. 2010 Jan; 9(44): 55-61), including MIP-1095 (Hillier et al. Cancer Res. 2009 Sep 1;69(17):6932-40).

Kelly et al. (Dual-Target Binding Ligands with Modulated Pharmacokinetics for Endoradiotherapy of Prostate Cancer. J Nucl Med. 2017 Sep;58(9):1442-1449. doi: 10.2967/jnumed.116.188722) evaluated agents exhibiting affinity for both PSMA and for human serum albumin (HSA). The ligands developed by Kelly et al. comprise a *p-*(iodophenyl)butyric acid entity for HSA binding and an urea-based PSMA binding entity. In the compounds developed by Kelly et al., radiotherapeutic iodine (¹³¹I) is covalently attached to the HSA binding moiety, which is in turn directly connected to the PSMA binding entity via a hydrocarbyl chain.

As mentioned above, the targeting moiety and the chelating moiety usually form together conjugates or molecules (suitable for radiolabeling). Various such conjugates/molecules are known in the art. Preferred conjugates comprising a chelating moiety and a targeting moiety, which is able to bind to PSMA, are disclosed in WO 2018/215627 A1, which is also incorporated herein by reference.

Preferred examples of conjugates comprising a targeting moiety and a chelating moiety include PSMA-617 developed by Benešová et al (JNM 2015, 56: 914-920 and EP 2862 857 A1) according to Formula (4) below; PSMA-I&T (Weineisen M et al., J Nucl Med. 2015; 56:1169-1176) according to Formula (5) below; lbu-PSMA according to Formula (6) below; Ibu-DAB-PSMA according to Formula (7) below; Ibu-N-PSMA according to Formula (8) below; and Ibu-Dα-PSMA according to Formula (9) below:
PSMA-617: 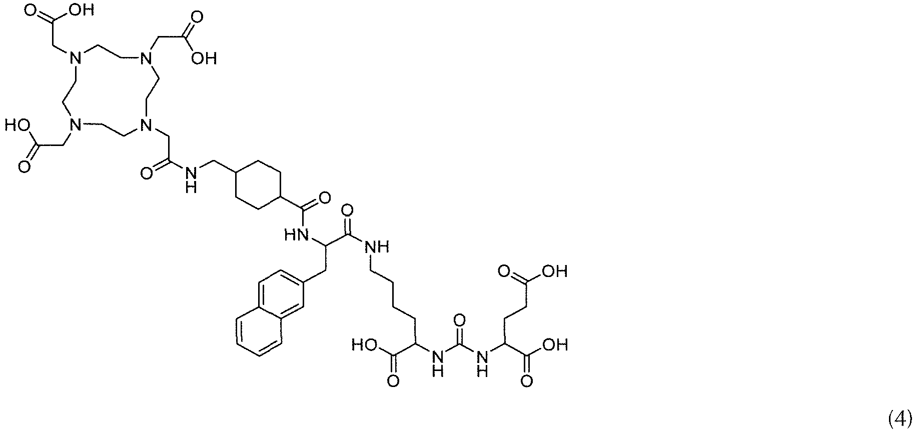
PSMA-I&T: 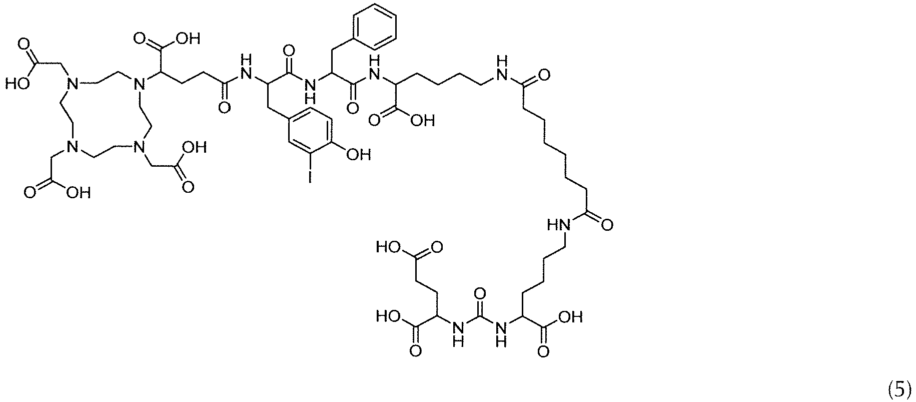
Ibu-PSMA: 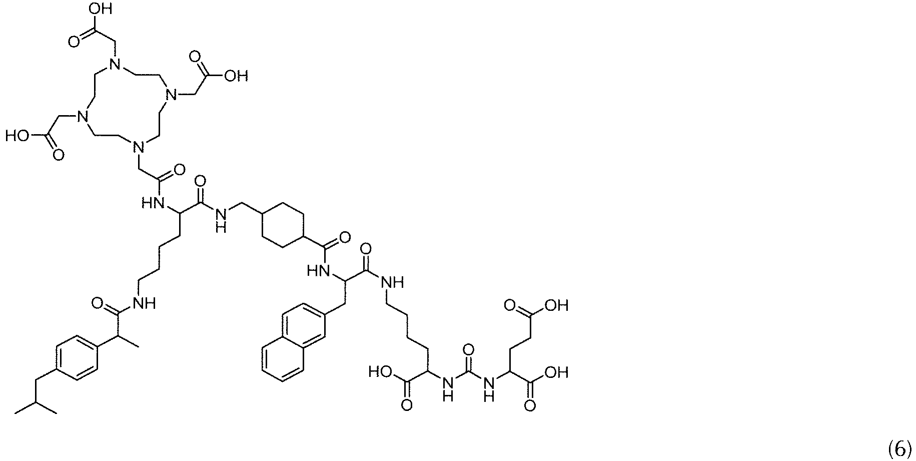
Ibu-DAB-PSMA: 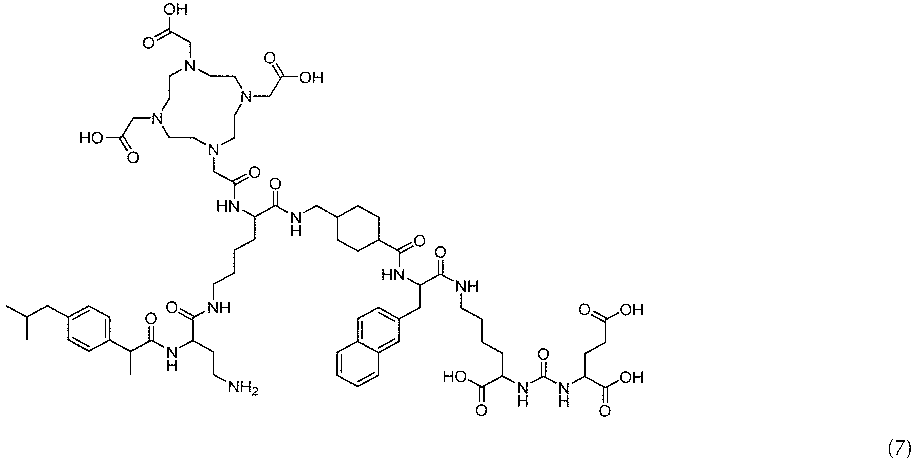
Ibu-N-PSMA: 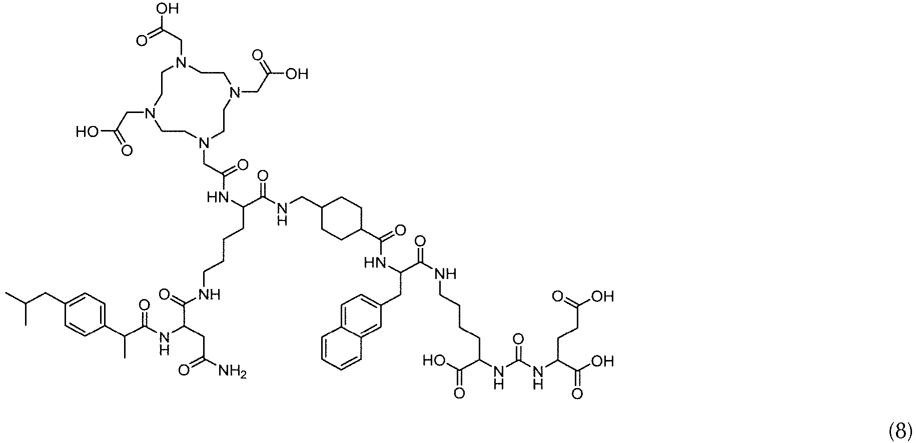
Ibu-Dα-PSMA:

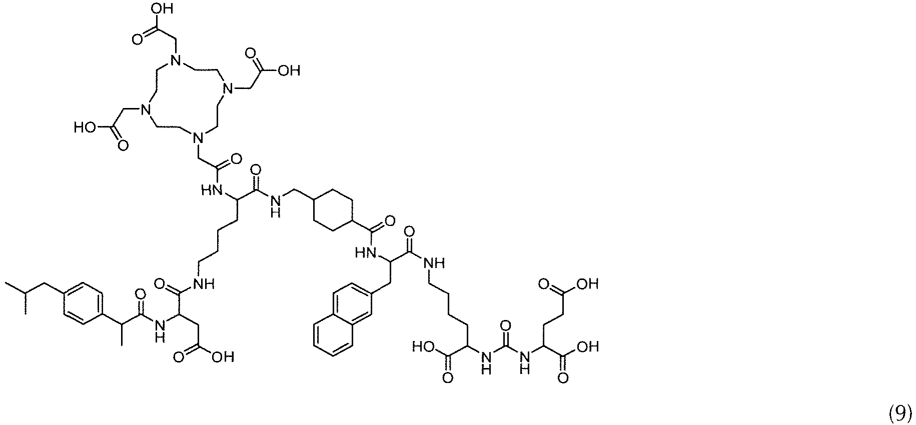

Accordingly, the radiolabeled complex (comprising the radionuclide, and the targeting moiety linked to the chelating moiety) of the pharmaceutical composition according to the present invention may be obtained from a (precursor) compound represented by above formulae (4) to (9), and is for example selected from [²²⁵Ac]Ac-Ibu-DAB-PSMA, [²²⁵Ac]Ac-PSMA-617, [²²⁵Ac]Ac-PSMA-I&T, [¹⁷⁷Lu]Lu-Ibu-DAB-PSMA, [¹⁷⁷Lu]Lu-PSMA-617, [¹⁷⁷Lu]Lu-PSMA-I&T, [¹⁶¹Tb]Tb-Ibu-DAB-PSMA, [¹⁶¹Tb]Tb-PSMA-617, and [¹⁶¹Tb]Tb-PSMA-I&T.

Further examples of (radio)pharmaceuticals based on PSMA targeting agents include, but are not limited to: [¹⁷⁷Lu]Lu-AAZTA-5-PSMA (Glu-NH-CO-NH-Lys-2-naphthyl-L-Ala-AMCH-AAZTA-5), [¹⁷⁷Lu]Lu-EB-PSMA-617, [¹⁷⁷Lu]Lu-RPS-072, [²²⁵Ac]Ac-EB-PSMA-617, [²²⁵Ac]Ac-RPS-072, [²¹²Pb]Pb-PSMA.

### Somatostatin receptor targeting compounds

Other particularly suitable targeting molecules bind to a somatostatin receptor. In particular for the treatment of well to moderately differentiated neuroendocrine tumors (NET), peptides targeting the somatostatin receptor (SSTR) may be used. In NET, radioligand therapy is well-established and may achieve high rates of long-lasting tumor remission and stabilization.

Molecules binding to a somatostatin receptor are known in the art, such as somatostatin analogues. Preferably, the targeting molecule is a somatostatin receptor binding peptide. More preferably, said somatostatin receptor binding peptide is selected from octreotide, octreotate, lanreotide, vapreotide, pasireotide, ilatreotide, pentetreotide, depreotide, satoreotide, veldoreotide. Even more preferably, the targeting molecule is a somatostatin receptor binding peptide selected from octreotide and octreotate.

Peptides targeting the somatostatin receptor are e.g. somatostatin analogs tyr3-octreotide (D-Phe-c(Cys-Tyr-D-Trp-Lys-Thr-Cys)-Thr(ol)) and tyr3-octeotrate (D-Phe-c(Cys-Tyr-D-Trp-Lys-Thr-Cys)-Thr) (Capello A et al.: Tyr3-octreotide and Tyr3-octreotate radiolabeled with Lu-1 77 or Y-90: peptide receptor radionuclide therapy results in vitro, Cancer Biother Radiopharm, 2003 Oct; 18(5): 761-8). Further examples of somatostatin receptor agonists are the peptides octreotide (D-Phe-cyclo(Cys-Phe-D-Trp-Lys-Thr-Cys)Thr(ol)), and NOC (D-Phe-cyclo(Cys-1-Nal-D-Trp-Lys-Thr-Cys)Thr(ol)).

Other examples of compounds targeting the somatostatin-receptor are somatostatin antagonistic peptides such as JR10 (p-NO₂-Phe-c(D-Cys-Tyr-D-Aph(Cbm)-Lys-Thr-Cys)D-Tyr-NH₂); JR11 (Cpa-c(D-Cys-Aph(Hor)-d-Aph(Cbm)-Lys-Thr-Cys)D-Tyr-NH2); BASS (p-NO₂-Phe-cyclo(D-Cys-Tyr-D-Trp-Lys-Thr-Cys)D-Tyr-NH₂; LM3 (p-Cl-Phe-cyclo(D-Cys-Tyr-D-Aph(Cbm)-Lys-Thr-Cys)D-Tyr-NH₂.

Thus, the targeting molecule in the radiolabeled complex may be a somatostatin receptor targeting molecule, which may be bound to a chelator molecule, as defined above, and complexed with a radionuclide, as defined above.

Exemplary conjugates comprising a chelating agent and a targeting molecule, which is able to bind to a somatostatin receptor, include DOTA-OC ([DOTA⁰,D-Phe¹]octreotride), DOTA-TOC ([DOTA⁰,D-Phe¹,Tyr³]octreotride; INN: edotreotide), DOTA-NOC ([DOTA⁰,D-Phe¹,₁-Nal³]octreotride), DOTA-TATE ([DOTA⁰,D-Phe¹,Tyr³]octreotate; INN: oxodotreotide), DOTA-LAN ([DOTA0,D-β-Nal³]octreotride), DOTA-VAP ([DOTA⁰,D-Phe¹,Tyr³]vapreotide), DOTA-JR11 (Satoreotide tetraxetan), NODAGA-JR11 (satoreotide trizoxetan) and DOTA-LM3 (DOTA-p-Cl-Phe-cyclo(D-Cys-Tyr-D-4-amino-Phe(carbamoyl)-Lys-Thr-Cys)D-Tyr-NH2).

For example, the conjugate comprising a chelating agent and a targeting molecule may be selected from DOTA-TOC according to Formula (10) below, DOTA-NOC according to Formula (11) below, DOTA-TATE (according to Formula (12) below, DOTA-JR11 according to Formula (13) below, and DOTA-LM3 according to Formula (14) below:
DOTA-TOC: 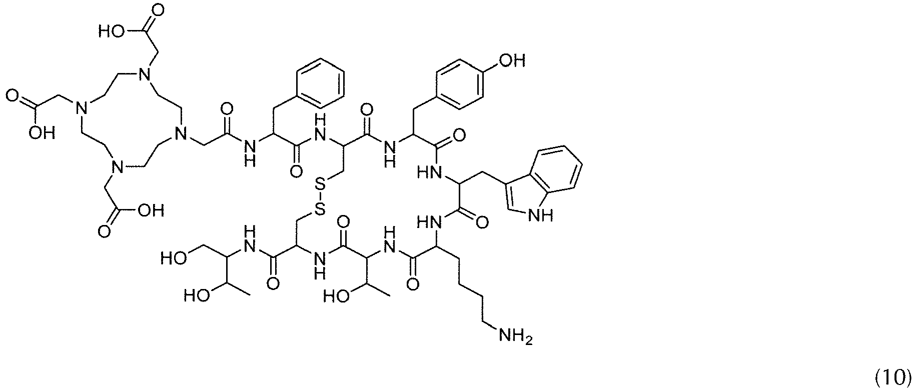
DOTA-NOC: 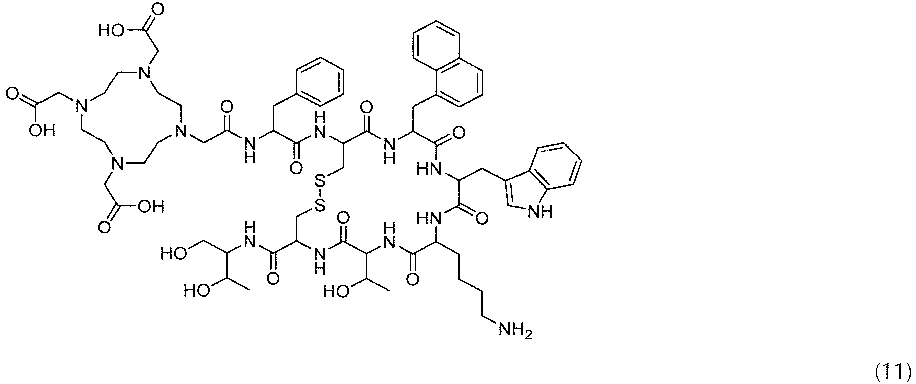
DOTA-TATE: 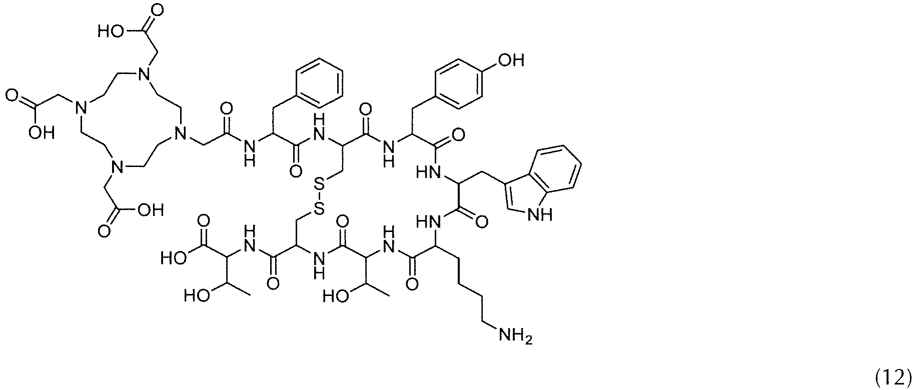
DOTA-JR11: 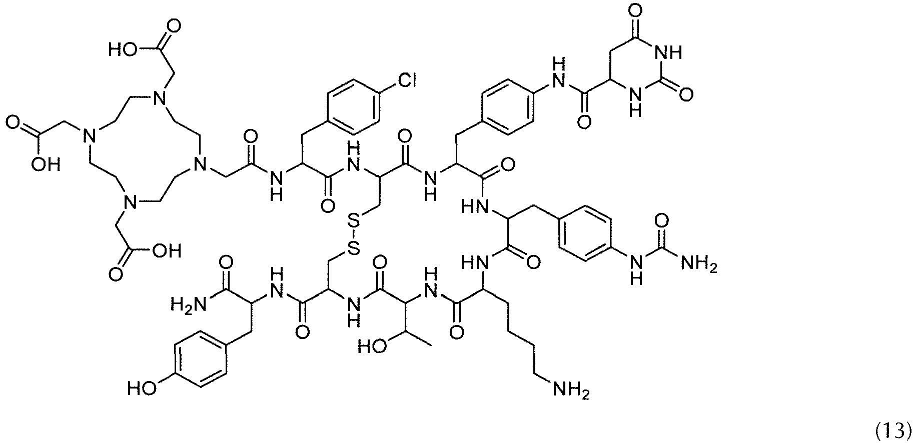
DOTA-LM3:

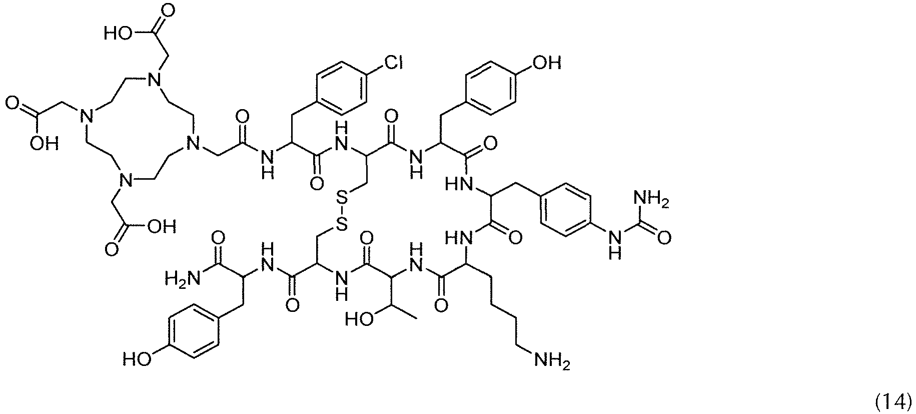

Accordingly, the radiolabeled complex (comprising the radionuclide, and the targeting moiety linked to the chelating moiety) of the pharmaceutical composition according to the present invention may be obtained from a (precursor) compound represented by above Formulae (10) to (14) which is complexed with a radionuclide, as defined above, such as Lu-177, Tb-161, Ac-225, Ga-68, In-111 or Y-90, for example.

Thus, the radiolabeled complex may e.g. be selected from [¹⁷⁷ Lu]Lu-DOTA-TOC, [¹⁷⁷Lu]Lu⁻DOTA-NOC, [¹⁷⁷Lu]Lu-DOTA-TATE, [¹⁷⁷Lu]Lu-DOTA-JR11, [¹⁷⁷Lu]Lu-DOTA-LM3, [¹⁶¹Tb]Tb-DOTA-TOC, [¹⁶¹Tb]Tb-DOTA-NOC, [¹⁶¹Tb]Tb-DOTA-TATE, [¹⁶¹Tb]Tb-DOTA-JR11, [¹⁶¹Tb]Tb-DOTA-LM3, [²²⁵Ac]Ac-DOTA-TOC, [²²⁵Ac]Ac-DOTA-NOC, [²²⁵Ac]Ac-DOTA-TATE, [²²⁵Ac]Ac⁻DOTA-JR11, and [²²⁵Ac]Ac-DOTA-LM3.

Further examples of radiolabeled complexes based on somatostatin analogues include, but are not limited to: [⁶⁸Ga]Ga-DOTA-TOC ([⁶⁸Ga]Ga-DOTA-D-Phe-cyclo(Cys-Tyr-D-Trp-Lys-Thr-Cys)Thr(ol)), [⁶⁸Ga]Ga-DOTA-NOC ([⁶⁸Ga]Ga-DOTA-D-Phe-cyclo(Cys-1-Nal-D-Trp-Lys-Thr-Cys)Thr(ol)), [⁶⁸Ga]Ga-DOTA-JR11 (Ga-OpS201) ([⁶⁸Ga]Ga-DOTA-Cpa-cyclo[D-Cys-Aph(Hor)-D-Aph(Cbm)-Lys-Thr-Cys]D-Tyr-NH₂), [⁶⁸Ga]Ga-NODAGA-JR11 (Ga-OPS202) ([⁶⁸Ga]Ga-NODAGA-Cpa-cyclo[D-Cys-Aph(Hor)-D-Aph(Cbm)-Lys-Thr-Cys]D-Tyr-NH₂), [⁶⁸Ga]Ga-DOTA-LM3, [⁶⁸Ga]Ga-NODAGA-LM3, [¹¹¹In]In-DOTA-JR11 ([¹¹¹In]In-DOTA-Cpa-cyclo[D-Cys-Aph(Hor)-D-Aph(Cbm)-Lys-Thr-Cys]D-Tyr-NH₂), [¹¹¹In]In-DTPA-octreotide ([¹¹¹In]In-DTPA-D-Phe-cyclo(Cys-Phe-D-Trp-Lys-Thr-Cys)Thr(ol)), [¹¹¹In]In-DOTA-BASS ([¹¹¹In]In-DOTA-p-NO₂-Phe-cyclo-(D-Cys-Tyr-D-Trp-Lys-Thr-Cys)D-Tyr-NH₂, [⁹⁰Y]Y-DOTA-TOC ([⁹⁰Y]Y-DOTA-D-Phe-cyclo(Cys-Tyr-D-Trp-Lys-Thr-Cys)Thr(ol)), [⁹⁰Y]Y-DOTA-TATE ([⁹⁰Y]Y-DOTA-D-Phe-cyclo(Cys-Tyr-D-Trp-Lys-Thr-Cys)Thr).

### Integrin-targeting compounds

In some embodiments, the radiolabeled complex of the pharmaceutical composition according to the present invention may comprise a targeting moiety which targets an integrin.

Integrins are heterodimeric glycoproteins consisting of an α-subunit and a β-subunit. There are 24 different combinations of the eight β-units and the eighteen α-units known. The integrins mediate cell-cell and cell-matrix interactions and transduce signals across the plasma membrane via insight-out and outside-in signaling. Some of the integrins play an important role during migration of endothelial as well as tumor cells during tumor-induced angiogenesis and tumor metastasis. Angiogenesis, the formation of new blood vessels out of the preexisting vasculature, is a critical step in the development and dissemination of various human tumors. A variety of therapeutic strategies in oncology are focused on the inhibition of tumor-induced angiogenesis. Concerning the integrins, significant attention has been paid to the role of integrin *α*V*β*3, *α*V*β*5, and *α*V*β*6, as they are prominent on proliferating vascular endothelial cells. Thus, one of the most prominent target structures used for the development of radiopharmaceuticals for imaging angiogenesis is the integrin *α*V*β*3.

Tumor-induced angiogenesis can be blocked *in vivo* by antagonizing the *α*V*β*3 integrin with small peptides containing the Arg-Gly-Asp (RGD) amino acid sequence. This tripeptidic sequence, naturally present in extracellular matrix proteins, is the primary binding site of the αvβ3 integrin. Because of selective expression of αvβ3 integrin in tumors, radiolabeled RGD peptides are attractive candidates for αvβ3 integrin targeting in tumors. Over the last decade, many radiolabeled linear and cyclic RGD peptides have been evaluated as radiotracers for imaging tumors by SPECT or PET, as well as therapeutic agents.

Thus, the targeting molecule in the radiolabeled complex may be an integrin targeting molecule, which may be bound to a chelator molecule, as defined above, and complexed with a radionuclide, as defined above.

As a chelating moiety, any suitable chelating moiety, e.g. as specified above, can be used, wherein DOTA, DOTAGA, and Macropa are preferred.

An example of a conjugate comprising the RGD peptide as the targeting moiety and DOTA as the chelating moiety is DOTA-RGD according to Formula (15):
DOTA-RGD:

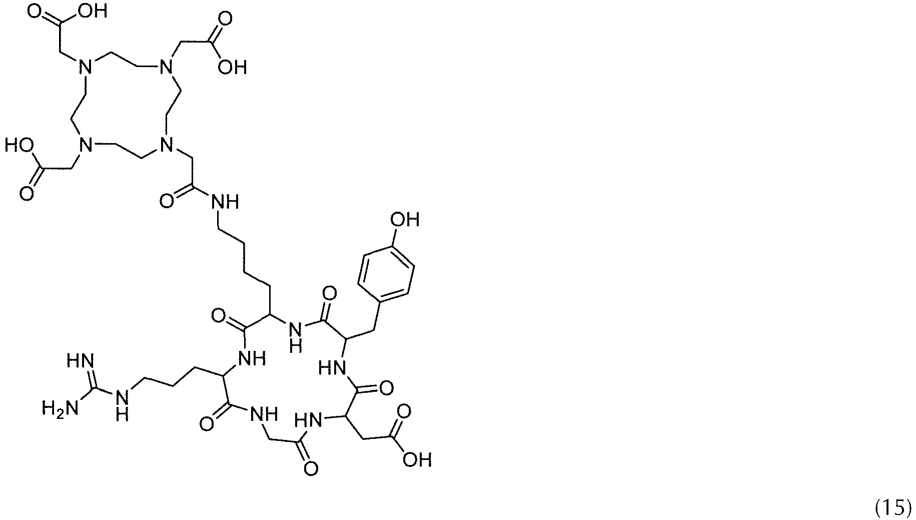

Suitable radionuclides complexed by the integrin targeting conjugates comprise the radionuclides specified above, and in particular comprise the radionuclides Lu-177, Tb-161, Ac-225, F-18, Tc-99m, Ga-68, In-111, I-131, Y-90, and Cu-67.

Thus, the radiolabeled complex may e.g. be selected from [¹⁷⁷Lu]Lu-DOTA-RGD, [¹⁶¹Tb]Tb-DOTA-RGD, and [²²⁵Ac]Ac-DOTA-RGD.

Further examples of integrin targeting radiolabeled complexes include, but are not limited to: [¹⁸F]F-Galacto-RGD, [^{99m}Tc]Tc-NC100692 ([^{99m}Tc]Tc-maracilatide), [¹⁸F]F-AH11185 ([¹⁸F]F-Fluciclatide), [¹⁸F]F-RGD-K5, [⁶⁸Ga]Ga-NOTA-RGD, [¹⁸F]F-FPPRGD2, Al[¹⁸F]F-NOTA-PRGD2 (¹⁸F-Alfatide), Al[¹⁸F]F-NOTA-E[PEG4-c(RGDfk)]₂ ([¹⁸F]F-Alfatide II), [⁶⁸Ga]Ga-NOTA-PRGD2, [⁶⁷Cu]Cu-cyclam-RAFT-c(-RGDfK-)₄, [¹¹¹In]In-DOTA-E-[c(RGDfK)₂, [^{99m}Tc]Tc-HYNIC-E-[c(RGDfK)]₂; cyclo(FRGDLAFp(AMe)K), [⁶⁸Ga]Ga-avebehexin, [⁶⁸Ga]Ga-Trivehexin.

### Folate conjugates

In some embodiments, the radiolabeled complex of the pharmaceutical composition according to the present invention may comprise a targeting moiety which targets the folate receptor.

Folate receptor-α attracted most interest as a tumor-associated target for targeted therapy concepts. Targeting of folate receptor-α-positive tumor cells *in vitro* and *in vivo* has been exemplified by a number of research groups using folic acid conjugates with a variety of therapeutic probes. The folate receptor has thus proven a valuable target for nuclear imaging and therapy using folic acid radioconjugates.

Thus, the targeting molecule in the radiolabeled complex of the pharmaceutical composition according to the present invention may be a folate receptor targeting molecule, which may be bound to a chelator molecule, as defined above, and complexed with a radionuclide, as defined above.

Conjugates based on 6*R*- or 6*S*-5-methyltetrahydrofolic acid (5-MTHF; a reduced folate version) as targeting entity can suitably be used in the radiolabeled complex of the pharmaceutical composition according to the present invention.

As a chelating moiety, any suitable chelating moiety, e.g. as specified above, can be used, wherein DOTA, DOTAGA, NOGADA, and DTPA are preferred.

An example of a conjugate comprising a folate moiety as the targeting moiety and DOTA as the chelating moiety is 6S-RedFol-25 according to Formula (16):
6S-RedFol-25:

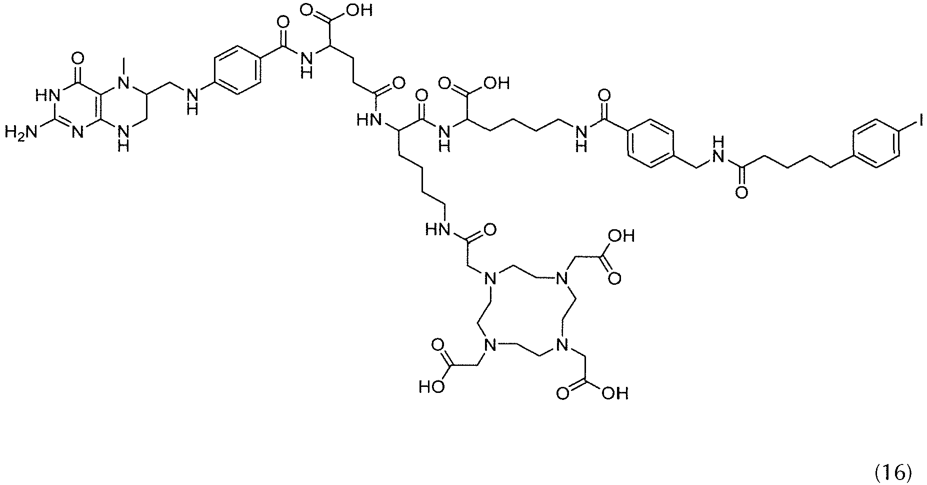

Further exemplary conjugates targeting the folate receptor are OxFol-1, 6R-RedFol-1, 6S-RedFol-1 (Patrycja Guzik, Martina Benešová, Magdalena Ratz, Josep M. Monné Rodríguez, Luisa M. Deberle, Roger Schibli, Cristina Müller: Preclinical evaluation of 5-methyltetrahydrofolate-based radioconjugates-new perspectives for folate receptor-targeted radionuclide therapy, European Journal of Nuclear Medicine and Molecular Imaging (2021) 48:972-983), as well as OxFol-14, 6*R*-RedFol-14, and 6*S*-RedFol-14 (Luisa M Deberle, Anna E Becker, Magdalena Ratz, Patrycja Guzik, Roger Schibli, Cristina Müller: Novel Synthetic Strategies Enable the Efficient Development of Folate Conjugates for Cancer Radiotheranostics, Bioconjug. Chem. 2021 Aug 18;32(8):1617-1628. doi: 10.1021/acs.bioconjchem.2c00198. Epub 2021 Jul 12).

Suitable radionuclides complexed by the folate receptor targeting conjugates comprise the radionuclides specified above, and in particular may comprise the radionuclides Lu-177, Tb-161, Ac-225, F-18, Tc-99m, Ga-66, Ga-67, and Ga-68.

Thus, the radiolabeled complex may be selected from [¹⁷⁷Lu]Lu-6S-RedFol-25, [¹⁶¹Tb]Tb-6S-RedFol-25, [²²⁵Ac]Ac-6S-RedFol-25, [¹⁷⁷ Lu]Lu-/[¹⁶¹Tb]Tb-/[²²⁵Ac]Ac-OxFol-1, [¹⁷⁷Lu]Lu-/[¹⁶¹Tb]Tb-/[²²⁵Ac]Ac-6R-RedFol-1, [¹⁷⁷Lu] Lu-/[¹⁶¹Tb]Tb-/[²²⁵Ac]Ac-6S-RedFol-1 , [¹⁷⁷Lu]Lu-/[¹⁶¹Tb]Tb-/[²²⁵Ac]Ac-OxFol-14, [¹⁷⁷Lu]Lu-/[¹⁶¹Tb]Tb-/[²²⁵Ac]Ac-6*R*-RedFol-14, and [¹⁷⁷Lu]Lu-/[¹⁶¹Tb]Tb-/[²²⁵Ac]Ac-6*S*-RedFol-14, for example.

Further representative folate conjugates which may be included in the pharmaceutical composition according to the present invention are e.g. [¹¹¹In]In-DTPA-folate, [¹⁷⁷Lu]Lu-EC0800, [¹⁷⁷Lu]Lu-cm09, [¹⁴⁹Tb]Tb-/[¹⁶¹Tb]Tb-cm09, [^{99m}Tc]Tc-(CO)₃, [^{99m}Tc]Tc-EC20, [¹¹¹In]In-DTPA-folate, [¹¹¹In]In-/[¹⁷⁷Lu]Lu-DOTA-click-folate, [⁶⁷Ga]Ga-DOTA-Bz-folate ([⁶⁷Ga]Ga-EC0800), and [⁶⁸Ga]Ga-NODAGA-folate.

### CCK2 receptor-targeting compounds

In some embodiments, the radiolabeled complex of the pharmaceutical composition according to the present invention may comprise a targeting moiety which targets the CCK2 receptor.

The CCK2 receptor (cholecystokinin) is located in areas of the central and peripheral nervous system and is overexpressed in several types of human cancer, as medullar thyroid carcinomas, small cell lung cancers and stromal ovarian carcinomas. Research has been done on developing suitable radioligands for targeting the CCK2-receptor *in vivo.* A variety of radiolabeled CCK/gastrin-related peptides has been synthesized and characterized. All peptides have the C-terminal CCK receptor-binding tetrapeptide sequence Trp-Met-Asp-Phe-NH₂ in common or derivatives thereof. The peptides can be categorized based on the sequence of their parent peptide (gastrin or CCK) and on their form (i.e. linear, cyclic, multimers).

Examples for CCK receptor ligands are gastrin analogs, such as Sargastrin (Gln-Gly-Pro-Trp-Leu-Glu-Glu-Glu-Glu-Glu-Ala-Tyr-Gly-Trp-Nle-Asp-Phe-NH₂), Minigastrin 0 (MG-0) D-Glu-(Glu)₅-Ala-Tyr-Gly-Trp-Met-Asp-Phe-NH₂), Minigastrin 11 (MG-11) (D-Glu-Ala-Tyr-Gly-Trp-Met-Asp-Phe-NH₂), cyclo-Minigastrin 1 (cyclo-MG1) (cyclo[γ-D-Glu-Ala-Tyr-D-Lys]-Trp-Met-Asp-Phe-NH₂), cyclo-Minigastrin 2 (cyclo-MG2) (cyclo[γ-D-Glu-Ala-Tyr-D-Lys]-Trp-Nle-Asp-Phe-NH₂, Demogastrin 1 (D-Glu-(Glu)₅-Ala-Tyr-Gly-Trp-Met-Asp-Phe-NH₂), Demogastrin 2 (D-Glu-(Glu)₅-Ala-Tyr-Gly-Trp-Met-Asp-Phe-NH₂, H2-Met (His-His-Glu-Ala-Tyr-Gly-Trp-Met-Asp-Phe-NH₂), H2-Nle (His-His-Glu-Ala-Tyr-Gly-Trp-Nle-Asp-Phe-NH₂), H6-Met (His)₆-Glu-Ala-Tyr-Gly-Trp-Met-Asp-Phe-NH₂); and CCK8 analogs, such as CCK8 (D-Asp-Tyr-Met-Gly-Trp-Met-Asp-Phe-NH₂), CCK8(Nle) (D-Asp-Tyr-Nle-Gly-Trp-Nle-Asp-Phe-NH₂), sCCK8 (D-Asp-Tyr(OSO₃H)-Met-Gly-Trp-Met-Asp-Phe-NH₂), sCCK8[Phe²(*p-*CH₂SO₃H), Nle^{3,6}] (D-Asp-Phe(p-CH₂SO₃H)-Nle-Gly-Trp-Nle-Asp-Phe-NH₂), sCCK8[Phe²(*p-*CH₂SO₃H), HPG^{3,6}] (D-Asp-Phe(*p*-CH₂SO₃H)-HPG-Gly-Trp-HPG-Asp-Phe-NH₂).

The CCK receptor targeting peptides are preferably radiolabeled with the radionuclides for imaging or therapeutic applications. Suitable radionuclides comprise the radionuclides specified above, and in particular comprise the radionuclides Tc-99m, In-111, F-18, Ga-68,I-131, Y-90, Tb-161 and Lu-177. As a chelator which allows radiolabeling with a radionuclide and which is conjugated to the peptide, the chelators specified above can be used, wherein DOTA, DOTAGA, DOTAM, DTPA and HYNIC are preferred.

Accordingly, the radiolabeled complex of the pharmaceutical composition according to the present invention may include a CCK2 receptor targeting molecule, such as [¹⁷⁷Lu]Lu-DOTA-Sargastrin, [¹¹¹In]In-DTPA-MG0, [¹¹¹In]In-DOTA-MG11, [¹¹¹In]In-DOTA-MG11 (Nle), [¹¹¹In]In-DOTA-H2-Met, [¹¹¹In]In-DOTA-H2-Nle, [¹¹¹In]In-DOTA-H6-Met, [^{99m}Tc]²N40, D-Glu1-MG (^{99m}Tc-Demogastrin 1), [^{99m}Tc]₂N₄⁰⁻¹,Gly⁰,D-Glu¹-MG (^{99m}Tc-Demogastrin 2), [^{99m}Tc]Tc-HYNIC-MG11, [^{99m}Tc]Tc-HYNIC-cyclo-MG1, [^{99m}Tc]Tc-HYNIC-cyclo-MG2; and CCK8 analogs, such as [¹¹¹In]In-DTPA-CCK8, [¹¹¹In]In-DTPA-CCK8(Nle), [^{99m}Tc]Tc-HYNIC-CCK8, [^{99m}Tc]Tc-HYNIC-sCCK8, [¹¹¹In]In-DOTA-sCCK8[Phe²(*p*-CH₂SO₃H), Nle^{3,6}], and [¹¹¹In]In-DOTA-sCCK8[Phe²(*p*-CH₂SO₃H), HPG^{3,6}].

### Neurotensin receptor 1-targeting compounds

In some embodiments, the radiolabeled complex of the pharmaceutical composition according to the present invention may comprise a targeting moiety which targets the Neurotensin receptor 1. Neurotensin receptor 1 (NTR1) is overexpressed in ductal pancreatic adenocarcinoma, which is one of the deadliest cancers.

Several NTR1 antagonists have been developed, such as SR142948A and SR48692. [¹⁷⁷Lu]Lu-3BP-2273, which is a ¹⁷⁷Lu-labeled DOTA-conjugated NTR1 antagonist that has been developed on the basis of SR142948A, has been used for the treatment of ductal pancreatic adenocarcinoma (Baum RP et al., The Journal of Nuclear Medicine, Vol. 59, No. 5, May 2018).

Therefore, the pharmaceutical composition according to the present invention may comprise a radiolabeled complex comprising a targeting moiety targeting the Neurotensin receptor 1, in particular a radiolabeled complex comprising, as a targeting moiety, NTR1 antagonists for cancer diagnosis or therapy, preferably ¹⁷⁷Lu- or ⁶⁸Ga-labeled NTR1 antagonists, more preferably [¹⁷⁷Lu]Lu-3BP-2273, even though other radionuclides, for example the radionuclides mentioned above, as well as other chelators, for example the chelators mentioned above, may be contemplated.

### Glucagon-like peptide-1 (GLP-1) receptor targeting compounds

In some embodiments, the radiolabeled complex of the pharmaceutical composition according to the present invention may comprise a targeting moiety which targets the Glucagon-like peptide-1 (GLP-1) receptor.

The GLP-1 receptor is overexpressed on essentially all benign insulinomas and also on gastrinomas. Benign insulinomas which emerge from β-cells of the pancreas and are present as small nodules, secrete insulin leading to potentially life-threatening hypoglycemia.

Non-limiting examples of radiopharmaceuticals targeting the GLP-1 receptor include ¹¹¹In-, ^{99m}Tc- and ⁶⁸Ga-labeled peptides based on the 39-mer peptide exendin-4, such as Lys⁴⁰(Ahx-DOTA-[¹¹¹In]In)NH₂-exendin-4, for example. However, other radionuclides, for example the radionuclides mentioned above, as well as other chelators, for example the chelators mentioned above, may be contemplated.

### Gastrin releasing peptide (GRP) receptor targeting compounds

In some embodiments, the radiolabeled complex of the pharmaceutical composition according to the present invention may comprise a targeting moiety which targets the GRP receptor.

GRP receptors have been demonstrated in major human tumors, such as breast cancer and prostate cancer. Bombesin is a tetradecapeptide neurohormone and an amphibian homolog of mammalian GRP (a 27mer peptide). Several bombesin analogs and bombesin antagonists have been developed and labeled with different radioisotopes (e.g. Ga-68, Cu-64, F-18) using different chelators. Examples thereof include a pan-bombesin analog ⁶⁸Ga-BZH3 (Zhang H et al., Cancer Res 2004; 64: 6707-6715), and a ¹⁷⁷Lu-labeled bombesin (7-14) derivative coupled to DOTA via a Gly-4-aminobenzoyl spacer (Bodei L et al., Eur J Nucl Med Mol Imaging 2007: 34(suppl 2): S221).

However, the radiolabeled complex of the pharmaceutical composition according to the present invention targeting the GRP receptor may comprise other radionuclides, for example the radionuclides mentioned above, as well as other chelators, for example the chelators mentioned above.

### Neurokinin type 1 receptor targeting compounds

In some embodiments, the radiolabeled complex of the pharmaceutical composition according to the present invention may comprise a targeting moiety which targets the neurokinin type 1 receptor.

The neurokinin type 1 receptor is consistently overexpressed on glioma cells and on tumor vessels (Hennig IM et al., Int J Cancer 1995; 61: 786-792). The radiolabeled 11-amino-acid peptide substance P (Arg Pro Lys Pro Gln Gln Phe Phe Gly Leu Met) acting via the neurokinin type 1 receptor can suitably be used to target malignant gliomas. In particular, substance P has been conjugated to the chelator DOTAGA, and ⁹⁰Y-labeled DOTAGA-substance P has been used in clinically studies (Kneifel S et al., Eur J Nucl Med Mol Imaging. 2007; 34: 1388-1395. In another study, the feasibility and effectiveness of targeted α-radionuclide therapy for brain tumors was assessed using the α-radiation-emitting conjugate [²¹³Bi]Bi-DOTA-[THi⁸,Met(O₂)¹¹]-substance P (Cordier et al., Eur J Nucl Med Mol Imaging. 2010; 37: 1335-1344).

However, the radiolabeled complex of the pharmaceutical composition according to the present invention targeting the neurokinin type 1 receptor, e.g. a substance P conjugate, may comprise other radionuclides, for example the radionuclides mentioned above, as well as other chelators, for example the chelators mentioned above.

### Fibroblast activation protein-α (FAP)

Fibroblast activation protein-α (FAP) is a specific marker of cancer-associated fibroblasts (CAFs) and plays a crucial role in tumor development (Chao Ma, Shuaishuai Xi, He Sun, Meng Zhang, Yuanmin Pei: Identifying the oncogenic roles of FAP in human cancers based on systematic analysis, AGING 2023, Vol. 15, No. 14

Thus, in some embodiments, the radiolabeled complex of the pharmaceutical composition according to the present invention may comprise a targeting moiety which targets the fibroblast activation protein (FAP). Several cyclic and linear peptides are known that bind to FAP, such as FAP-2286 (Zboralski et al.: Preclinical evaluation of FAP-2286 for fibroblast activation protein targeted radionuclide imaging and therapy; European Journal of Nuclear Medicine and Molecular Imaging (2022) 49:3651-3667), and 3BP-3940 (Greifenstein et al.: 3BP-3940, a highly potent FAP-targeting peptide for theranostics - production, validation and first in human experience with Ga-68 and Lu-177; iScience, Volume 26, Issue 12, 15 December 2023, 108541), for example.

### Glypican-3

Glypican-3 (GPC3) is a membrane-associated proteoglycan that is specifically up-regulated in hepatocellular carcinoma (HCC) although rarely or not expressed in normal liver tissues, making it a perfect diagnostic and treatment target for HCC (Xiufeng Zheng, Xun Liu, Yanna Lei, Gang Wang, Ming Liu: Glypican-3: A Novel and Promising Target for the Treatment of Hepatocellular Carcinoma; Front Oncol. 2022 Feb 16:12:824208. doi: 10.3389/fonc.2022.824208. eCollection 2022.)

Thus, in some embodiments, the radiolabeled complex of the pharmaceutical composition according to the present invention may comprise a targeting moiety which targets Glypican-3 (GPC3). Several cyclic and linear peptides are known that bind to GPC3 (Wenzhu Hu, et al.: Preclinical evaluation of GPC3-targeting peptides for PET imaging of hepatocellular carcinoma; Journal of Nuclear Medicine June 2024, 65 (supplement 2) 241870).

DOTA-RYZ-GPC3 (RAYZ-8009), for example, which comprises a novel macrocyclic peptide binder to GPC3, a linker, and a chelator that can be complexed with different radioisotopes showed high binding affinity to GPC3 (Fanching Lin et al.: Peptide Binder to Glypican-3 as a Theranostic Agent for Hepatocellular Carcinoma; Journal of Nuclear Medicine, published on February 29, 2024 as doi:10.2967/jnumed.123.266766).

Further, the radiolabeled complex of the pharmaceutical composition according to the present invention may comprise cyclic and linear peptides, and small molecule radiopharmaceuticals targeting Nectin-4 (Kaiyue Li, et al.: Therapeutic prospects of nectin-4 in cancer: applications and value; Front. Oncol., 28 March 2024, Sec. Cancer Molecular Targets and Therapeutics, Volume 14 - 2024, https://doi.org/10.3389/fonc.2024.1354543); delta-like ligand 3 (DLL3) (Annarita Peddio et al.: DLL3 as a potential diagnostic and therapeutic target in neuroendocrine neoplasms: A narrative review; Critical reviews in Oncology/Hematology; Volume 204, December 2024, 104524); Trophoblast cell surface antigen 2 (Trop2) (Xinlin Liu et al.: Trop2-targeted therapies in solid tumors: advances and future directions; Theranostics 2024 Jun 11;14(9):3674-3692. doi: 10.7150/thno.98178; WO/2024/049046); IL-13Rα2 (Knudson K. et al.: Recent Advances in IL-13Rα2-Directed Cancer Immunotherapy; Front. Immunol., 08 April 2022, Sec. Cancer Immunity and Immunotherapy, Volume 13 - 2022, https://doi.org/10.3389/fimmu.2022.878365); B7H3 (Elodie Picarda, Kim C Ohaegbulam, Xingxing Zang: Molecular Pathways: Targeting B7-H3 (CD276) for Human Cancer Immunotherapy; Clin Cancer Res. 2016 May 20;22(14):3425-3431. doi: 10.1158/1078-0432.CCR-15-2428); CAIX (Michael S. Hofman et al.: First-inHuman Safety, Imaging, and Dosimetry of a Carbonic Anhydrase IX-Targeting Peptide, [68Ga]Ga-DPI-4452, in Patients with Clear Cell Renal Cell Carcinoma, Journal of Nuclear Medicine February 2024, jnumed.123.267175; DOl: https://doi.org/10.2967/jnumed.123.2671 75.

Thus, in some embodiments, the radiolabeled complex of the pharmaceutical composition according to the present invention may comprise a targeting moiety which targets the above molecules, and in particular may comprise cyclic and linear peptides, and small molecule radiopharmaceuticals that bind to cancer cells.

### Affilins

In some embodiments, the radiolabeled complex of the pharmaceutical composition according to the present invention may comprise antibody mimetics, such as affilins.

Affilins are artificial proteins designed to selectively bind antigens. Affilin proteins are structurally derived from human ubiquitin or gamma-B crystallin, respectively. Affilin proteins are constructed by modification of surface-exposed amino acids of these proteins and isolated by display techniques such as phage display and screening. They resemble antibodies in their affinity and specificity to antigens but not in structure, which makes them a type of antibody mimetic. Affilin^{®} was developed by Scil Proteins GmbH as potential biopharmaceutical drugs, diagnostics and affinity ligands. Radiolabeled Affilin molecules can be easily modified and are suitable to kill tumor cells specifically by irradiation.

Multispecific Affilin molecules can be generated, binding different targets simultaneously. Radionuclides can be conjugated to Affilin proteins, making them potential tumor therapeutics and diagnostics. Radionuclide-chelator-Affilin conjugates, e.g. ¹⁷⁷Lu-DOTA-Affilin, have been designed for therapy purposes. Further Affilin conjugates comprising other radionuclides (for example as specified above) and chelators (for example as specified above), respectively, may be contemplated.

Preferably, the radiolabeled complex of the pharmaceutical composition according to the present invention targets cell surface molecules, such as PSMA, a somatostatin receptor, a folate receptor or integrin. Accordingly, the targeting moiety of the radiolabeled complex is preferably able to bind to PSMA, a somatostatin receptor, a folate receptor or integrin, e.g. as described above.

The targeting moiety may be either directly or indirectly (e.g., by using linkers or spacers) linked to the chelating agent. Preferably, the linking bond(s) is/are covalent bond(s) between the targeting molecule, optionally the linker or spacer, and the chelating agent. Particularly suitable linkers and spacers are described in WO 2018/215627 A1, which is incorporated herein by reference, and in WO 2020/109523 A1, which is also incorporated herein by reference.

The concentration of the sum of radiolabeled complex (comprising (i) the radionuclide, e.g. Lu-177, and (ii) the targeting moiety covalently linked to the chelating moiety), and the precursor (non-radioactive) in the pharmaceutical composition is preferably in a range from about 1 µg/mL to about 100 µg/mL, preferably from about 5 µg/mL to about 50 µg/mL, more preferably from about 7 µg/mL to about 30 µg/mL.

Further, for alpha-emitting radionuclides, such as Ac-225, the radionuclide is preferably present in the pharmaceutical composition at a concentration providing radioactivity of about 0.05 MBq/mL to about 4.0 MBq/mL, preferably of about 0.1 to about 2.0 MBq/mL, more preferably of about 0.2 to about 1.0 MBq/mL.

For beta-emitting radionuclides, such as Lu-177 or Tb-161, the radionuclide/radiolabeled complex is preferably present in the pharmaceutical composition at a concentration providing radioactivity of about 0.05 GBq/mL to about 4.0 GBq/mL, preferably of about 0.1 to about 2.0 GBq/mL, more preferably of about 0.3 to about 1.0 GBq/mL).

The radioactivity per amount of substance contained in the pharmaceutical composition depends to a large extent on the radionuclide and precursor (targeting moiety covalently linked to the chelating moiety) used in the radiolabeling step of the method for preparing the pharmaceutical composition.

For example, provided that 100-400 µg precursor are labeled with 1-6 MBq Ac-225, Ac-225 may be present in the pharmaceutical composition in an amount of about 0.0025 MBq/pg to about 0.06 MBq/pg (depending on the respective radiolabeled precursor).

To give another example, provided that 100-400 pg precursor are labeled with 1000-8000 MBq Lu-177, Lu-177 may be present in the pharmaceutical composition in an amount of about 2.5 MBq/µg to about 80 MBq/pg.

As another example, provided that 100-400 pg precursor are labeled with 500-5000 MBq Tb-161, Tb-161 may be present in the pharmaceutical composition in an amount of about 1.25 MBq/pg to about 50 MBq/pg.

As still another example, provided that 10-100 µg precursor are labeled with 100-250 MBq Ga-68, Ga-68 may be present in the pharmaceutical composition in an amount of about 1 MBq/pg to about 25 MBq/pg.

### Stabilizer

In order to ensure stability of the radiolabeled complex against radiolytic degradation, the pharmaceutical composition according to the present invention comprises a stabilizer. As used herein, the term "stabilizer" (against radiolytic degradation or radiolysis) refers to an agent which protects organic molecules against radiolytic degradation. In particular, the stabilizer may be able to scavenge radicals, which may be generated, for example, when radiation emitted by the radionuclides cleaves a bond between the atoms of organic molecules, thereby forming radicals. Therefore, the stabilizer can avoid or reduce that those radicals undergo other chemical reactions, which might lead to undesired, potentially ineffective or even toxic molecules. The present invention thus envisages to protect the radiolabeled complex as the active principle of the composition against radiolytic degradation or radiolysis as well as the non-radioactive targeting moiety linked to the chelating moiety to maintain the radiochemical purity and chemical purity of such.

### Para-aminohippuric acid (PAH)

The stabilizer included in the pharmaceutical composition according to the present invention comprises para-aminohippuric acid or a pharmaceutically acceptable salt or derivative thereof according to Formula (1): 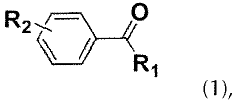 wherein R₁ is selected from the group consisting of H, OH, NH₂, an alanine residue, and a glycine residue, and
R₂ is selected from the group consisting of H, OH, NH₂, F, Cl, Br, I, SO₂, CH₃ (methyl), O-CH₃ (O-methyl), and O-C₂H₅ (O-ethyl).

Aminohippuric acid or para-aminohippuric acid, a derivative of hippuric acid, is an amide derivative of the amino acid glycine and para-aminobenzoic acid that is not naturally found in humans. They are covalently linked by an amide bond. The structural formula of para-aminohippuric acid is shown in Formula (17):

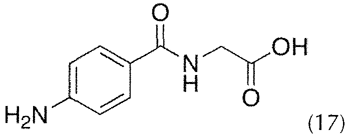

Preferred salts of para-aminohippuric acid include alkaline or alkaline earth salts of para-aminohippuric acid. The sodium salt of para-aminohippuric acid, sodium para-aminohippurate, is particularly preferred. It is understood that the salt of para-aminohippuric acid is usually a pharmaceutically acceptable salt of para-aminohippuric acid, in particular a salt which is not toxic. Para-aminohippuric acid's sodium salt, aminohippurate sodium, is a known diagnostic agent which is widely used in diagnostic testing of the kidney function, in particular for measuring renal plasma flow. The structural formula of sodium para-aminohippurate is shown in Formula (18):

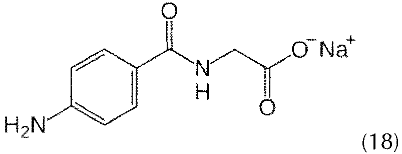

As used herein, the terms "aminohippuric acid", "para-aminohippuric acid" and "PAH" are used synonymously and generally refer to para-aminohippuric acid, salts thereof (aminohippurate salt, in particularalkaline or alkaline earth salt, such as the sodium salt) and (carboxylic acid) derivates thereof, unless specifically stated otherwise. Preferably, the terms "aminohippuric acid", "para-aminohippuric acid" and "PAH" refer to para-aminohippuric acid and salts thereof (aminohippurate salt, in particular alkaline or alkaline earth salt, such as the sodium salt).

"Carboxylic acid derivatives" of para-aminohippuric acid are derivatives, which retain the "carboxylic acid" moiety of the aminoacetic acid (glycine) group covalently linked to the benzoyl moiety via an amide bond. Typically, such "carboxylic acid derivatives" are thus hippuric acid or hippuric acid derivatives, which exhibit a substitution pattern at the phenyl ring system other than one single amino substituent at the para ring position (corresponding to para-aminohippuric acid).

In some embodiments, the "carboxylic acid derivative" of para-aminohippuric acid may be hippuric acid or a hippuric acid derivatized with one or more substitutents selected from the group consisting of NH₂, I, Cl and CH₃ (other than PAH). Thus, in some embodiments, the "carboxylic acid derivative" of para-aminohippuric acid may be a compound according to Formula (1): 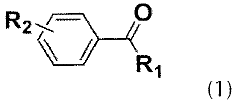 wherein R₁ is an alanine residue, and R₂ is selected from the group consisting of NH₂, Cl, I, and CH₃.

The "carboxylic acid derivative" may e.g. be an aminohippuric acid with an amino substituent at a ring position other than the para ring position (corresponding to para-aminohippuric acid), with two or more amino substitutents or with one or more of I, Cl and CH₃. A more specific group of such "carboxylic acid derivatives" may be hippuric acid, p-methylhippuric acid, orthochlorohippuric acid and orthoiodohippuric acid. Preferred, non-limiting examples of carboxylic acid derivatives of para-aminohippuric acid include hippuric acid and orthoiodohippuric acid.

Para-aminohippuric acid or a pharmaceutically acceptable salt or carboxylic derivative thereof is present in the pharmaceutical composition in a concentration of 1 mg/mL to 300 mg/mL.

Preferably, the concentration of para-aminohippuric acid or of a pharmaceutically acceptable salt or carboxylic derivative thereof in the pharmaceutical composition according to the present invention is in the range from about 10 mg/mL to about 300 mg/mL, more preferably from about 20 mg/mL to about 300 mg/mL, still more preferably from about 30 mg/mL to about 250 mg/mL, most preferably from about 50 mg/mL to about 200 mg/mL, such as about 50 mg/mL, 60 mg/mL, 70 mg/mL, 80 mg/mL, 90 mg/mL, 100 mg/mL, 110 mg/mL, 120 mg/mL, 130 mg/mL, 140 mg/mL, 150 mg/mL, 160 mg/mL, 170 mg/mL, 180 mg/mL, 190 mg/mL, and 200 mg/mL.

As shown in the Examples below, such a formulation comprising para-aminohippuric acid or a pharmaceutically acceptable salt thereof in concentrations as defined above, not only decreases the complexity of the composition (and its preparation), but surprisingly exhibits long-term stability of the radiolabeled complex, in particular a low decrease of the radiochemical purity of less than 2% over at least 96 hours.

In some embodiments, para-aminohippuric acid or a pharmaceutically acceptable salt or carboxylic derivative thereof is the only stabilizer comprised by the pharmaceutical composition. In other embodiments, in addition to para-aminohippuric acid or a pharmaceutically acceptable salt or carboxylic derivative thereof, other stabilizers may be present in the pharmaceutical composition according to the present invention. Examples of such further stabilizers include sodium acetate (NaOAc), ascorbic acid and/or a salt thereof, ethanol, gentisic acid, methionine, histidine, melatonine, and Se-methionine.

In a particular embodiment, the pharmaceutical composition comprises, in addition to para-aminohippuric acid (or a pharmaceutically acceptable salt or carboxylic derivative thereof), sodium acetate (NaOAc). In a further particular embodiment, the pharmaceutical composition comprises, in addition to para-aminohippuric acid (or a pharmaceutically acceptable salt or carboxylic derivative thereof) ascorbic acid, and/or a salt thereof.

Preferred salts of ascorbic acid include the alkaline salts of ascorbic acid. The term "alkali salt" refers to salts that produce hydroxide ions when dissolved in water. Non-limiting examples of preferred salts of ascorbic acid include sodium, potassium, calcium, magnesium and lithium salts of ascorbic acid; such as sodium ascorbate, sodium ascorbyl phosphate, potassium ascorbate, calcium ascorbate, magnesium ascorbate, magnesium ascorbyl phosphate and lithium ascorbate. Most preferably, the salt of ascorbic acid is a sodium salt of ascorbic acid, in particular sodium ascorbate.

The concentration of further stabilizers in the pharmaceutical composition according to the present invention, such as ethanol, sodium acetate (NaOAc), ascorbic acid, and/or sodium ascorbate, may e.g. be in a range from 1 mg/mL to 100 mg/mL, preferably in the range from 10 mg/mL to 90 mg/mL, more preferably in the range from 10 mg/mL to 80 mg/mL, still more preferably in the range from 10 mg/mL to 70 mg/mL, still more preferably in the range from 10 mg/mL to 60 mg/mL, particular preferably in the range from 10 mg/mL to 50 mg/mL, most preferably in the range from 10 mg/mL to 40 mg/mL.

In a particular embodiment, the pharmaceutical composition according to the present invention optionally comprises both, ascorbic acid and sodium ascorbate, as further stabilizers of the pharmaceutical composition, wherein the concentration of ascorbic acid and sodium ascorbate in the pharmaceutical composition preferably does not exceed 40 mg/mL.

In a particular embodiment, the pharmaceutical composition according to the present invention comprises, optionally in addition to a further stabilizer, such as NaOAc, ascorbic acid/sodium ascorbate, ethanol as a further stabilizer. Ethanol may present in the pharmaceutical composition in an amount of about 0,1% to 10% (v/v), particularly in an amount of 1% to 8% (v/v).

However, the present inventors found that, apart from para-aminohippuric acid or a pharmaceutically acceptable salt or carboxylic derivative thereof, no further ingredient was needed to stabilize the formulation of the pharmaceutical composition. Thus, in a particular embodiment, in addition to para-aminohippuric acid or a pharmaceutically acceptable salt or carboxylic derivative thereof, the pharmaceutical compositions according to the present invention essentially contain no other stabilizer(s) or, if so, only in minor amounts due to the presence of stabilizers employed by the labeling reaction, as will be described later. However, in some cases, a pH modulator, such as NaOH or HCl, may be required, also depending on the nature of the radiolabeled complex contained in the pharmaceutical composition.

Therefore, stabilizers other than para-aminohippuric acid or a pharmaceutically acceptable salt or carboxylic derivative thereof, which originate from the labeling reaction of the precursor, such as sodium acetate and/or ascorbic acid/sodium ascorbate, may still be present in the pharmaceutical composition, preferably only in minor amounts of e.g. ≤ 40 mg/mL, preferably of ≤ 10 mg/mL, or ≤ 5 mg/mL.

In some embodiments, the pharmaceutical composition according to the present invention comprising para-aminohippuric acid or a pharmaceutically acceptable salt or carboxylic derivative thereof may comprise a sequestering agent, such as diethylene triamine pentaacetic acid (DTPA) or a salt thereof. As used herein, the term "sequestering agent" refers to an agent suitable to complex/chelate traces of unreacted radionuclide metal ions. For example, the pharmaceutical composition may comprise the sequestering agent, such as DTPA, in a concentration of 0.01 to 1 mM, e.g. 0.1 mM.

The pharmaceutical composition according to the present invention is preferably an aqueous solution, in particular a radiopharmaceutical aqueous solution. As used herein, an "aqueous solution" is usually a solution of one or more solute(s) in water.

In some embodiments, the pharmaceutical composition may comprise a further buffer, e.g. a citrate buffer or a phosphate buffer. However, in some embodiments, the pharmaceutical composition does not contain any buffer in addition to para-aminohippuric acid (or a pharmaceutically acceptable salt or carboxylic derivative thereof), which is present as a stabilizer and also provides buffering functionality.

Thus, in some embodiments, the pharmaceutical composition may consist essentially of an aqueous solution comprising the radiolabeled complex as described herein, para-aminohippuric acid (or a pharmaceutically acceptable salt or carboxylic derivative thereof), and, optionally, (a) further stabilizer(s), such as sodium acetate, ascorbic acid/sodium ascorbate, and/or ethanol, which also may originate from the labeling reaction. In case they occur in the pharmaceutical composition as a result of the labeling reaction only, their amounts are minor, e.g. ≤ 40 mg/mL, preferably ≤ 10 mg//mL, or ≤ 5 mg/mL.

In a particular embodiments, the pharmaceutical composition may comprise
(a) a radiolabeled complex, as defined above, comprising a radionuclide, as defined above, and a targeting moiety, as defined above, covalently linked to a chelating moiety, as defined above;
(b) a stabilizer against radiolytic degradation of the radiolabeled complex comprising 1 to 300 mg/mL para-aminohippuric acid (or a pharmaceutically acceptable salt or carboxylic derivative thereof);
(c) water;
(d) optionally buffer (as defined herein); and
(e) optionally precursor (without radionuclide) of the radiolabeled complex.

As mentioned above, stabilizers originating from the labeling reaction, such as sodium acetate, and/or ascorbic acid/sodium ascorbate, ethanol, may present in the pharmaceutical composition in minor amounts. Also NaOH, HCl or other pH modulators may be present in the pharmaceutical composition. Since, in the labeling reaction, the radionuclide usually comes with a counter ion such as Cl⁻, or NO₃²⁻, such counter ions may be present in the pharmaceutical composition. Also, the precursor can be used in the labeling reaction in form of a salt, such as a chloride, actetate, TFA salt. Ions originating from those (or other) salts may also be present in the final pharmaceutical composition.

Thus, in a particular embodiment, the pharmaceutical composition may comprise
(a) a radiolabeled complex, as defined above, e.g. an ²²⁵Ac-labeled complex targeting a cell surface molecule, such as e.g. PSMA, a somatostatin receptor, a folate receptor or integrin;
(b) a stabilizer against radiolytic degradation of the radiolabeled complex comprising 1 to 300 mg/mL para-aminohippuric acid (or a pharmaceutically acceptable salt or carboxylic derivative thereof);
(c) water;
(d) optionally buffer (as defined herein); and
(e) optionally precursor (without radionuclide) of the radiolabeled complex.

For alpha-emitting radionuclides, such as Ac-225, the pharmaceutical composition may ensure radiolysis-stable radioactivity concentrations of 0.1 to 2.0 MBq/mL, preferably of 0.2 to 1.0 MBq/mL.

In another particular embodiment, the pharmaceutical composition may comprise
(a) a radiolabeled complex, as defined above, e.g. a ¹⁶¹Tb-labeled complex targeting a cell surface molecule, such as e.g. PSMA, a somatostatin receptor, a folate receptor or integrin;
(b) a stabilizer comprising 1 to 300 mg/mL para-aminohippuric acid (or a pharmaceutically acceptable salt or carboxylic derivative thereof);
(c) water;
(d) optionally buffer (as defined herein); and
(e) optionally precursor (without radionuclide) of the radiolabeled complex.

In a further particular embodiment, the pharmaceutical composition may comprise
(a) a radiolabeled complex, as defined above, e.g. a ¹⁷⁷Lu-labeled complex targeting a cell surface molecule, such as e.g. PSMA, a somatostatin receptor, a folate receptor or integrin;
(b) a stabilizer comprising 1 to 300 mg/mL para-aminohippuric acid (or a pharmaceutically acceptable salt or carboxylic derivative thereof);
(c) water;
(d) optionally buffer (as defined herein); and
(e) optionally precursor (without radionuclide) of the radiolabeled complex.

For beta-emitting radionuclides, such as Tb-161 or Lu-177, the pharmaceutical composition may ensure radiolysis-stable radioactivity concentrations of 0.1 to 2.0 GBq/mL, preferably of 0.3 to 1.0 GBq/mL.

In the pharmaceutical composition according to the present invention, the amount of free radionuclides in the pharmaceutical composition preferably is ≤0.5%, preferably ≤0.4%, more preferably ≤0.3%, or even ≤0.2%, after 72h, preferably after 96 h.

As shown in the appended examples, the pharmaceutical compositions according to the present invention comprising para-aminohippuric acid (or a pharmaceutically acceptable salt thereof) as a stabilizer provide a radiochemical purity of the radiolabeled compounds of ≥ 95% for at least 96 hours, in particular when stored at RT. For example, for various formulations according to the present invention as described herein a radiochemical purity of at least 95% and even up to >99% and ≤0.3% uncomplexed "free" radionuclides was found after 96 hours at RT.

For various formulations according to the present invention comprising para-aminohippuric acid (or a pharmaceutically acceptable salt thereof) as a stabilizer a radiochemical purity of the radiolabeled compound of ≥ 95% for at least 240 hours was found, even when stored at 40°C.

Radiochemical purity may be determined by radio TLC or HPLC as known in the art and described in the Examples.

The PAH stabilized pharmaceutical compositions according to the present invention ensure radiolysis-stable radioactivity concentrations of 0.2-1.0 MBq/mL for alpha-emitting radionuclides, and radiolysis-stable radioactivity concentrations of 0.3-1.0 GBq/mL for beta-emitting radionuclides, thereby guaranteeing radiolysis-stable volumes of 1-20 mL.

According to preferred embodiments, the pharmaceutical composition is administered parenterally, in particular via intravenous or intratumoral injection, and accordingly may be formulated in liquid or lyophilized form for parenteral administration. Parenteral formulations may be stored in vials, IV bags, ampoules, cartridges, or prefilled syringes and can be administered as injections, inhalants, or aerosols, with injections being preferred.

Liquid pharmaceutical compositions administered via injection and in particular via intravasal, more preferably intravenous (i.v.) injection should preferably be sterile and stable under the conditions of manufacture and storage. Such compositions are typically formulated as parenterally acceptable aqueous solutions that are pyrogen-free, have suitable pH, are isotonic and maintain stability of the active ingredient(s).

In particular embodiments, the pharmaceutical composition may be formulated to include a pharmaceutically acceptable excipient, diluent or carrier. The term "pharmaceutically acceptable", as used herein, refers to a compound or agent that is compatible with the components of the pharmaceutical composition, in particular the active (anti-cancer) compounds, and does not interfere with and/or substantially reduce its therapeutic activities. Pharmaceutically acceptable carriers preferably have sufficiently high purity and sufficiently low toxicity to make them suitable for administration to a subject to be treated.

The sodium, calcium and, optionally, potassium salts may occur in the form of their halogenides, e.g. chlorides, iodides, or bromides; in the form of their hydroxides, carbonates, hydrogen carbonates, or sulfates, etc. Without being limited thereto, examples of sodium salts include e.g. NaCl, Nal, NaBr, Na₂CO₃, NaHCO₃, Na₂SO₄, examples of the optional potassium salts include e.g. KCI, KI, KBr, K₂CO₃, KHCO₃, K₂SO₄, and examples of calcium salts include e.g. CaCl₂, Cal₂, CaBr₂, CaCO₃, CaSO₄, Ca(OH)₂. Furthermore, organic anions of the aforementioned cations may be contained in the buffer.

Buffers suitable for injection purposes as defined above, may contain salts selected from sodium chloride (NaCl), calcium chloride (CaCl₂) and optionally potassium chloride (KCl), wherein further anions may be present additional to the chlorides. Typically, the salts in the injection buffer are present in a concentration of at least 50 mM sodium chloride (NaCl), at least 3 mM potassium chloride (KCI) and at least 0.01 mM calcium chloride (CaCl₂). The injection buffer may be hypertonic, isotonic or hypotonic with reference to the specific reference medium, i.e. the buffer may have a higher, identical or lower salt content with reference to the specific reference medium, wherein preferably such concentrations of the afore mentioned salts may be used, which do not lead to damage of cells due to osmosis or other concentration effects.

The pharmaceutical formulations may be provided in lyophilized form. Lyophilized pharmaceutical compositions are preferably reconstituted in water (WFI) or a suitable buffer, e.g. PAH, advantageously based on an aqueous carrier, prior to administration.

The pharmaceutical compositions are also provided for use in a method of treatment, e.g. as medicament, or of diagnosis, e.g. for the treatment or diagnosis of cancer, in particular malignant cancer diseases. Also disclosed is the use of a radiolabeled complex and a stabilizer as described herein for the preparation of a pharmaceutical composition, e.g. for therapy or diagnosis of cancer.

### Medical treatment and uses

The present invention provides the pharmaceutical composition as described above for use in medicine. For example, the pharmaceutical composition as described above may be preferably used in the treatment or in the (*in vitro or in vivo*) diagnosis of cancer (e.g., by using an isolated sample, for example a blood sample or tumor tissue). Accordingly, the present invention also provides a method for treating cancer or initiating, enhancing or prolonging an anti-tumor-response in a subject in need thereof comprising administering to the subject the pharmaceutical composition as described above.

It is understood that for medical purposes, the pharmaceutical composition usually comprises an effective amount of the radiolabeled complex. As used herein, an "effective amount" means an amount of the agent(s) that is sufficient to allow for diagnosis and/or significantly induce a positive modification of the disease to be treated. At the same time, however, an "effective amount" may be small enough to avoid serious side-effects, i.e. to say to permit a sensible relationship between advantage and risk. An "effective amount" may vary depending on the particular condition to be diagnosed or treated and also with the age and physical condition of the patient to be treated, the severity of the condition, the duration of the treatment, the nature of the accompanying therapy, of the particular pharmaceutically acceptable excipient or carrier used, and similar factors. Accordingly, an "effective amount" may be readily determined in a specific situation by the physician. In general, effective doses may be determined by routine experiments, e.g. by using animal models. Such models include, without implying any limitation, rabbit, sheep, mouse, rat, dog and non-human primate models. Therapeutic efficacy and toxicity of radiolabeled complexes can be determined by standard pharmaceutical procedures in cell cultures or experimental animals, e.g., for determining the LD50 (the dose lethal to 50% of the population) and the ED50 (the dose therapeutically effective in 50% of the population). The dose ratio between toxic and therapeutic effects is the therapeutic index and can be expressed as the ratio LD50/ED50. The data obtained from the cell culture assays and animal studies can be used in determining a dose range for use in humans. The dose of said conjugates lies preferably within a range of circulating concentrations that include the ED50 with little or no toxicity.

As used herein, the term "diagnosis" or "diagnosing" refers to identifying a disease from its signs and symptoms and/or as in the present case the analysis of biological markers (such as proteins) indicative of the disease, e.g. *in vivo* or *in vitro.*

As used herein, the term "treatment" or "treating" of a disease includes preventing or protecting against the disease (that is, causing the clinical symptoms not to develop); inhibiting the disease (i.e., arresting or suppressing the development of clinical symptoms; and/or relieving the disease (i.e., causing the regression of clinical symptoms). As will be appreciated, it is not always possible to distinguish between "preventing" and "suppressing" a disease or disorder since the ultimate inductive event or events may be unknown or latent. Accordingly, the term "prophylaxis" will be understood to constitute a type of "treatment" that encompasses both "preventing" and "suppressing." The term "treatment" thus includes "prophylaxis". Accordingly, the term "treatment" includes prophylactic treatment (before onset of the disease) as well as therapeutic treatment (after onset of the disease). Typically, the pharmaceutical composition of the invention is applied for the treatment of cancer diseases, in particular malignant cancer diseases involving cells of neuroectodermal origin, such as NET as described herein.

The pharmaceutical compositions as described herein are typically administered parenterally. Administration may preferably be accomplished systemically, for instance by intravenous (i.v.), intraarterial (i.a.), subcutaneous, intramuscular or intradermal injection, preferably i.v. Alternatively, administration may be accomplished locally, for instance by intra-tumoral injection. The pharmaceutical compositions as described above may be administered to a subject in need thereof several times a day, daily, every other day, weekly, or monthly.

Pharmaceutical compositions of the invention comprising radiolabeled complexes with a targeting moiety binding to PSMA, may be used in the treatment or diagnosis of any cancer expressing PSMA. In particular, the presence of PSMA-expressing cells or tissues may be indicative of a prostate tumor (cell), a metastasized prostate tumor (cell), a renal tumor (cell), a pancreatic tumor (cell), a bladder tumor (cell), and combinations thereof. Accordingly, the cancer is preferably prostate cancer, pancreatic cancer, renal cancer or bladder cancer.

Pharmaceutical compositions of the invention comprising radiolabeled complexes with a targeting moiety binding to *α*V*β*3, may be used in the treatment or diagnosis of tumor-induced angiogenesis, as described above.

Radiolabeled complexes with a targeting molecule binding to a somatostatin receptor, in particular somatostatin receptor 2 (SSTR-2) or somatostatin receptor 5 (SSTR-5), more particularly somatostatin receptor 2 (SSTR-2), may be used in the treatment of any cancer expressing a somatostatin receptor. Accordingly, the cancer may be a neuroendocrine tumor (NET). In particular, the NET may be selected from the group consisting of gastroenteropancreatic neuroendocrine tumor, carcinoid tumor, pheochromocytoma, paraganglioma, medullary thyroid cancer, pulmonary neuroendocrine tumor, thymic neuroendocrine tumor, a carcinoid tumor or a pancreatic neuroendocrine tumor, pituitary adenoma, adrenal gland tumors, Merkel cell carcinoma, breast cancer, Non-Hodgkin lymphoma, Hodgkin lymphoma, Head & Neck tumor, urothelial carcinoma (bladder), Renal Cell Carcinoma, Hepatocellular Carcinoma, GIST, neuroblastoma, bile duct tumor, cervix tumor, Ewing sarcoma, osteosarcoma, small cell lung cancer (SCLC), prostate cancer, melanoma, meningioma, glioma, medulloblastoma, hemangioblastoma, supratentorial primitive, neuroectodermal tumor, and esthesioneuroblastoma. Further non-limiting examples of NET tumors include functional carcinoid tumor, insulinoma, gastrinoma, vasoactive intestinal peptide (VIP) oma, glucagonoma, serotoninoma, histaminoma, ACTHoma, pheocromocytoma, and somatostatinoma. In some embodiments, the human being to be treated suffers from a tumor, which has been been diagnosed as SSTR-2 positive.

In some embodiments, the NET may be selected from the group consisting of carcinoid tumor, pheochromocytoma, paraganglioma, medullary thyroid cancer, pulmonary neuroendocrine tumor, thymic neuroendocrine tumor, a carcinoid tumor, pituitary adenoma, adrenal gland tumors, Merkel cell carcinoma, breast cancer, Non-Hodgkin lymphoma, Hodgkin lymphoma, head & neck tumor, urothelial carcinoma (bladder), renal Cell Carcinoma, hepatocellular Carcinoma, GIST, neuroblastoma, bile duct tumor, cervix tumor, Ewing sarcoma, osteosarcoma, small cell lung cancer (SCLC), prostate cancer, melanoma, meningioma, glioma, medulloblastoma, hemangioblastoma, supratentorial primitive, neuroectodermal tumor, and esthesioneuroblastoma.

In general, preferred types of cancers to be treated include those, for which radionuclide therapy is established and currently under investigation. Usually, the type of cancer investigated primarily reflects developments related to the available targets (and targeting molecules for the specific delivery of the radionuclide to the cancer cells). Examples of cancers include, but are not limited to, thyroid malignancies, haematological malignancies, hepatic malignancies, prostate cancer, colorectal cancer, breast cancer, neuroendocrine and somatostatin receptor cancers. In some embodiments, the cancer is selected from neuroendocrine tumors, prostate cancer, pancreatic cancer, renal cancer, bladder cancer, brain cancer, gastrointestinal cancer, medullar thyroid carcinomas, small or non-small cell lung cancers, stromal ovarian carcinomas, ductal pancreatic adenocarcinoma, insulinomas, gastrinomas, breast cancer, and sarcoma.

In some embodiments, the cancer is selected from neuroendocrine tumors, prostate cancer, renal cancer, bladder cancer, brain cancer, gastrointestinal cancer, medullar thyroid carcinomas, small or non-small cell lung cancer, stromal ovarian carcinomas, insulinomas, gastrinomas, breast cancer, and sarcoma.

In some embodiments, the cancer is selected from neuroendocrine tumors, prostate cancer, small cell lung cancer, breast cancer, and hepatocellular cancer.

The pharmaceutical composition as described above may be used for both imaging and therapeutic purposes, i.e. as a "theragnostic" agent. As used herein, the term "theragnostic" includes "therapeutic-only", "diagnostic-only" and "therapeutic and diagnostic" applications. Thus, the pharmaceutical composition according to the present invention can be used in an (*in vitro*) method of detecting the presence of cancerous cells and/or tissues comprising contacting said cancerous cells and/or tissues with the pharmaceutical composition of the invention and applying detection means, optionally radiographic imaging, to detect said cells and/or tissues.

In the *in vivo* and *in vitro* uses and methods of the present invention, radiographic imaging may be accomplished using any means and methods known in the art. Preferably, radiographic imaging may involve positron emission tomography (PET) or single-photon emission computed tomography (SPECT). The targeted cells or tissues detected by radiographic imaging of the inventive conjugate may preferably comprise (optionally cancerous) prostate cells or tissues, (optionally cancerous) spleen cellsortissues, or (optionally cancerous) kidney cells or tissues.

### Method for preparing the pharmaceutical composition

The present invention also provides a method for preparing a pharmaceutical composition according to the present invention.

A method for preparing a pharmaceutical composition according to the present invention comprises
(a) radiolabeling a precursor comprising a targeting moiety covalently linked to a chelating moiety with a radionuclide in a radiolabeling buffer; and
(b) adding a formulation buffer comprising para-aminohippuric acid or a pharmaceutically acceptable salt or carboxylic derivative thereof as a stabilizer.

In step (a) of the method according to the present invention, formation of the radiolabeled complex is achieved by radiolabeling a precursor (comprising a targeting moiety covalently linked to a chelating moiety) with a radionuclide in a labeling buffer. Thus, step (a) is accomplished in a reaction mixture (also referred to as "radiolabeling composition") for the complex formation (radiolabeling).

To obtain the radiolabeling reaction mixture (radiolabeling composition), a radionuclide solution comprising a radionuclide precursor, e.g. a radionuclide chloride solution (e.g. [¹⁷⁷Lu]LuCl₃, [¹⁶¹Tb]TbCl₃, [²²⁵Ac]AcCl₃, etc.) is added to the solution containing the conjugate (chelating moiety linked to the targeting moiety) precursor (or vice versa). Alternatively, the radionuclide precursor may be provided in form a nitrate, phosphate, carbonate, sulfate or sulphite, for example. Accordingly, any agent/compound present during complex formation (radiolabeling), such as a stabilizer, may be contained in either the radionuclide solution, in the solution containing the conjugate (the chelating moiety linked to the targeting moiety), or in a separate solution to be added. After obtaining the radiolabeling composition, elevated temperatures, such as 20 to 110°C, may be applied to the radiolabeling composition (including the agents/compounds comprised therein) for a predetermined period of time, such as 1 to 60 minutes, to facilitate the complex formation (radiolabeling).

Thereby, it is preferred that a buffer comprising a stabilizer, such as ascorbic acid and/or a salt thereof, e.g. sodium ascorbate, is present during complex formation (i.e., in the radiolabeling composition). Alternatively, sodium acetate may be used as a stabilizer in the radiolabeling buffer. The stabilizer, if present, e.g. sodium acetate, may be present in the radiolabeling composition in a concentration of about 10 mg/mL to 300 mg/mL, or about 10 mg/mL to 250 mg/mL

Preferably, the radiolabeling composition in step (a) has a pH of about 4.0 to 7.5. The buffer is useful to maintain such an advantageous pH range. Preferably, the radiolabeling buffer has a pH of about 5.0 to 9.0. The use of a radiolabeling buffer in step (a) has the advantage that the pH can be maintained even for different amounts of the radionuclide chloride solution used for radiolabeling, as required, while the pH is maintained.

In a particular embodiment, alternatively or additionally to buffering stabilizers, such as ascorbic acid and/or a salt thereof, e.g. sodium ascorbate, or sodium acetate, ethanol may be present during complex formation (i.e., in the radiolabeling composition) at a concentration of 1 to 60 % (vol/vol), e.g. 2 to 20% (vol/vol), or 5 to 10% (vol/vol).

As used herein, the expression "after the complex formation (radiolabeling)" refers to the time when the complex forming (radiolabeling) reaction is finished. For example, when elevated temperatures were applied for radiolabeling, "after the complex formation (radiolabeling)" may refer to a time when the radiolabeling composition (radiolabeling reaction mixture) is no longer exposed to an elevated temperature (for example, when ambient temperature is reached again, e.g. by cooling down the radiolabeling composition). In particular, "after the complex formation (radiolabeling)" may refer to the formulation of the (final) pharmaceutical composition, e.g. by dilution of the radiolabeling mix with a formulation buffer and water.

In this respect, it should be noted that the reaction mixture resulting from the labeling reaction does not have to be removed, and the labeled product does not have to be purified or isolated before formulation.

As described above, formulation of the pharmaceutical composition (step (b)) is accomplished by adding a formulation buffer comprising para-aminohippuric acid or a pharmaceutically acceptable salt or carboxylic derivative thereof to the radiolabeled complex to obtain the pharmaceutical composition. Preferably, the salt of para-aminohippuric acid is sodium para-aminohippurate.

Preferably, para-aminohippuric acid or a pharmaceutically acceptable salt or carboxylic derivative thereof, e.g. sodium para-aminohippurate, is added in step (b) such that para-aminohippuric acid or a pharmaceutically acceptable salt or carboxylic derivative thereof is present in the pharmaceutical composition in a concentration of about 1 mg/mL to about 300 mg/mL.

For example, para-aminohippuric acid or a pharmaceutically acceptable salt or carboxylic derivative thereof is added in step (b) such that para-aminohippuric acid or a pharmaceutically acceptable salt or carboxylic derivative thereof is present in the pharmaceutical composition in a range from about 10 mg/mL to about 300 mg/mL, more preferably from about 20 mg/mL to about 300 mg/mL, still more preferably from about 30 mg/mL to about 250 mg/mL, most preferably from about 50 mg/mL to about 200 mg/mL, such as about 50 mg/mL, 60 mg/mL, 70 mg/mL, 80 mg/mL, 90 mg/mL, 100 mg/mL, 110 mg/mL, 120 mg/mL, 130 mg/mL, 140 mg/mL, 150 mg/mL, 160 mg/mL, 170 mg/mL, 180 mg/mL, 190 mg/mL, and 200 mg/mL.

In some embodiments, para-aminohippuric acid and/or a pharmaceutically acceptable salt and/or carboxylic derivative thereof is/are the only stabilizer(s) added in step (b).

As mentioned above, para-aminohippuric acid or a pharmaceutically acceptable salt or carboxylic derivative thereof used as a stabilizer and antioxidant may also serve as a pH modulator or buffer in the pharmaceutical formulation providing a pH range of 4.5 - 8 suitable for parenteral injection purposes so that complicated mixtures can be avoided. Moreover, the concentration of para-aminohippuric acid or a pharmaceutically acceptable salt or carboxylic derivative thereof requires no further osmotic adjustment which further simplifies the preparation of the stabilised pharmaceutical composition. In contrast to ascorbic acid/ascorbate formulations, para-aminohippuric acid or a pharmaceutically acceptable salt or carboxylic derivative thereof can be safely administered parenterally, e.g. by injection, even in large quantities.

After addition of para-aminohippuric acid or a pharmaceutically acceptable salt or carboxylic derivative thereof in step (b), the concentration of the sum of radiolabeled complex (comprising (i) the radionuclide, e.g. Lu-177, and (ii) the targeting moiety covalently linked to the chelating moiety), and the precursor (non-radioactive) in the pharmaceutical composition is preferably in a range from about 1 µg/mL to about 100 µg/mL, preferably from about 5 µg/mL to about 50 µg/mL, more preferably from about 7 µg/mL to about 30 µg/mL.

It is understood that the above detailed description of the pharmaceutical composition according to the present invention applies accordingly to the method for preparing the pharmaceutical composition according to the present invention. For example, detailed embodiments described above for the radiolabeled complex of the pharmaceutical composition apply accordingly to the method for preparing the pharmaceutical composition according to the present invention.

For example, the precursor compound (the targeting moiety linked to the chelating moiety) used in the radiolabeling step (a) to form the radiolabeled complex, may be as described above (with respect to the pharmaceutical composition).

Accordingly, the targeting moiety of the precursor for radiolabeling used in step (a) may be selected from peptides, peptidomimetics, antibodies, antibody fragments, and antibody mimetics.

As described with respect to the pharmaceutical composition according to the present invention, the targeting moiety of the precursor for radiolabeling may target any cell surface molecule known in the art, such as the prostate specific membrane antigen (PSMA), the somatostatin receptor, integrin, a folate receptor, the CCK2-receptor, the neurotensin receptor 1, the glucagon-like peptide 1 receptor, the neurokinin type 1 receptor, FAP, Glypican-3, Nectin-4, DLL3, Trop2, GPC3, IL-13Rα2 B7H3, CAIX or affilins, for example, and in particular may target and preferably bind to the somatostatin receptor, the prostate specific membrane antigen (PSMA), integrin, fibroblast activation protein, or a folate receptor.

Accordingly, the targeting moiety of the precursor for radiolabeling may e.g. be selected from the group consisting of Tyr³-octeotride, Tyr³-octreotate, JR11, PSMA-11, RGD and folate.

As described with respect to the pharmaceutical composition according to the present invention, the chelating moiety may be selected based on its ability to coordinate the desired central (metal) ion, usually the radionuclide as described herein. Accordingly, the chelating moiety may be a macrocyclic or linear chelator characterized by one of the above Formulae (2a) to (2ff).

In a particular embodiment, the chelating moiety of the precursor for radiolabeling may be a macrocyclic chelator, e.g. selected from the group consisting of DOTA, AAZTA, NOTA, NODAGA, DOTAGA, DOTAM, HBED-CC, TRAP, NOPO, PCTA, DTPA, HYNIC, DFO, DO3AP, DO3AP^{PrA}, and DO3AP^{ABn}, or derivatives thereof.

In a preferred embodiment, the chelating moiety of the precursor for radiolabeling is DOTA. Accordingly, in a preferred embodiment, the precursor for radiolabeling may comprise or consist of PSMA-617, PSMA-I&T, Ibu-PSMA, Ibu-DAB-PSMA, Ibu-N-PSMA, Ibu-Dα-PSMA, DOTA-TOC, DOTA-NOC, DOTA-TATE, DOTA-JR11, DOTA-LM3, DOTA-RGD, and DOTA-folate as presented in formulas (4) to (16) above.

As described with respect to the pharmaceutical composition according to the present invention, the choice of a suitable radionuclide may depend *inter alia* on the chemical structure and chelating capability of the chelating agent, and the intended application of the resulting (complexed) conjugate (e.g. diagnostic vs. therapeutic). Preferably, the radionuclide may be useful for cancer imaging or therapy. Accordingly, the used in the method according to the present invention radionuclide may be selected from the group consisting of Ac-225, At-211, Bi-212, Bi-213, Co-55, Co-57, Cu-61,Cu-64, Cu-67, Dy-166, Er-165, Er-169, F-18, Ga-67, Ga-68, Ho-166,I-123, I-124, I-131, In-111, Lu-177, Pb-212, Pd-103, Pd-109, Re-186, Re-188, Rh-103m, Sc-43, Sc-44, Sc-47, Sm-153, Tb-149, Tb-152, Tb-155, Tb-161, Tc-99m, Th-227, Tc-99m, Y-86, and Y-90, and may preferably be selected from the group consisting of Ac-225, At-211, Bi-212, Bi-213, Co-55, Co-57, Cu-61, Cu-64, Cu-67, Dy-166, Ga-67, Ga-68, Ho-166, In-111, Lu-177, Pb-212, Sc-43, Sc-44, Sc-47, Sm-153, Tb-161, Y-86, and Y-90.

In a preferred embodiment, the radionuclide used in the method according to the present invention is a beta-emitting radionuclide, such as Lu-177 or Tb-161, and the radionuclide/radiolabeled complex is present in step (a) such that after adding para-aminohippuric acid or a pharmaceutically acceptable salt or carboxylic derivative thereof in step (b) as a stabilizer, the pharmaceutical composition has a radioactivity concentration of about 0.3 to 1.0 GBq/mL.

In another preferred embodiment, the radionuclide used in the method according to the present invention is an alpha-emitting radionuclide, such as Ac-255, and the radionuclide/radiolabeled complex is present in step (a) such that after adding para-aminohippuric acid or a pharmaceutically acceptable salt or carboxylic derivative thereof in step (b) as a stabilizer, the pharmaceutical composition has a radioactivity concentration of about 0.2 to 1.0 MBq/mL.

As described above, in particular embodiments, further stabilizers, such as sodium acetate (NaOAc), ascorbic acid and/or a salt thereof, ethanol, gentisic acid, methionine, histidine, melatonine, and Se-methionine, may be added in step (b). However, it is preferred that no other stabilizers (other than para-aminohippuric acid or a pharmaceutically acceptable salt or carboxylic derivative thereof, e.g. sodium para-aminohippurate) are used in the formulation step (b) of the pharmaceutical composition.

Nevertheless, in some embodiments other diluents (such as saline) may be added in the formulation step. In this way, a final volume (and concentration) of the pharmaceutical composition may be reached, e.g. as ready-to-use pharmaceutical composition, for example as single-dose product. To this end, a final volume of about 1 to 20 mL of the pharmaceutical composition, preferably 1 to 10 ml of the pharmaceutical composition may be reached by appropriate dilution (and, optionally, addition of further pharmaceutical acceptable components as described above).

In some embodiments, a sequestering agent, such as DTPA, may be added to the pharmaceutical composition in the formulation step (b). For example, DTPA may be added in a concentration of 0.01 to 1 mM, e.g. 0.1 mM.

As shown in the Examples below, the pharmaceutical composition prepared by the method according to the present invention has a radiochemical purity of ≥ 95% over a period of at least 96 hours when stored at RT.

Preferably, the pharmaceutical composition prepared by the method according to the present invention is administered parenterally, in particular via intravenous or intratumoral injection, and is accordingly formulated in liquid or lyophilized form for parenteral administration. Parenteral formulations may be stored in vials, IV bags, ampoules, cartridges, or prefilled syringes and can be administered as injections, inhalants, or aerosols, with injections being preferred.

Liquid pharmaceutical compositions administered via injection and in particular via intravasal, more preferably intravenous (i.v.) injection should preferably be sterile and stable under the conditions of manufacture and storage. Such compositions are typically formulated as parenterally acceptable aqueous solutions that are pyrogen-free, have suitable pH, are isotonic and maintain stability of the active ingredient(s).

Before administering to a subject, a pharmaceutically acceptable excipient, diluent or carrier may be added to the pharmaceutical composition prepared by the method according to the present invention. The term "pharmaceutically acceptable", as used herein, refers to a compound or agent that is compatible with the components of the pharmaceutical composition, in particular the active (anti-cancer) compounds, and does not interfere with and/or substantially reduce its therapeutic activities. Pharmaceutically acceptable carriers preferably have sufficiently high purity and sufficiently low toxicity to make them suitable for administration to a subject to be treated.

For liquid pharmaceutical formulations, suitable pharmaceutically acceptable excipients and carriers include water, typically pyrogen-free water; isotonic saline or buffered (aqueous) solutions, e.g. phosphate, citrate etc. buffered solutions. Particularly for injection of the (pharmaceutical) compositions, water or preferably a buffer, more preferably an aqueous buffer, may be used, which may contain a sodium salt, e.g. at least 50 mM of a sodium salt, a calcium salt, e.g. at least 0.01 mM of a calcium salt, and optionally a potassium salt, e.g. at least 3 mM of a potassium salt.

The sodium, calcium and, optionally, potassium salts may occur in the form of their halogenides, e.g. chlorides, iodides, or bromides, in the form of their hydroxides, carbonates, hydrogen carbonates, or sulfates, etc. Without being limited thereto, examples of sodium salts include e.g. NaCl, Nal, NaBr, Na₂CO₃, NaHCO₃, Na₂SO₄, examples of the optional potassium salts include e.g. KCI, KI, KBr, K₂CO₃, KHCO₃, K₂SO₄, and examples of calcium salts include e.g. CaCl₂, Cal₂, CaBr₂, CaCO₃, CaSO₄, Ca(OH)₂. Furthermore, organic anions of the aforementioned cations may be contained in the buffer.

Buffers suitable for injection purposes as defined above, may contain salts selected from sodium chloride (NaCl), calcium chloride (CaCl₂) and optionally potassium chloride (KCI), wherein further anions may be present additional to the chlorides. Typically, the salts in the injection buffer are present in a concentration of at least 50 mM sodium chloride (NaCl), at least 3 mM potassium chloride (KCl) and at least 0.01 mM calcium chloride (CaCl₂). The injection buffer may be hypertonic, isotonic or hypotonic with reference to the specific reference medium, i.e. the buffer may have a higher, identical or lower salt content with reference to the specific reference medium, wherein preferably such concentrations of the afore mentioned salts may be used, which do not lead to damage of cells due to osmosis or other concentration effects.

However, as mentioned above, in the formulation of the pharmaceutical composition prepared by the method according to the present invention no further buffer may be required, as PAH itself also acts as a pH modulator/buffer in a pH range of 4.5 - 8 suitable for injection purposes and requires no further osmotic adjustment.

The pharmaceutical composition prepared by the method according to the present invention may be provided in lyophilized form. Lyophilized pharmaceutical compositions are preferably reconstituted in water for injection (WFI) or a suitable buffer, e.g. PAH, prior to administration.

The present invention also provides a pharmaceutical composition obtained by the method of the invention as described above. Such a pharmaceutical composition typically exhibits the features of the above-described pharmaceutical composition. The present inventors have found that a pharmaceutical composition obtained by the method of the present invention has excellent radiochemical purity over a prolonged period of time, in particular a radiochemical purity of ≥95% over 96 hours at RT.

### Use of PAH as a stabilizer against radiolytic degradation in a pharmaceutical composition

In a further aspect, the present invention provides para-aminohippuric acid or a pharmaceutically acceptable salt or carboxylic derivative thereof for use as a stabilizer against radiolytic degradation of the radiolabeled complex as comprised in a pharmaceutical composition comprising a radiolabeled complex comprising (i) a radionuclide, and (ii) a targeting moiety covalently linked to a chelating moiety.

It is understood that the above detailed description of the pharmaceutical composition according to the present invention, and the method for preparing a pharmaceutical composition, respectively, apply accordingly to the inventive use of para-aminohippuric acid or a pharmaceutically acceptable salt or carboxylic derivative thereof as a stabilizer against radiolytic degradation of the radiolabeled complex in a pharmaceutical composition, e.g. as described herein.

Accordingly, as described with respect to the pharmaceutical composition according to the present invention and the method for preparing the pharmaceutical composition, respectively, the salt of para-aminohippuric acid used as a stabilizer of the pharmaceutical composition is analkaline salt, preferably sodium para-aminohippurate.

Further, the concentration of para-aminohippuric acid or a pharmaceutically acceptable salt or carboxylic derivative thereof, e.g. of sodium para-aminohippurate, used as a stabilizer in the pharmaceutical composition may be in a range from about 1 mg/mL to 300 mg/mL, preferably 10 mg/mL to about 300 mg/mL, more preferably from about 20 mg/mL to about 300 mg/mL, still more preferably from about 30 mg/mL to about 250 mg/mL, most preferably from about 50 mg/mL to about 200 mg/mL, such as about 50 mg/mL, 60 mg/mL, 70 mg/mL, 80 mg/mL, 90 mg/mL, 100 mg/mL, 110 mg/mL, 120 mg/mL, 130 mg/mL, 140 mg/mL, 150 mg/mL, 160 mg/mL, 170 mg/mL, 180 mg/mL, 190 mg/mL, and 200 mg/mL.

Moreover, the pharmaceutical composition stabilized by para-aminohippuric acid or a pharmaceutically acceptable salt or carboxylic derivative thereof may additionally comprise further stabilizers/antioxidants, such as ascorbic acid and/or a salt thereof, in particular sodium ascorbate, gentisic acid, ethanol or methionine, for example.

However, in a preferred embodiment, the pharmaceutical composition stabilized by para-aminohippuric acid or a pharmaceutically acceptable salt or carboxylic derivative thereof does not contain further stabilizers and/or antioxidants. In other words, para-aminohippuric acid or a pharmaceutically acceptable salt or carboxylic derivative thereof, in particular sodium para-aminohippurate, preferably is (essentially) the only stabilizer used to stabilize the pharmaceutical composition.

However, it is to be noted that the pharmaceutical composition stabilized by para-aminohippuric acid or a pharmaceutically acceptable salt or carboxylic derivative thereof may contain minor amounts of other stabilizers, such as ascorbic acid/sodium ascorbate or sodium acetate buffer, originating from the labeling reaction (step (a) of the method of preparing the pharmaceutical composition).

In a preferred embodiment, the sum of radiolabeled complex (comprising the radionuclide, e.g. Lu-1 77, and the targeting moiety covalently linked to the chelating moiety) and the (non-radioactive) precursor in the pharmaceutical composition stabilized by para-aminohippuric acid or a pharmaceutically acceptable salt or carboxylic derivative thereof is preferably in a range from about 1 µg/mL to about 100 µg/mL, preferably from about 5 µg/mL to about 50 µg/mL, more preferably from about 7 µg/mL to about 30 µg/mL.

It is understood that the detailed embodiments described above for the radiolabeled complex of the pharmaceutical composition apply accordingly to the use of para-aminohippuric acid or a pharmaceutically acceptable salt or carboxylic derivative thereof as a stabilizer against radiolytic degradation of the radiolabeled complex in a pharmaceutical composition according to the invention.

Accordingly, the targeting moiety, the chelating moiety and the radionuclide of the radiolabeled complex stabilized by para-aminohippuric acid or a pharmaceutically acceptable salt or carboxylic derivative thereof may be as described above (with respect to the pharmaceutical composition).

In particular, the targeting moiety of the radiolabeled complex stabilized by para-aminohippuric acid or a pharmaceutically acceptable salt or carboxylic derivative thereof may be selected from peptides, peptidomimetics, antibodies, antibody fragments, and antibody mimetics.

As described with respect to the pharmaceutical composition according to the present invention, the targeting moiety of the radiolabeled complex stabilized by para-aminohippuric acid or a pharmaceutically acceptable salt or carboxylic derivative thereof may target any cell surface molecule known in the art, such as the somatostatin receptor, the prostate specific membrane antigen (PSMA), integrin, a folate receptor, the CCH-2-receptor, the neurotensin receptor 1, the glucagon-like peptide 1 receptor, the neurokinin type 1 receptor, affilins, FAP, Glypican-3, Nectin-4, DLL3, Trop2, GPC3, IL-13Rα2 B7H3, or CAIX, for example, and in particular may target and preferably bind to the somatostatin receptor, the prostate specific membrane antigen (PSMA), integrin, or a folate receptor.

Therefore, the targeting moiety of the radiolabeled complex stabilized by para-aminohippuric acid or a pharmaceutically acceptable salt or carboxylic derivative thereof may e.g. be selected from the group consisting of Tyr3-octeotride, Tyr3-octreotate, JR11, PSMA-11, RGD and folate.

As described with respect to the pharmaceutical composition according to the present invention, the chelating moiety of the radiolabeled complex stabilized by para-aminohippuric acid or a pharmaceutically acceptable salt or carboxylic derivative thereof may be selected based on its ability to coordinate the desired central (metal) ion, usually the radionuclide as described herein. Accordingly, the chelating moiety may be a macrocyclic or linear chelator characterized by one of the above Formulae (2a) to (2ff).

In a particular embodiment, the chelating moiety of the radiolabeled complex stabilized by para-aminohippuric acid or a pharmaceutically acceptable salt or carboxylic derivative thereof may be a macrocyclic chelator, e.g. selected from the group consisting of DOTA, AAZTA, NOTA, NODAGA, DOTAGA, DOTAM, HBED-CC, TRAP, NOPO, PCTA, DTPA, HYNIC, DFO, DO3AP, DO3AP^{PrA}, and DO3AP^{ABn}, or derivatives thereof.

In a preferred embodiment, the chelating moiety of the radiolabeled complex stabilized by para-aminohippuric acid or a pharmaceutically acceptable salt or carboxylic derivative thereof is DOTA.

Accordingly, in a preferred embodiment, the radiolabeled complex stabilized by para-aminohippuric acid or a pharmaceutically acceptable salt or carboxylic derivative thereof may comprise PSMA-617, PSMA-I&T, Ibu-PSMA, Ibu-DAB-PSMA, Ibu-N-PSMA, Ibu-Dα-PSMA, DOTA-TOC, DOTA-NOC, DOTA-TATE, DOTA-JR11, DOTA-LM3, DOTA-RGD, and DOTA-folate as presented in formulas (4) to (16) above.

As described with respect to the pharmaceutical composition according to the present invention, the choice of a suitable radionuclide may depend *inter alia* on the chemical structure and chelating capability of the chelating agent, and the intended application of the resulting (complexed) conjugate (e.g. diagnostic vs. therapeutic). Preferably, the radionuclide may be useful for cancer imaging or therapy. Accordingly, the radionuclide of the radiolabeled complex stabilized by para-aminohippuric acid or a pharmaceutically acceptable salt or carboxylic derivative thereof may be selected from the group consisting of Ac-225, At-211, Bi-212, Bi-213, Co-55, Co-57, Cu-61, Cu-64, Cu-67, Dy-166, Er-165, Er-169, F-18, Ga-67, Ga-68, Ho-166, I-123, I-124, I-131,In-111, Lu-177, Pb-212, Pd-103, Pd-109, Re-186, Re-188, Rh-103m, Sc-43, Sc-44, Sc-47, Sm-153, Tb-149, Tb-152, Tb-155, Tb-161, Tc-99m, Th-227, Tc-99m, Y-86, and Y-90, and may particularly be selected from the group consisting of Ac-225, At-211, Bi-212, Bi-213, Co-55, Co-57, Cu-61, Cu-64, Cu-67, Dy-166, Ga-67, Ga-68, Ho-166, In-111, Lu-177, Pb-212, Sc-43, Sc-44, Sc-47, Sm-153, Tb-161, Y-86, and Y-90

In a preferred embodiment, the radionuclide of the radiolabeled complex stabilized by para-aminohippuric acid or a pharmaceutically acceptable salt or carboxylic derivative thereof according to the present invention is a beta-emitting radionuclide, such as Lu-177 or Tb-161. Preferably, the beta-emitting radionuclide of the radiolabeled complex stabilized by para-aminohippuric acid or a pharmaceutically acceptable salt or carboxylic derivative thereof is present in the pharmaceutical composition in such an amount that the pharmaceutical composition has a radioactivity concentration of about 0.3 to 1.0 GBq/mL.

In another preferred embodiment, the radionuclide of the radiolabeled complex stabilized by para-aminohippuric acid or a pharmaceutically acceptable salt or carboxylic derivative thereof according to the present invention is an alpha-emitting radionuclide, such as Ac-225. Preferably, the alpha-emitting radionuclide of the radiolabeled complex stabilized by para-aminohippuric acid or a pharmaceutically acceptable salt or carboxylic derivative thereof is present in the pharmaceutical composition in such an amount that the pharmaceutical composition has a radioactivity concentration of about 0.2 to 1.0 MBq/mL.

Preferably no other stabilizers (other than para-aminohippuric acid or a pharmaceutically acceptable salt or carboxylic derivative thereof, e.g. sodium para-aminohippurate) are present in the pharmaceutical composition stabilized by para-aminohippuric acid or a pharmaceutically acceptable salt or carboxylic derivative thereof.

However, in some embodiments other diluents (such as saline) may present in the formulation stabilized by para-aminohippuric acid or a pharmaceutically acceptable salt or carboxylic derivative thereof. In this way, a final volume (and concentration) of the pharmaceutical composition may be reached, e.g. as ready-to-use pharmaceutical composition, for example as single-dose product. To this end, a final volume of about 1 to 20 mL of the pharmaceutical composition, preferably 1 to 10 mL of the pharmaceutical composition may be reached by appropriate dilution (and, optionally, addition of further pharmaceutical acceptable components as described above).

In some embodiments, a sequestering agent, such as DTPA may be included in the pharmaceutical composition stabilized by para-aminohippuric acid or a pharmaceutically acceptable salt or carboxylic derivative thereof. For example, DTPA may be included in a concentration of 0.01 to 1 mM, e.g. 0.1 mM.

As shown in the Examples below, the pharmaceutical composition stabilized by para-aminohippuric acid or a pharmaceutically acceptable salt or carboxylic derivative thereof according to the present invention has a radiochemical purity of ≥ 95% over a period of at least 96 hours at RT.

According to preferred embodiments, the stabilized pharmaceutical composition is administered parenterally, in particular via intravenous or intratumoral injection, and is accordingly formulated in liquid or lyophilized form for parenteral administration. Parenteral formulations may be stored in vials, IV bags, ampoules, cartridges, or prefilled syringes and can be administered as injections, inhalants, or aerosols, with injections being preferred.

Liquid pharmaceutical compositions administered via injection and in particular via intravasal, more preferably intravenous (i.v.) injection should preferably be sterile and stable under the conditions of manufacture and storage. Such compositions are typically formulated as parenterally acceptable aqueous solutions that are pyrogen-free, have suitable pH, are isotonic and maintain stability of the active ingredient(s).

The pharmaceutical composition stabilized by para-aminohippuric acid or a pharmaceutically acceptable salt or carboxylic derivative thereof may comprise a pharmaceutically acceptable excipient, diluent or carrier. The term "pharmaceutically acceptable", as used herein, refers to a compound or agent that is compatible with the components of the pharmaceutical composition, in particular the active (anti-cancer) compounds, and does not interfere with and/or substantially reduce its therapeutic activities. Pharmaceutically acceptable carriers preferably have sufficiently high purity and sufficiently low toxicity to make them suitable for administration to a subject to be treated.

For liquid pharmaceutical compositions, suitable pharmaceutically acceptable excipients and carriers include water, typically pyrogen-free water; isotonic saline or buffered (aqueous) solutions, e.g. phosphate, citrate etc. buffered solutions. Particularly for injection of the (pharmaceutical) compositions, water or preferably a buffer, more preferably an aqueous buffer, may be used, which may contain a sodium salt, e.g. at least 50 mM of a sodium salt, a calcium salt, e.g. at least 0,01 mM of a calcium salt, and optionally a potassium salt, e.g. at least 3 mM of a potassium salt.

The sodium, calcium and, optionally, potassium salts may occur in the form of their halogenides, e.g. chlorides, iodides, or bromides, in the form of their hydroxides, carbonates, hydrogen carbonates, or sulfates, etc. Without being limited thereto, examples of sodium salts include e.g. NaCl, Nal, NaBr, Na₂CO₃, NaHCO₃, Na₂SO₄, examples of the optional potassium salts include e.g. KCI, KI, KBr, K₂CO₃, KHCO₃, K₂SO₄, and examples of calcium salts include e.g. CaCl₂, Cal₂, CaBr₂, CaCO₃, CaSO₄, Ca(OH)₂. Furthermore, organic anions of the aforementioned cations may be contained in the buffer.

Buffers suitable for injection purposes as defined above, may contain salts selected from sodium chloride (NaCl), calcium chloride (CaCl₂) and optionally potassium chloride (KCI), wherein further anions may be present additional to the chlorides. Typically, the salts in the injection buffer are present in a concentration of at least 50 mM sodium chloride (NaCl), at least 3 mM potassium chloride (KCI) and at least 0,01 mM calcium chloride (CaCl₂). The injection buffer may be hypertonic, isotonic or hypotonic with reference to the specific reference medium, i.e. the buffer may have a higher, identical or lower salt content with reference to the specific reference medium, wherein preferably such concentrations of the afore mentioned salts may be used, which do not lead to damage of cells due to osmosis or other concentration effects.

However, as mentioned above, in the formulation of the pharmaceutical composition stabilized by para-aminohippuric acid or a pharmaceutically acceptable salt or carboxylic derivative thereof no further buffer may be required, as PAH itself also acts as a pH modulator/buffer in a pH range of 4.5 - 8 suitable for injection purposes and requires no further osmotic adjustment.

The pharmaceutical composition stabilized by para-aminohippuric acid or a pharmaceutically acceptable salt or carboxylic derivative thereof according to the present invention may be provided in lyophilized form. Lyophilized pharmaceutical compositions are preferably reconstituted in a suitable buffer, e.g. PAH, prior to administration.

The present inventors found that a pharmaceutical composition stabilized by para-aminohippuric acid or a pharmaceutically acceptable salt or carboxylic derivative thereof has excellent radiochemical purity over a prolonged period of time.

The present invention is further illustrated by the following set of items and combinations of items resulting from the dependencies and back-references as indicated. In particular, it is noted that in each instance where a range of items is mentioned, for example in the context of a term such as "The pharmaceutical composition according to any one of items 1 to 3", every item in this range is meant to be explicitly disclosed for the skilled person, i.e. the wording of this term is to be understood by the skilled person as being synonymous to "The pharmaceutical composition according to any one of items 1, 2 and 3". Further, it is explicitly noted that the following set of items represents a suitably structured part of the general description directed to preferred aspects of the present invention, and, thus, suitably supports, but does not represent the claims of the present invention.
1. A pharmaceutical composition comprising
   (a) a radiolabeled complex comprising (i) a radionuclide, and (ii) a targeting moiety covalently linked to a chelating moiety; and
   (b) a stabilizer against radiolytic degradation of the radiolabeled complex, wherein the stabilizer comprises a compound according to Formula (1) or a pharmaceutically acceptable salt thereof: 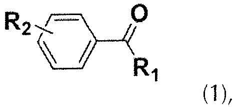
      wherein R₁ is selected from the group consisting of H, OH, NH₂, an alanine residue, and a glycine residue, and
      R₂ is selected from the group consisting of H, OH, NH₂, F, Cl, Br, I, SO₂, O-methyl, methyl, and O-ethyl,
      and wherein the compound according to Formula (1) or a pharmaceutically acceptable salt thereof is present in the pharmaceutical composition in a concentration of 1 mg/mL to 300 mg/mL.
2. The pharmaceutical composition according to item 1, wherein the stabilizer comprises para-aminohippuric acid and/or sodium para-aminohippurate.
3. The pharmaceutical composition according to item 1 or 2, wherein the concentration of the compound according to Formula (1), preferably para-aminohippuric acid, or a pharmaceutically acceptable salt thereof in the composition is in a range from about 1 mg/mL to about 100 mg/mL, preferably from about 2 mg/mL to about 80 mg/mL, more preferably from about 5 mg/mL to about 50 mg/mL, most preferably from about 7 mg/mL to about 30 mg/mL.
4. The pharmaceutical composition according to any one of items 1 to 3, wherein the excipients of the pharmaceutical composition essentially consist of the compound according to Formula (1), preferably para-aminohippuric acid, or a pharmaceutically acceptable salt thereof, and water.
5. The pharmaceutical composition according to any one of items 1 to 3, wherein the composition comprises a further stabilizer against radiolytic degradation of the radiolabeled complex.
6. The pharmaceutical composition according to item 5, wherein the further stabilizer against radiolytic degradation is selected from ascorbic acid and/or a salt thereof, in particular sodium ascorbate, an acetate buffer, in particular a sodium acetate buffer, ethanol gentisic acid, methionine, histidine, melatonine, and Se-methionine.
7. The pharmaceutical composition according to any one of items 1 to 6, wherein the composition additionally comprises a sequestering agent.
8. The pharmaceutical composition according to item 7, wherein the sequestering agent is DTPA.
9. The pharmaceutical composition according to any one of items1 to 8, wherein the sum of the radiolabeled complex and a conjugate comprising the targeting moiety linked to the chelating moiety (non-radioactive precursor) in the composition is in the range from about 1 µg/mL to about 100 µg/mL, preferably from about 3 µg/mL to about 80 µg/mL, more preferably from about 5 µg/mL to about 50 µg/mL, most preferably from about 7 pg/mL/30 mg/mL.
10. The pharmaceutical composition according to any one of items 1 to 9, wherein the targeting moiety of the radiolabeled complex is selected from peptides, peptidomimetics, synthetic small molecules, antibodies, antibody fragments, and antibody mimetics.
11. The pharmaceutical composition according to any one of items 1 to 10, wherein the targeting moiety targets and preferably binds to the somatostatin receptor, the prostate specific membrane antigen (PSMA), integrin, a folate receptor, the CCK2-receptor, the neurotensin receptor 1, the glucagon-like peptide 1 receptor, the neurokinin type 1 receptor, FAP, Glypican-3, Nectin-4, DLL3, Trop2, GPC3, IL-13Rα2 B7H3, or CAIX.
12. The pharmaceutical composition according to any one of items 1 to 11, wherein the targeting molecule is selected from the group consisting of Tyr3-octeotride, Tyr3-octreotate, JR11, PSMA-11, RGD, folate, neoBOMB, RM2, CM10, FAPi-04, FAPi-74, and FAPi-02.
13. The pharmaceutical composition according to any one of items 1 to 12, wherein the chelating moiety is a macrocyclic chelator, preferably selected from the group consisting of DOTA, AAZTA, NOTA, NODAGA, DOTAGA, DOTAM, HBED-CC, TRAP, NOPO, PCTA, DTPA, HYNIC, DFO, DO3AP, DO3AP^{PrA}, and DO3AP^{ABn}, or derivatives thereof.
14. The pharmaceutical composition according to any one of items 1 to 13, wherein the chelator is DOTA.
15. The pharmaceutical composition according to any one of items 1 to 14, wherein the radiolabeled complex comprises or consists of (i) a radionuclide and (ii) DOTA-TOC, DOTA-NOC, DOTA-TATE, DOTA-LM3, DOTA-JR11, PSMA-617, PSMA-I&T, PSMA-Ibu-DAB, DOTA-RGD, and DOTA-folate.
16. The pharmaceutical composition according to any one of items 1 to 14, wherein the radionuclide is selected from the group consisting of Ac-225, At-211, Bi-212, Bi-213, Co-55, Co-57, Cu-61, Cu-64, Cu-67, Dy-166, Er-165, Er-169, F-18, Ga-67, Ga-68, Ho-166, I-123, I-124, I-131, In-111, Lu-177, Pb-212, Pd-103, Pd-109, Re-186, Re-188, Rh-103m, Sc-43, Sc-44, Sc-47, Sm-153, Tb-149, Tb-152, Tb-155, Tb-161, Tc-99m, Th-227, Tc-99m, Y-86, and Y-90, and is preferably selected from the group consisting of Ac-225, At-211, Bi-212, Bi-213, Co-55, Co-57, Cu-61, Cu-64, Cu-67, Dy-166, Ga-67, Ga-68, Ho-166, In-111, Lu-177, Pb-212, Sc-43, Sc-44, Sc-47, Sm-153, Tb-161, Y-86, and Y-90.
17. The pharmaceutical composition according to any one of items 1 to 16, wherein the radionuclide is a beta-emitting radionuclide, such as Lu-177 or Tb-161, and the pharmaceutical composition has a radioactivity concentration of about 0.3 to 1.0 GBq/mL.
18. The pharmaceutical composition according to any one of items 1 to 16, wherein the radionuclide is an alpha-emitting radionuclide, such as Ac-225, and the pharmaceutical composition has a radioactivity concentration of about 0.2 to 1.0 MBq/mL.
19. The pharmaceutical composition according to any one of items 1 to 18, wherein the content of free nucleotides in the composition is ≤0.3%.
20. The pharmaceutical composition according to any one of items 1 to 19, wherein the radiochemical purity of the composition is maintained at ≥ 95% for at least 96 h after formulation when stored at room temperature.
21. The pharmaceutical composition according to any one of items 1 to 20, wherein the radiochemical purity of the composition is maintained at ≥ 95% for at least 48 h after formulation when stored at 40°C.
22. The pharmaceutical composition according to any one of items 1 to 21, wherein the composition is provided in a volume of 1 to 20 mL.
23. The pharmaceutical composition according to any one of items 1 to 22 for use in medicine.
24. The pharmaceutical composition according to any one of items 1 to 23 for use in the treatment of cancer.
25. A method for treating cancer or initiating, enhancing or prolonging an anti-tumor-response in a subject in need thereof comprising administering to the subject the pharmaceutical composition according to any one of items 1 to 24.
26. A method for preparing a pharmaceutical composition according to any one of items 1 to 22, comprising the steps of
   (a) radiolabeling a precursor comprising a targeting moiety covalently linked to a chelating moiety with a radionuclide in a radiolabeling buffer; and
   (b) adding a formulation buffer comprising a compound according to Formula (1) or a pharmaceutically acceptable salt thereof as a stabilizer: 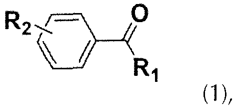
   wherein R₁ is selected from the group consisting of H, OH, NH₂, an alanine residue, and a glycine residue, and
   R₂ is selected from the group consisting of H, OH, NH₂, F, Cl, Br, I, SO₂, O-methyl, methyl, and O-ethyl.
27. The method according to item 26, wherein the stabilizer comprises para-aminohippuric acid and/or a pharmaceutically acceptable salt thereof.
28. The method according to any one of items 26 to 27, wherein the compound according to Formula (1), preferably para-aminohippuric acid, or a pharmaceutically acceptable salt thereof is added in step (b) such that the compound according to Formula (1), preferably para-aminohippuric acid, or a pharmaceutically acceptable salt thereof is present in the pharmaceutical composition in a range from about 10 mg/mL to about 300 mg/mL, more preferably from about 20 mg/mL to about 300 mg/mL, still more preferably from about 30 mg/mL to about 250 mg/mL, most preferably from about 50 mg/mL to about 200 mg/mL.
29. The method according to any one of items 26 to 28, wherein the salt of para-aminohippuric acid is sodium para-aminohippurate.
30. The method according to any one of items 26 to 29, wherein step (a) comprises heating a mixture of the radiolabeling precursor and the radionuclide to a temperature of about 20 to 110°C.
31. The method according to any one of items 26 to 30, wherein the radiolabeling buffer used in step (a) comprises ascorbic acid and/or a salt thereof, in particular sodium ascorbate.
32. The method according to any one of items 26 to 31, wherein the radiolabeling buffer used in step (a) comprises an acetate buffer, in particular a sodium acetate buffer.
33. The method according to any one of items 26 to 32, wherein the compound according to Formula (1), preferably para-aminohippuric acid, and/or a pharmaceutically acceptable salt thereof, such as sodium para-aminohippurate, is/are the only stabilizer(s) added in step (b).
34. The method according to any one of items 26 to 32, wherein a further stabilizer against radiolytic degradation is added in step (b), wherein the further stabilizer is preferably selected from ascorbic acid and/or a salt thereof, in particular sodium ascorbate, an acetate buffer, in particular a sodium acetate buffer, ethanol gentisic acid, methionine, histidine, melatonine, and Se-methionine.
35. The method according to any one of items 26 to 34, wherein a sequestering agent, such as e.g. DTPA is added in step (b).
36. The method according to any one of items 26 to 35, wherein para-aminohippuric acid or a pharmaceutically acceptable salt or carboxylic derivative thereof is added in step (b) such that the concentration of the sum of the radiolabeled complex and the (non-radiactive) precursor in the composition is in a range from about 1 µg/mL to about 100 µg/mL, preferably from about 3 µg/mL to about 80 µg/mL, more preferably from about 5 µg/mL to about 50 µg/mL, most preferably from about 7 µg/mL to about 30 µg/mL.
37. The method according to any one of items 26 to 36, wherein the targeting moiety of the precursor for radiolabeling is selected from peptides, peptidomimetics, synthetic small molecules, antibodies, antibody fragments, and antibody mimetics.
38. The method according to any one of items 26 to 37, wherein the targeting moiety of the precursor for radiolabeling targets and preferably binds to the somatostatin receptor, the prostate specific membrane antigen (PSMA), integrin, a folate receptor, the CCK2-receptor, the neurotensin receptor 1, the glucagon-like peptide 1 receptor, the neurokinin type 1 receptor, FAP, Glypican-3, Nectin-4, DLL3, Trop2, GPC3, IL-13Rα2, B7H3, or CAIX.
39. The method according to any one of items 26 to 38, wherein the targeting moiety of the precursor for radiolabeling is selected from the group consisting of Tyr3-octeotride, Tyr3-octreotate, JR11, PSMA-11, RGD and folate
40. The method according to any one of items 26 to 39, wherein the chelating moiety of the precursor for radiolabeling is a macrocyclic chelator, preferably selected from the group consisting of DOTA, AAZTA, NOTA, NODAGA, DOTAGA, DOTAM, HBED-CC, TRAP, NOPO, PCTA, DTPA, HYNIC, DFO, DO3AP, DO3AP^{PrA}, and DO3AP^{ABn}, or derivatives thereof.
41. The method according to any one of items 26 to 40, wherein the chelating moiety of the precursor for radiolabeling is DOTA.
42. The method according to any one of items 26 to 41, wherein the precursor for radiolabeling comprises or consists of DOTA-TOC, DOTA-NOC, DOTA-TATE, DOTA-LM3, DOTA-JR11, PSMA-617, PSMA-I&T, PSMA-lbu-DAB, DOTA-RGD, and DOTA-folate.
43. The method according to any one of items 26 to 42, wherein the radionuclide is selected from the group consisting of Ac-225, At-211, Bi-212, Bi-213, Co-55, Co-57, Cu-61, Cu-64, Cu-67, Dy-166, Er-165, Er-169, F-18, Ga-67, Ga-68, Ho-166, I-123, I-124, I-131, In-111, Lu-177, Pb-212, Pd-103, Pd-109, Re-186, Re-188, Rh-103m, Sc-43, Sc-44, Sc-47, Sm-153, Tb-149, Tb-152, Tb-155, Tb-161, Tc-99m, Th-227, Tc-99m, Y-86, and Y-90, and is preferably selected from the group consisting of Ac-225, At-211, Bi-212, Bi-213, Co-55, Co-57, Cu-61, Cu-64, Cu-67, Dy-166, Ga-67, Ga-68, Ho-166, In-111, Lu-177, Pb-212, Sc-43, Sc-44, Sc-47, Sm-153, Tb-161, Y-86, and Y-90.
44. The method according to any one of items 26 to 43, wherein the radionuclide is a beta-emitting radionuclide, such as Lu-177 or Tb-161, and the radionuclide is present in step (a) such that after adding para-aminohippuric acid in step (b) as a stabilizer, the pharmaceutical composition has a radioactivity concentration of about 0.3 to 1.0 GBq/mL.
45. The method according to any one of items 26 to 43, wherein the radionuclide is an alpha-emitting radionuclide, such as Ac-225, and the radionuclide is present in step (a) such that after adding PAH in step (b) as a stabilizer, the pharmaceutical composition has a radioactivity concentration of about 0.2 to 1.0 MBq/mL.
46. A pharmaceutical composition obtained by the method according to any one of items 26 to 45.
47. The pharmaceutical composition of item 46, wherein the pharmaceutical composition is as defined in any one of items 1 to 22.
48. Use of a compound according to Formula (1) or a pharmaceutically acceptable salt thereof as a stabilizer against radiolytic degradation of a radiolabeled complex in a pharmaceutical composition comprising the radiolabeled complex comprising (i) a radionuclide, and (ii) a targeting moiety covalently linked to a chelating moiety.
49. Use according to item 48, wherein the compound according to Formula (1) is para-aminohippuric acid or a pharmaceutically acceptable salt thereof.
50. Use according to item 49, wherein the salt of para-aminohippuric acid is sodium para-aminohippurate.
51. Use according to any one of items 48 to 50, wherein the concentration of the compound according to Formula (1), preferably para-aminohippuric acid, or a pharmaceutically acceptable salt thereof, such as sodium para-aminohippurate, in the composition is in a range from about 10 mg/mL to about 300 mg/mL, more preferably from about 20 mg/mL to about 300 mg/mL, still more preferably from about 30 mg/mL to about 250 mg/mL, most preferably from about 50 mg/mL to about 200 mg/mL.
52. Use according to any one of items 48 to 51, wherein the compound according to Formula (1), preferably para-aminohippuric acid or a pharmaceutically acceptable salt thereof, such as sodium para-aminohippurate, is the only stabilizer in the pharmaceutical composition.
53. Use according to any one of items 48 to 51, wherein the composition comprises a further stabilizer against radiolytic degradation, wherein the further stabilizer is preferably selected from ascorbic acid and/or a salt thereof, in particular sodium ascorbate, an acetate buffer, in particular a sodium acetate buffer, ethanol gentisic acid, methionine, histidine, melatonine, and Se-methionine.
54. Use according to any one of items 48 to 53, wherein the composition additionally comprises a sequestering agent, such as e.g. DTPA.
55. Use according to any one of items 48 to 54, wherein the concentration of the sum of the radiolabeled complex and the non-radioactive precursor in the composition is in the range from about 1 µg/mL to about 100 µg/mL, preferably from about 3 µg/mL to about 80 µg/mL, more preferably from about 5 µg/mL to about 50 µg/mL, most preferably from about 7 µg/mL to about 30 µg/mL.
56. Use according to any one of items 48 to 55, wherein the targeting moiety is selected from peptides, peptidomimetics, synthetic small molecules, antibodies, antibody fragments, and antibody mimetics.
57. Use according to any one of items 48 to 56, wherein the targeting moiety targets and preferably binds to the somatostatin receptor, the prostate specific membrane antigen (PSMA), integrin, a folate receptor, the CCK2-receptor, the neurotensin receptor 1, the glucagon-like peptide 1 receptor, the neurokinin type 1 receptor, FAP, Glypican-3, Nectin-4, DLL3, Trop2, GPC3, IL-13Rα2, B7H3, or CAIX.
58. Use according to any one of items 48 to 57, wherein the targeting moiety is selected from the group consisting of Tyr3-octeotride, Tyr3-octreotate, JR11, PSMA-11, RGD and folate.
59. Use according to any one of items 48 to 58, wherein the chelating moiety is a macrocyclic chelator, preferably selected from the group consisting of DOTA, AAZTA, NOTA, NODAGA, DOTAGA, DOTAM, HBED-CC, TRAP, NOPO, PCTA, DTPA, HYNIC, Macropa, TCMC, PEPA, HEHA, TETA DFO, DO3AP, DO3AP^{PrA}, and DO3AP^{ABn}, or derivatives thereof.
60. Use according any one of items 48 to 59, wherein the chelator is DOTA.
61. Use according to any one of items 48 to 60, wherein the radiolabeled complex comprises or consists of (i) a radionuclide and (ii) DOTA-TOC, DOTA-NOC, DOTA-TATE, DOTA-LM3, DOTA-JR11, PSMA-617, PSMA-I&T, PSMA-lbu-DAB, DOTA-RGD, and DOTA-folate, neoBOMB, RM2, CM10, FAPi-04, FAPi-74, FAPi-02.
62. Use according to any one of items 48 to 61, wherein the radionuclide is selected from the group consisting of Ac-225, At-211, Bi-212, Bi-213, Co-55, Co-57, Cu-61, Cu-64, Cu-67, Dy-166, Er-165, Er-169, F-18, Ga-67, Ga-68, Ho-166, I-123, I-124, I-131, In-111, Lu-177, Pb-212, Pd-103, Pd-109, Re-186, Re-188, Rh-103m, Sc-43, Sc-44, Sc-47, Sm-153, Tb-149, Tb-152, Tb-155, Tb-161, Tc-99m, Th-227, Tc-99m, Y-86, and Y-90, and is preferably selected from the group consisting of Ac-225, At-211, Bi-212, Bi-213, Co-55, Co-57, Cu-61, Cu-64, Cu-67, Dy-166, Ga-67, Ga-68, Ho-166, In-111, Lu-177, Pb-212, Sc-43, Sc-44, Sc-47, Sm-153, Tb-161, Y-86, and Y-90.
63. Use according to any one of items 48 to 62, wherein the radionuclide is a beta-emitting radionuclide, such as Lu-177 or Tb-161, and the pharmaceutical composition has a radioactivity concentration of about 0.3 to 1.0 GBq/mL.
64. Use according to any one of items 48 to 62, wherein the radionuclide is an alpha-emitting radionuclide, such as Ac-225, and the pharmaceutical composition has a radioactivity concentration of about 0.2 to 1.0 MBq/mL.
65. Use according to any one of items 48 to 64, wherein the content of free nucleotides in the pharmaceutical composition is ≤0.3%.
66. Use according to any one of items 48 to 65, wherein the radiochemical purity of the pharmaceutical composition is maintained at ≥ 98% for at least 96 h at room temperature.
67. Use according to any one of items 48 to 66, wherein the radiochemical purity of the composition is maintained at ≥ 95% for at least 48 h at 40°C.
68. Use according to any one of items 48 to 67, wherein the composition is provided in a volume of 1 to 20 mL.
69. Use according to any one of items 48 to 68, wherein the pharmaceutical composition is for use in medicine.
70. Use according to any one of items 48 to 69, wherein the pharmaceutical composition is for use in the treatment of cancer.

### EXAMPLES

In the following, particular examples illustrating various embodiments and aspects of the invention are presented. However, the present invention shall not to be limited in scope by the specific embodiments described herein. The following preparations and examples are given to enable those skilled in the art to more clearly understand and to practice the present invention. The present invention, however, is not limited in scope by the exemplified embodiments, which are intended as illustrations of single aspects of the invention only, and methods which are functionally equivalent are within the scope of the invention. Indeed, various modifications of the invention in addition to those described herein will become readily apparent to those skilled in the art from the foregoing description and the examples below. All such modifications fall within the scope of the appended claims.

### Example 1: Preparation of para-aminohippurate sodium (PAH) stabilized formulations comprising different radiolabeled complexes

Various formulations comprising different kinds and concentrations of radiolabeled complexes and different concentrations of para-aminohippurate sodium (PAH) were prepared according to the protocol below.

### 1.1 Radiolabeling and formulation with Lu-177 and Tb-161

Radiolabeling with Lu-177 and Tb-161 was performed in a qualified heating block (Thermo Fisher Scientific Inc. USA). To this end, 100 to 400 µg of the precursor for radiolabeling in ascorbic acid/sodium ascorbate buffer were added directly to a 2 mL vial containing the radionuclide precursor [¹⁷⁷Lu]LuCl₃ or [¹⁶¹Tb]TbCl₃, respectively (ITM Medical Isotopes GmbH, Munich, Germany), dissolved in 0.04 M HCl (40 MBq/mL). The reaction mixture was heated for 20 - 30 min at 80 - 95°C to obtain ¹⁷⁷Lu and ¹⁶¹Tb labeled complexes, respectively. After cooling, the labeling solution was diluted with formulation buffer in concentrations and volumes shown in Table 1A.

### 1.2 Radiolabeling and formulation with Ac-225

Radiolabeling was performed in a qualified heating block (Thermo Fisher Scientific Inc. USA). To this end, 100 to 400 µg of the precursor for radiolabeling in sodium acetate buffer were added directly to a 2 mL vial containing the radionuclide precursor [²²⁵Ac]AcCl₃ dissolved in 0.04 M HCl (40 MBq/mL) (ITM Medical Isotopes GmbH, Munich, Germany). The reaction mixture was heated for 15 - 20 min at 80 - 95 °C to obtain ²²⁵Ac labeled complexes. After cooling, the labeling solution was diluted with formulation buffer in concentrations and volumes shown in Table 1A.

**Table 1A. Formulations**

| Radionuclide | Precursor | PAH [mg/mL] | Other Excipient | A [MBq] | V [mL] | C (MBq/mL) | pH |
|---|---|---|---|---|---|---|---|
| Lu-177 | Redfol-25 | 50 | - | 8000 | 18,5 | 432 | 5.5-6.0 |
| | | 100 | - | | | | 6 |
| | | 100 | 0.1 mM DTPA | | | | 6 |
| | | 200 | | | | | 6 |
| | | - | 40 mg/mL Ascorbate | | | | 5.5-6.0 |
| | DOTA-JR11 | 200 | - | 7500 | 20 | 375 | 7-8 |
| | PSMA-617 | | | | | | |
| | PSMA-Ibu-DAB | | | | | | |
| | DOTA-RGD | | | | | | |
| | DOTA-LM3 | | | | | | |
| | DOTA-NOC | | | | | | |
| Tb-161 | Redfol-25 | 200 | - | 5000 | 20 | 250 | 7-8 |
| | DOTA-JR11 | | | | | | |
| | PSMA-617 | | | | | | |
| | PSMA-Ibu-DAB | | | | | | |
| | DOTA-RGD | | | | | | |
| | DOTA-LM3 | | | | | | |
| | DOTA-NOC | | | | | | |
| | DOTA-TOC | | | | | | |
| Ac-225 | Redfol-25 | 200 | - | 1 | 2,5 | 0,4 | 7-8 |
| | DOTA-JR11 | | | | | | |
| | DOTA-TOC | | | | | | |
| | PSMA-Ibu-DAB | | | | | | |
| | PSMA-617 | | | | | | |
| | DOTA-NOC | | | | | | |
| | DOTA-LM3 | | | | | | |
| | PSMA-Ibu-DAB | - | In 40 mg/mL Na-Ascorbate + 5% EtOH | | | | 6-7 |

| | | | | | | | |
|---|---|---|---|---|---|---|---|
| A= radioactivity; V= volume; C= concentration | | | | | | | |

The structural formulas of the precursors of the radiolabelled complexes used in the Examples are shown in Table 1B below:

**Table 1B. Precursor compounds**

| | |
|---|---|
| 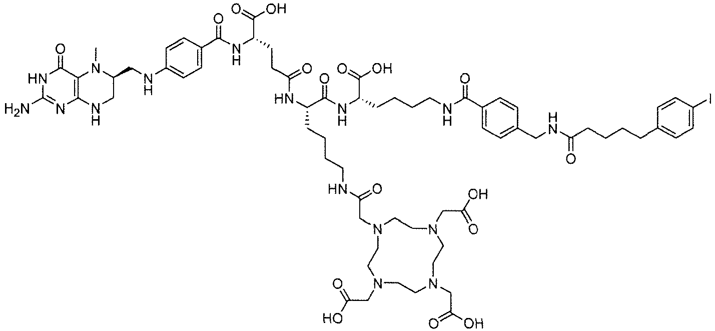 | 6*S-*RedFol-25 |
| 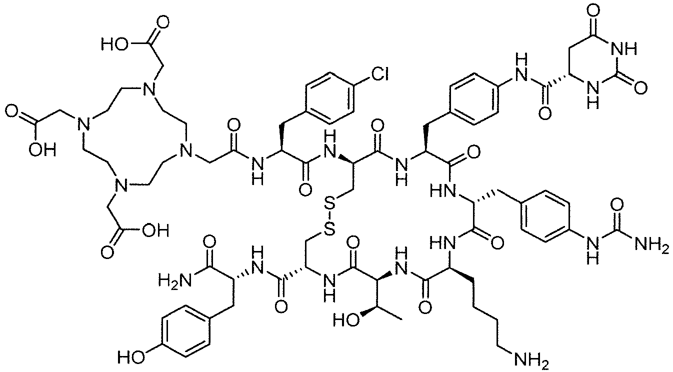 | DOTA-JR 11 |
| 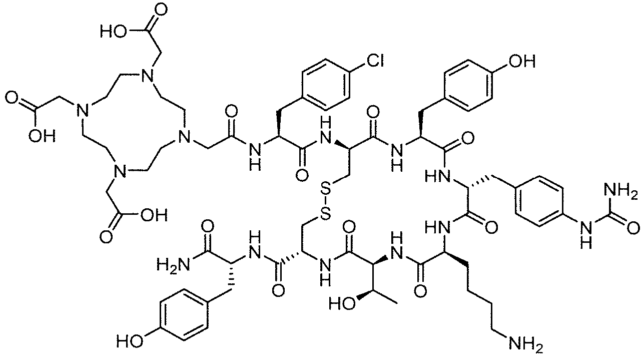 | DOTA-LM3 |
| 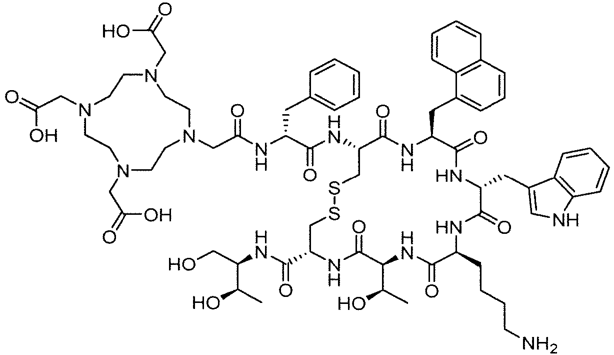 | DOTA-NOC |
| 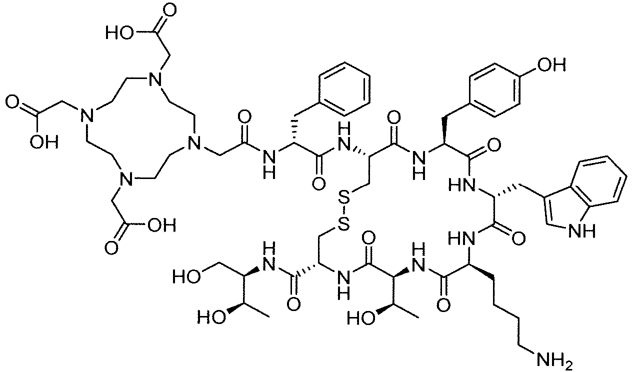 | DOTA-TOC |
| 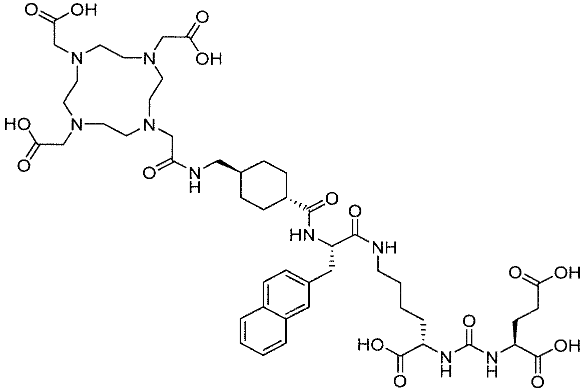 | PSMA-617 |
| 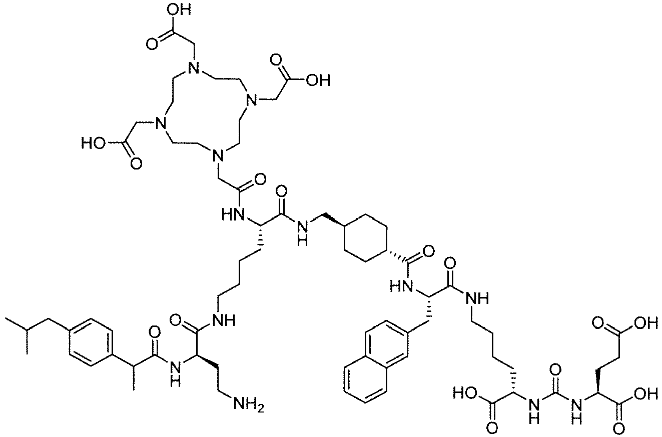 | Ibu-DAB-PSMA |
| 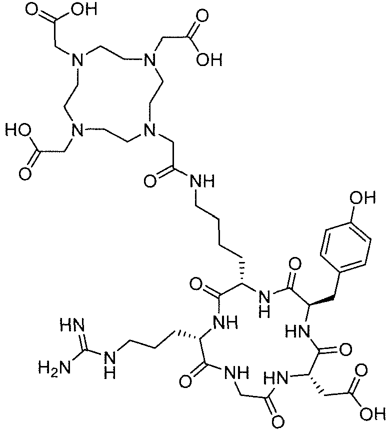 | DOTA-RGD |

The formulations were sterile filtered, and their radiochemical purities and stabilities were determined employing radio thin-layer chromatography (TLC) and radio-HPLC, respectively, as described below.

### 1.3 Radio thin-layer chromatography (TLC):

TLC 1: 1 - 5 µL of formulation solution were spotted onto the instant thin-layer chromatography strips (iTLC strips) and developed in 0.1 M ammonium acetate:DMF (1:1). The iTLC strips were read out on a radiograph, and radiochemical purity of the radiolabeled complex was determined as follows: Rf value (impurity) = 0.0 - 0.2; Rf value (radiolabeled complex) = 0.7 - 1.0.

TCL 2: 1 - 5 µL of formulation solution were spotted onto Silica on aluminum strip 60 F254 (Merck Millipore) and developed in 0.1 M sodium citrate (pH5). The TLC strips were read out on a radiograph, and radiochemical purity of the radiolabeled complex was determined as follows: Rf value (impurity) = 0.8 - 1.0; Rf value (radiolabeled complex) = 0.0 - 0.2.

### 1.4 Radio-HPLC:

Radiochemical purity was further determined by radio HPLC using a mobile phase Acclaim C18 column A. 0.1% TFA in water, B: 0.1 % TFA in MeCN.

### 1.5 Stability tests

The formulated radiolabeled complexes were tested in a climate cabinet at the following conditions:
- 25°C (room temperature, RT), 60% humidity
- 40°C, 70% humidity

Samples were taken at regular intervals and analysed for purity using HPLC or TLC as described above.

### Example 2: Stability of Lu-177-labeled DOTA-folate in PAH stabilized formulations

Various formulations of Lu-1 77-labeled DOTA-folate ([1 77Lu]Lu-RedFol-25) were tested with regard to radiochemical stability of the radiolabeled complex over a period of 72 hours.

To this end, a Lu-177-labeled DOTA-folate ([¹⁷⁷Lu]Lu-RedFol-25), prepared as described in Example 1, was diluted with formulation buffer comprising different concentrations of PAH or, for comparison, comprising sodium ascorbate (40 mg/mL) in a reaction vial, transferred to a product vial by sterile filtration, and the stability of the radiolabeled complexes were determined by regular analysis of free Lu-177, radiolabeled complex ([¹⁷⁷Lu]Lu-RedFol-25) and impurities at predefined points in time (EOS =end of synthesis; 48 h, 72 h) using HPLC as described above.

Formulations tested and results are shown in Table 2 below.

**Table 2. Stability of DOTA-folate in different formulations**

| **Tested formulation** | **Species** | **Area % HPLC EOS** | **Area % HPLC 48 h** | **Area % HPLC 72 h** |
|---|---|---|---|---|
| | Free Lu-177 | 0.1 % | 0.2 % | 0.2 % |
| 50 mg/mL PAH | [¹⁷⁷Lu]Lu-RedFol-25 | 99.4 % | 99.0 % | 98.9 % |
| | ∑ (unknown impurities) | 0.6 % (RRT: 0.54 → 0.2 %) | 1.0 % (RRT: 0.54 → 0.5 %) | 0.9 % (RRT: 0.54 → 0.5 %) |
| 100 mg/mL PAH | Free Lu -177 | 0.1 % | 0.1 % | 0.2 % |
| | [¹⁷⁷Lu] Lu-RedFol-25 | 99.4 % | 99.1 % | 99.1 % |
| | ∑ (unknown impurities) | 0.6 % (RRT: 0.54 → 0.2 %) | 0.9 % (RRT: 0.54 → 0.4 %) | 0.9 % (RRT: 0.54 → 0.4 %) |
| 100 mg/mL PAH + 0.1 mM DTPA | Free Lu -177 | 0.1 % | 0.1 % | 0.2 % |
| | [¹⁷⁷Lu] Lu-RedFol-25 | 99.4 % | 99.1 % | 99.0 % |
| | ∑ (unknown impurities) | 0.6 % (RRT: 0.54 → 0.2 %) | 0.9 % (RRT: 0.54 → 0.4 %) | 1.0 % (RRT: 0.54 → 0.4 %) |
| 200 mg/mL PAH | Free Lu -177 | 0.1 % | 0.1 % | 0.2 % |
| | [¹⁷⁷Lu]Lu-RedFol-25 | 99.4 % | 99.3 % | 99.0 % |
| | ∑ (unknown impurities) | 0.6 % (RRT: 0.54 → 0.2 %) | 0.7 % (RRT: 0.54 → 0.3 %) | 1.0 % (RRT: 0.54 → 0.4 %) |
| For comparison: 40 mg/mL Na-ascorbate solution | Free Lu -177 | 0.3 % | 0.4 % | 0.5 % |
| | [¹⁷⁷Lu]Lu-RedFol-25 | 99.2 % | 95.9 % | 94.6 % |
| | ∑ (unknown impurities) | 0.5 % (RRT: 0.54 → 0.2 %) | 3.7 % (RRT: 0.54 → 2.3%) | 4.9 % (RRT: 0.54 → 3.1 %) |

| | | | | |
|---|---|---|---|---|
| EOS = end of synthesis; RRT = relative retention time | | | | |

The results shown in Table 2 indicate that a strong protective effect against radiolysis of a Lu-177-labeled DOTA-folate is observed with a formulation containing different concentrations (50 mg/mL, 100 mg/mL, 200 mg/mL) PAH as a stabilizer. In particular, a radiochemical purity of about 99% still can be observed 72 hours after synthesis. In contrast thereto, a formulation containing 40 mg/mL sodium ascorbate as a stabilizer, shows reduced stability compared to the PAH stabilized formulations in particular at 48h and 72h after preparation.

### Example 3: Stability of different Lu-177-labeled complexes in PAH stabilized formulations

Formulations comprising various Lu-177-labeled complexes stabilized with 200 mg/mL PAH were tested with regard to radiochemical stability of the radiolabeled complex over a period of 96 hours.

To this end, various Lu-177-labeled complexes targeting the somatostatin receptor (DOTA-NOC, DOTA-LM3), targeting PSMA (PSMA-617, PSMA-lbu-DAB), or targeting integrin (DOTA-RGD), prepared as described in Example 1, were diluted with formulation buffer comprising 200 mg/mL PAH in the reaction vial, transferred to the product vial by sterile filtration, and the stability of the Lu-177-labeled complexes were determined by regular analysis of free Lu-177, Lu-177-labeled complex and impurities at predefined points in time (EOS = end of synthesis; 24 h, 96 h) using HPLC as described above. Formulations tested and results are shown in Table 3 below.

**Table 3. Stability of Lu-177-labeled complexes in PAH stabilized formulations**

| **Lu-177-labeled complex** | **Test time** | **% RCP (area % HPLC)** | **% free Lu-177 (area % HPLC)** | **% sum of unknown impurities (area % HPLC)** |
|---|---|---|---|---|
| DOTA-NOC | EOS | 97.2 | 0.2 | 2.7 |
| | 24 h | 97.8 | 0.2 | 1.9 |
| | 96 h | 97.7 | 0.2 | 2.1 |
| PSMA-617 | EOS | 95.2 | 0.3 | 4.5 |
| | 24 h | 95.2 | 0.1 | 4.8 |
| | 96 h | 95.8 | 0.2 | 4.0 |
| PSMA-lbu-DAB | EOS | 95.1 | 0.4 | 4.5 |
| | 24 h | 95.9 | 0.2 | 3.9 |
| | 96 h | 94.9 | 0.2 | 5.0 |
| DOTA-RGD | EOS | 98.2 | 0.2 | 1.6 |
| | 24 h | 98.1 | 0.1 | 1.8 |
| | 96 h | 97.1 | n.a. | 2.9 |
| DOTA-LM3 | EOS | 97.5 | 0.2 | 2.3 |
| | 24 h | 96.9 | 0.3 | 2.9 |
| | 96 h | 97.2 | 0.2 | 2.6 |

| | | | | |
|---|---|---|---|---|
| EOS = end of synthesis; RCP = radiochemical purity | | | | |

The results shown in Table 3 indicate that a strong protective effect against radiolysis of various ¹⁷⁷Lu-labeled complexes targeting different target molecules is observed up to 96 hours after the synthesis with a formulation containing 200 mg/mL PAH as a stabilizer. In particular, with all tested formulations, a radiochemical purity of ≥95% still can be observed 96 hours after synthesis.

### Example 4: Stability of different Tb-161-labeled complexes in PAH stabilized formulations

Formulations comprising various Tb-161-labeled complexes stabilized with 200 mg/mL PAH were tested with regard to radiochemical stability of the radiolabeled complex over a period of at least 96 hours.

To this end, various Tb-161-labeled complexes targeting the somatostatin receptor (DOTA-NOC, DOTA-TOC, DOTA-LM3), targeting PSMA (PSMA-617, PSMA-Ibu-DAB), or targeting the folate receptor (DOTA-folate), prepared as described in Example 1, were diluted with formulation buffer comprising 200 mg/mL PAH in the reaction vial, transferred to the product vial by sterile filtration, and the stability of the Tb-161-labeled complexes were determined by regular analysis of free Tb-161, Tb-161-labeled complex and impurities at predefined points in time (EOS = end of synthesis; 24 h, 96 h) using HPLC as described above.

Formulations tested and results are shown in Table 4 below.

**Table 4. Stability of Tb-161-labeled complexes in PAH stabilized formulations**

| **Tb-161-labeled complex** | **Test time** | **%RCP (area % HPLC)** | **% free Tb-161 (area % HPLC)** | **% sum of unknown impurities (area % HPLC)** |
|---|---|---|---|---|
| DOTA-NOC | EOS | 97.7 | 0.1 | 2.3 |
| | 24 h | 97.3 | 0.1 | 2.6 |
| | 96 h | 97.0 | 0.3 | 2.7 |
| PSMA-617 | EOS | 95.4 | 0.2 | 4.5 |
| | 24 h | 95.4 | 0.2 | 4.5 |
| | 96 h | 95.6 | 0.1 | 4.3 |
| PSMA-lbu-DAB | EOS | 95.8 | 0.1 | 4.1 |
| | 24 h | 95.8 | 0.1 | 4.1 |
| | 96 h | 95.7 | 0.2 | 4.2 |
| DOTA-LM3 | EOS | 97.9 | 0.1 | 2.0 |
| | 24 h | 98.1 | 0.1 | 1.8 |
| | 96 h | 97.8 | 0.1 | 2.1 |
| DOTA-TOC | EOS | 98.4 | 0.2 | 1.4 |
| | 24 h | 97.8 | 0.2 | 2.0 |
| | 96 h | 98.6 | 0.2 | 1.2 |
| DOTA-Folate | EOS | 99.0 | 0.1 | 1.1 |
| | 24 h | 98.9 | 0.1 | 0.9 |
| | 96 h | 98.7 | 0.2 | 0.8 |
| | 168 h | 98.7 | 0.2 | 0.9 |

| | | | | |
|---|---|---|---|---|
| EOS = end of synthesis; RCP = radiochemical purity | | | | |

The results shown in Table 4 indicate that a strong protective effect against radiolysis of various Tb-161-labeled complexes targeting different target molecules (somatostatin receptor, PSMA, folate receptor) is observed with a formulation containing 200 mg/mL PAH as a stabilizer. In particular, with all tested formulations, a radiochemical purity of >95% is observed 96 hours after synthesis. Notably, a radiochemical purity of 98.7% is still observed after 168 hours (7 days) with Tb-161-labeled DOTA-folate.

### Example 5: Stability of different Ac-225-labeled complexes in PAH stabilized formulations

Formulations comprising various Ac-225-labeled complexes stabilized with 200 mg/mL PAH were tested with regard to radiochemical stability of the radiolabeled complex over a period of 96 hours.

To this end, various Ac-225-labeled complexes targeting the somatostatin receptor (DOTA-NOC, DOTA-TOC), targeting PSMA (PSMA-lbu-DAB), or targeting the folate receptor (6S-RedFol-25), prepared as described in Example 1, were diluted with formulation buffer comprising 200 mg/mL PAH in the reaction vial, transferred to the product vial by sterile filtration, and the stability of the Ac-225-labeled complexes were determined by regular analysis of free Ac-225, radiochemical purity (RSP) of the Ac-225-labeled complex and impurities at predefined points in time (EOS = end of synthesis; 12 h, 48h, 72h, 96 h) using HPLC as described above.

Formulations tested and results are shown in Table 5 below.

**Table 5. Stability of Ac-225-labeled complexes in PAH stabilized formulations**

| **Ac-225-labeled complex** | **Test time** | **%RCP (% HPLC-FC Gamma)** | **%free Ac-225 (% HPLC-FC Gamma)** | % Sum **unknown impuritie s (% HPLC-FC Gamma)** |
|---|---|---|---|---|
| DOTA-NOC | 12 h | 99.0 | 0.2 | 0.8 |
| | 48 h | 98.9 | 0.3 | 0.7 |
| | 72 h | 98.9 | 0.2 | 0.8 |
| | 96 h | 99.1 | 0.2 | 0.7 |
| PSMA-lbu-DAB | 12 h | 97.0 | 0.1 | 3.0 |
| | 48 h | 97.0 | 0.1 | 3.0 |
| | 72 h | 96.4 | 0.2 | 3.3 |
| | 96 h | 96.8 | 0.2 | 3.0 |
| DOTA-TOC | 12 h | 99.3 | 0.2 | 0.5 |
| | 48 h | 99.3 | 0.3 | 0.3 |
| | 72 h | 99.3 | 0.3 | 0.4 |
| | 96 h | 99.2 | 0.3 | 0.6 |
| 6S-RedFol-25 | 12 h | 99.3 | 0.2 | 0.4 |
| | 48 h | 99.0 | 0.6 | 0.4 |
| | 72 h | 99.0 | 0.4 | 0.7 |
| | 96 h | 99.2 | 0.3 | 0.5 |
| DOTA-LM3 | EOS | 98.7 | 0.3 | 0.9 |
| | 48 h | 99.4 | 0.1 | 0.5 |
| | 72 h | 98.4 | 0.4 | 1.1 |
| | 96 h | 98.6 | 0.4 | 1.0 |
| DOTA-JR-11 | EOS | 99.0 | 0.6 | 0.4 |
| | 48 h | 99.3 | 0.2 | 0.4 |
| | 72 h | 99.4 | 0.2 | 0.4 |
| | 96 h | 99.2 | 0.3 | 0.5 |
| PSMA-617 | EOS | 99.7 | 0.2 | 0.2 |
| | 48 h | 99.7 | 0.2 | 0.1 |
| | 72 h | 99.6 | 0.2 | 0.1 |
| | 96 h | 99.7 | 0.2 | 0.1 |
| PSMA-lbu-DAB in 40 mg/mL Na-ascorbate + 5% EtOH | EOS | 94.7 | 0.2 | 4.7 |
| | 24 h | 95.4 | 0.4 | 4.2 |
| | 48 h | 95.4 | 0.7 | 3.9 |
| | 72 h | 94.9 | 1.2 | 4.0 |

| | | | | |
|---|---|---|---|---|
| EOS = end of synthesis; RCP = radiochemical purity | | | | |

The results shown in Table 5 indicate that a strong protective effect against radiolysis of various ²²⁵Ac-labeled complexes targeting different target molecules (somatostatin receptor, PSMA, folate receptor) is observed with a formulation containing 200 mg/mL PAH as a stabilizer. In particular, with all tested formulations, a radiochemical purity of >96% and up to 99% is observed 96 hours after synthesis, whereby free 225-Ac is present in the formulation to a maximum of 0.3%. In contrast, the radiochemical purity of a formulation stabilized with 40 mg/mL Na-ascorbate + 5% EtOH is <95% even after 72h, and free 225-Ac is present in the Na-ascorbate stabilized formulation in an amount of 1.2%.

### Example 6: Stability of Lu-177-labeled complexes in PAH stabilized formulations at elevated temperatures

Formulations comprising a Lu-177-labeled complex stabilized with 50 mg/mL PAH were tested with regard to radiochemical stability of the radiolabeled complex at different temperatures (RT and 40°C) over a period up to 240 hours (10 days).

To this end, a Lu-177-labeled complex (DOTA-folate; RedFol-25), also prepared as described in Example 1, was diluted with formulation buffer comprising 50 mg/mL PAH in the reaction vial, and transferred to the product vial by sterile filtration. The vials were placed at a climate cabinet at 25°C (room temperature, RT), 60% humidity, and at 40°C, 70% humidity, respectively, and the stability of the Lu-177-labeled complex were determined by regular analysis of free Lu-177, radiochemical purity (RCP) of the Lu-177-labeled complex and impurities at predefined points in time indicated in Table 6 below using HPLC as described above. Parallel, a reference sample not stabilized with PAH was tested at RT for comparison. Activity reference times (ART) are indicated in Table 6.

**Table 6. Stability of Lu-177-labeled complexes in formulations stabilized with 5% PAH at different temperatures**

| **Formulation** | **Test time** | **% RCP (area % HPLC)** | **% free Lu-177 (area % HPLC)** | **% Sum of unknown impurities (area % HPLC)** |
|---|---|---|---|---|
| 5% PAH - RT ART: EOS+96 h | EOS | 99.7 | 0.1 | 0.2 |
| | 96 h | 98.7 | 0.5 | 0.9 |
| | 168 h (7d) | 98.3 | 0.2 | 1.4 |
| | 240 h (10d) | 98.1 | 0.3 | 1.6 |
| 5% PAH - 40°C ART: EOS+7d (200 µg/8 GBq @ART) | EOS | 99.4 | 0.1 | 0.6 |
| | 24 h | 99.1 | 0.2 | 0.7 |
| | 48 h | 98.7 | 0.4 | 1.0 |
| | 168 h (7d) | 96.1 | 1.1 | 2.8 (ind. Imp. 2.0%) |
| | 240 h (10 d) | 95.9 | 1.1 | 3.0 (ind. Imp. 2.0%) |
| without PAH - RT ART: EOS+7d Formulation in 0.9% NaCl | EOS | 99.2 | 0.2 | 0.6 |
| | 24 h | 90.5 | 0.6 | 8.9 |
| | 48 h | 83.0 | 1.2 | 15.8 |
| | 168 h (7d) | 57.1 | 4.0 | 38.9 |
| | 240 h (10d) | 46.1 | 8.6 | 45.4 |

| | | | | |
|---|---|---|---|---|
| EOS: End of Synthesis; ART: activity reference time; RT: room temperature; RCP: radiochemical purity | | | | |

The results shown in Table 6 indicate that even at an elevated temperature of 40°C, a strong protective effect against radiolysis of the tested Lu-177-labeled complex is observed with a formulation containing 50 mg/mL PAH as a stabilizer. In particular, a radiochemical purity of >98% is observed 48 hours after synthesis, when the sample is stored at 40°C. In contrast, the radiochemical purity of a formulation which does not comprise PAH as a stabilizer is <83% 48h after synthesis even when stored at RT. Notably, free 177-Lu is present in the 0.9% NaCl formulation in an amount of 15.8%.

Thus, stabilization of the radiolabeled complexes with PAH according to the present invention ensures superior radiolysis stability and thus superior radiochemical purity of radiolabeled complexes over a longer period of time compared to formulations lacking PAH or stabilized with common stabilizers such as sodium ascorbate, respectively. The high radiochemical purity and prolonged stability of radiolabeled complexes are even preserved at elevated temperatures such as 40°C. Due to the prolonged stability with consistently high quality over several days even at elevated temperatures, centralized production with subsequent distribution of the radiopharmaceutical pharmaceutical composition to the treatment centers can be guaranteed.

## Claims

1. A pharmaceutical composition comprising
(a) a radiolabeled complex comprising (i) a radionuclide, and (ii) a targeting moiety covalently linked to a chelating moiety; and
(b) a stabilizer against radiolytic degradation of the radiolabeled complex,
wherein the stabilizer comprises a compound according to Formula (1) or a pharmaceutically acceptable salt thereof: 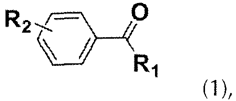 wherein R₁ is selected from the group consisting of H, OH, NH₂, an alanine residue, and a glycine residue, and
R₂ is selected from the group consisting of H, OH, NH₂, F, Cl, Br, I, SO₂, O-methyl, methyl, and O-ethyl,
and wherein the compound according to Formula (1) or a pharmaceutically acceptable salt thereof is present in the pharmaceutical composition in a concentration of 1 mg/mL to 300 mg/mL.

2. The pharmaceutical composition according to claim 1, wherein the stabilizer comprises para-aminohippuric acid and/or sodium para-aminohippurate.

3. The pharmaceutical composition according to claim 1 or 2, wherein the concentration of the compound according to Formula (1), preferably para-aminohippuric acid, or a pharmaceutically acceptable salt thereof in the composition is in a range from about 10 mg/mL to about 300 mg/mL, preferably from about 20 mg/mL to about 300 mg/mL, more preferably from about 30 mg/mL to about 250 mg/mL, most preferably from about 50 mg/mL to about 200 mg/mL.

4. The pharmaceutical composition according to any one of the previous claims, wherein the excipients of the pharmaceutical composition essentially consist of the compound according to Formula (1), preferably para-aminohippuric acid, or a pharmaceutically acceptable salt thereof, and water.

5. The pharmaceutical composition according to any one of the previous claims, wherein the concentration of the sum of the radiolabeled complex comprising the radionuclide and the targeting moiety covalently linked to the chelating moiety, and the non-radioactive precursor in the pharmaceutical composition is in the range from about 1 µg/mL to about 100 µg/mL, preferably from about 5 µg/mL to about 50 µg/mL, more preferably from about 7 µg/mL to about 30 µg/mL.

6. The pharmaceutical composition according to any one of the previous claims, wherein the targeting moiety of the radiolabeled complex is selected from peptides, peptidomimetics, synthetic small molecules, antibodies, antibody fragments, and antibody mimetics.

7. The pharmaceutical composition according to any one of the previous claims, wherein the targeting moiety targets and preferably binds to the somatostatin receptor, the prostate specific membrane antigen (PSMA), integrin, a folate receptor, the CCK2-receptor, the neurotensin receptor 1, the glucagon-like peptide 1 receptor, the neurokinin type 1 receptor, FAP, Glypican-3, Nectin-4, DLL3, Trop2, GPC3, IL-13Rα2, B7H3, CAlX.

8. The pharmaceutical composition according to any one of the previous claims, wherein the chelating moiety is a macrocyclic chelator, preferably selected from the group consisting of DOTA, AAZTA, NOTA, NODAGA, DOTAGA, DOTAM, HBED-CC, TRAP, NOPO, PCTA, DTPA, HYNIC, Macropa, TCMC, PEPA, HEHA, TETA, DFO, DO3AP, DO3AP^{PrA}, and DO3AP^{ABn}, or derivatives thereof.

9. The pharmaceutical composition according to any one of the previous claims, wherein the macrocyclic chelator is DOTA or a derivative thereof, and the radiolabeled complex comprises or consists of (i) a radionuclide and (ii) DOTA-TOC, DOTA-NOC, DOTA-TATE, DOTA-LM3, DOTA-JR11, PSMA-617, PSMA-I&T, PSMA-lbu-DAB, DOTA-RGD, and DOTA-folate, neoBOMB, RM2, CM10, FAPi-04, FAPi-74, FAPi-02.

10. The pharmaceutical composition according to any one of the previous claims, wherein the radionuclide is selected from the group consisting of Ac-225, At-211, Bi-212, Bi-213, Co-55, Co-57, Cu-61, Cu-64, Cu-67, Dy-166, Er-165, Er-169, F-18, Ga-67, Ga-68, Ho-166, I-123, I-124, I-131, In-111, Lu-177, Pb-212, Pd-103, Pd-109, Re-186, Re-188, Rh-103m, Sc-43, Sc-44, Sc-47, Sm-153, Tb-149, Tb-152, Tb-155, Tb-161, Tc-99m, Th-227, Tc-99m, Y-86, and Y-90, and is preferably selected from the group consisting of Ac-225, At-211, Bi-212, Bi-213, Co-55, Co-57, Cu-61, Cu-64, Cu-67, Dy-166, Ga-67, Ga-68, Ho-166, In-111, Lu-177, Pb-212, Sc-43, Sc-44, Sc-47, Sm-153, Tb-161, Y-86, and Y-90.

11. A method for preparing a pharmaceutical composition according to any one of claims 1 to 10, comprising the steps of
(a) radiolabeling a precursor comprising a targeting moiety covalently linked to a chelating moiety with a radionuclide in a radiolabeling buffer; and
(b) adding a formulation buffer comprising a compound according to Formula (1) or a pharmaceutically acceptable salt thereof: 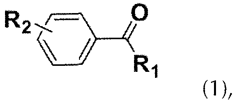 wherein R₁ is selected from the group consisting of H, OH, NH₂, an alanine residue, and a glycine residue, and
R₂ is selected from the group consisting of H, OH, NH₂, F, Cl, Br, I, SO₂, O-methyl, methyl, and O-ethyl,
preferably para-aminohippuric acid and/or a pharmaceutically acceptable salt thereof, such as sodium para-aminohippurate, as a stabilizer.

12. The method according to claim 11, wherein step (a) comprises heating a mixture of the precursor for radiolabeling and the radionuclide to a temperature of about 20 to 110°C.

13. The method according to any one of claims 11 or 12, wherein the radiolabeling buffer used in step (a) comprises ascorbic acid and/or a salt thereof, in particular sodium ascorbate, and/or an acetate buffer, in particular a sodium acetate buffer.

14. Use of a compound according to Formula (1), preferably para-aminohippuric acid, or a pharmaceutically acceptable salt thereof as a stabilizer against radiolytic degradation of the radiolabeled complex in a pharmaceutical composition comprising the radiolabeled complex comprising (i) a radionuclide, and (ii) a targeting moiety covalently linked to a chelating moiety.

15. Use according to claim 14, wherein the pharmaceutical composition is as defined in any one of claims 1 to 10.
